# EUROPEAN PATENT SPECIFICATION

(11) **EP 2 445 987 B1**
(45) Date of publication and mention of the grant of the patent: **02.05.2018**
(21) Application number: 10742624.9
(22) Date of filing: 24.06.2010
(51) Int. Cl.: C09K 11/06, C09K 11/07

(54) **BLUE/VIOLET DIPHENYLANTHRACENE CHEMILUMINESCENT FLUORESCERS**
BLAUE/VIOLETTE CHEMILUMINESZENTE DIPHENYLANTHRACEN-FLUORESZENZSTOFFE
AGENTS FLUORESCENTS CHIMIO-LUMINESCENTS BLEUS/VIOLETS DE TYPE DIPHÉNYLANTHRACÈNE

(30) Priority: 24.06.2009 US 220072 P
(43) Date of publication of application: 02.05.2012
(73) Proprietor: Cyalume Technologies, Inc, West Springfield, MA 01089 (US)
(72) Inventor: CRANOR, Earl, Longmeadow MA 01106 (US); JACOB, Linda, Woodbridge CT 06525 (US)
(74) Representative: HGF Limited
(86) International application number: PCT/US2010/039800
(87) International publication number: WO 2010/151654

(56) References cited:
- EP-A1- 1 312 605
- EP-A1- 1 380 556
- US-A- 5 597 517
- US-A1- 2005 224 768
- US-A1- 2008 308 776
- ZEHM, D., FUDICKAR, W., LINKER, T.: "Molecular Switches Flipped by Oxygen" ANGEW. CHEM. INT. ED., vol. 46, no. 40, 8 October 2007 (2007-10-08), pages 7689-7692, XP002599392
- TANNACI, J. ET AL: "9,10-Disubstituted Octafluoroanthracene Derivatives via Palladium-Catalyzed Cross-Coupling" J. ORG. CHEM., vol. 73, no. 20, 17 October 2008 (2008-10-17), pages 7895-7900, XP002599393
- MATSUBARA, Y. ET AL: "Meso-Disubstituted Anthracenes with Fluorine-Containing Groups: Synthesis, Light-Emitting Characteristics, and Photostability" ORG. LETT., vol. 10, no. 24, 13 November 2008 (2008-11-13), pages 5541-5544, XP002599394
- KOTHA, S., GHOSH, A.K., DEODHAR, K.D.: "Snyhtesis of Symmetrical and Unsymmetrical 9,10-Diarylanthracene Derivatives via Bis-Suzuki-Miyaura Cross-Coupling Reaction" SYNTHESIS, vol. 4, March 2004 (2004-03), pages 549-557, XP002599395

## Description

### FIELD OF THE INVENTION

This invention relates to the production of chemiluminescent light, particularly to the production of blue/violet light within the range of about 390nm to less than 438 nm and most particularly to the use of compounds composed of symmetrically and asymmetrically substituted anthracenes which are effective for increasing the production of such blue/violet light when used as fluorescers in conjunction with chemiluminescent systems.

### BACKGROUND OF THE INVENTION

The principle techniques for the production of chemiluminescent light comprising the reaction of an oxalate solution including a dye with a solution of hydrogen peroxide containing a suitable catalyst have been well described in the following U.S. Pat. Nos. 6,740,263; 5,232,635; 4,717,511; 4,678,608; 3,888,786; 3,775,336; 3,749,679; 3,597,362; 3,557,233; 3,391,068; 3,391,069.

Chemiluminescence is produced by a reaction, in the liquid phase, of an activator such as hydrogen peroxide with a fluorescent agent and an oxalate. Optionally, other secondary compounds can be present such as catalysts, dyes etc. The production of chemiluminescent light by the reaction of a catalyzed hydrogen peroxide solution with a fluorescer solution is well known in the art. Blue, green and yellow chemiluminescent light has been produced depending upon the particular fluorescer employed in the fluorescer solution. Examples of these prior art chemiluminescent light-systems can be found in one or more of the following U.S. Pat. Nos. 3,749,679; 3,391,068; 3,974,368; 3,557,233; 3,597,362; 3,775,336; 3,888,786.

Historically, the light output of blue chemiluminescent devices has been based upon 9,10-diphenylanthracene (DPHA) and 2-chloro-9,10-bis(4-methoxyphenyl)anthracene (BPEN). However, although the light output is measured from 400-500 nm, most of the substituted 9,10-diphenylanthracene derivatives have chemiluminescent emission maxima greater than 445 nm. The most common blue used in chemiluminescent devices is BPEN with an emission maximum of 451 nm. None of the reported non-polymeric substituted diphenylanthracene dyes used for chemiluminescence has a listed emission maximum below DPHA (438 nm). This invention teaches the development of dyes suitable for oxalate/peroxide chemiluminescent systems with a spectrum having an emission maximum at a wavelength below the one observed for DPHA, but at wavelengths greater than the ultra-violet region. This would enable the production of a chemiluminescent system with greater output in the violet region of the visible spectrum, particularly, a chemiluminescent system which produces light having an emission maximum from about 390nm to less than 438 nm without the need for filters to protect the observer from ultraviolet radiation.

### DESCRIPTION OF THE PRIOR ART

United States Patent 3,888,786 to Maulding relates to a chemiluminescent system to obtain chemiluminescent light by reacting an oxalic-type compound of the group consisting of an oxalic-type ester with a hydroperoxide compound in the presence of a solvent and chloro, fluoro, or lower alkyl bis(phenylethynyl)-substituted aromatic compound as a fluorescer. Similarly, U.S. Patents 3,729,426, 3,948,797, 4,017,415 and 5,122,306 teach the use of substitutued anthracene derivatives such as 9,10-bis(phenylethynyl)anthracene, 9,10-bis(4-methoxyphenyl)-2-chloroanthracene, monochloro-substituted 9,10-bis(phenylethynyl)anthracenes such as 1-chloro-9,10-bis(phenylethynyl)anthracene, dichloro-substituted 9,10-bis(phenylethynyl)anthracenes such as 1,8-dichloro-9,10-bis(phenylethynyl)anthracene, 1,5-dichloro-9,10-bis(phenylethynyl)anthracene, 2,3-dichloro-9,10-bis(phenylethynyl)anthracene, 9,10-diphenylanthracene and the like, as suitable fluorescers for chemiluminescent systems. United States Patent 3,970,660 to Bollyky is directed toward a chemiluminescent system comprising (1) derivatives of a polycarbonyl compound substituted with at least one nitrogen-containing hetero group and which may have an alkanol or amine substituent, (2) a hydroperoxide compound, (3) a diluent, and (4) a fluorescer, inclusive of halogen substituted anthracenes, said ingredients in combination producing visible chemiluminescent light and a process of producing such light. United States Patent 4,076,645 to Vega teaches compositions and methods for producing chemiluminescence by the reaction of bis(6-carbopentoxy-2,4,5-trichlorophenyl)oxalate with a peroxide in presence of 9,10-bis(phenylethynyl)anthracene the initial luminosity on mixing these reaction components in a suitable diluent is increased by using the specific concentration of bis-oxalate ester component in the range from 0.05 to 0.09 mole per liter in the reaction mixture.

United States Patent 4,859,369 to Baretz et al, teaches aqueous chemiluminescent systems, inclusive of halogen substituted anthracene fluorescers including those emitting in the blue region of the spectrum chemiluminescent systems, which have been found to exhibit improved chemiluminescence due to the presence of minor amounts of a water-soluble polymer.

United States Patent 5,232,635 to Van Moer et al is directed toward new derivatives of anthracene for the emission of a blue chemiluminescent light. The derivatives are of 9,10-bisphenylanthracene substituted at the 2 position of the anthracene ring and on the phenyl groups including 9,10-bis(4-fluorophenyl)-2-fluoroanthracene. The production of turquoise chemiluminescent light is also disclosed by mixing said derivatives with a green 9,10-bis(phenylethynyl) anthracene.

United States Patent 6,740,263 to Park et al describes an anthracene and chemiluminescent compositions as shown below. Wherein, R is an alkyl group having 1-8 carbon atoms, and X¹ and X² are independently hydrogen or a halogen.

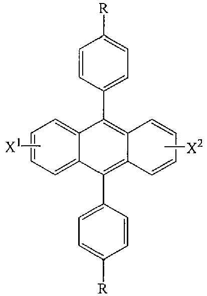

The disclosure of Park et al relates to an anthracene compound and a chemiluminescent composition comprising the compound, and more particularly to a novel anthracene compound, which is capable of emitting a blue light of a high intensity for a prolonged period of time compared with conventional anthracene compounds used as luminescent dyes in chemiluminescent compositions emitting blue light, and a chemiluminescent composition comprising the anthracene compound.

US 2008/308776 relates to chemiluminescent compositions, and methods for producing such, that can be applied to a variety of substrates that are generally sensitive to existing chemiluminescent compositions.

US 2005/224768 relates to a chemiluminescent composition producing white light.

US 5597517 relates to a chemiluminescent composition comprising an oxalate component comprising an oxalate ester and a solvent, an activator component comprising a peroxide compound and a catalyst and a fluorescer contained in the oxalate component and/or the activator component.

The above referenced prior art disclosures fail to teach or suggest the novel compounds as instantly disclosed, nor do they teach a chemiluminescent system which produces light having an emission maximum from about 390nm to less than 438 nm.

### SUMMARY OF THE INVENTION

It is a primary objective of the instant invention to teach new species of fluoro-substituted 9,10-diphenylanthracenes including symmetrically and/or asymmetrically substituted constituents.

It is a further objective of the instant invention to provide a composition comprising an oxalate solution and an anthracene compound represented by Formula (1): 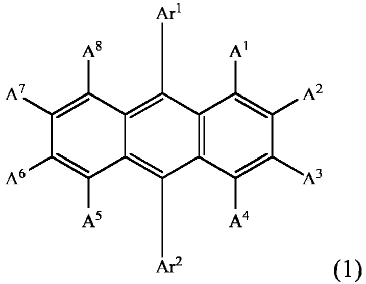 wherein Ar¹ has a structure represented by Formula 2, where An shows the attachment to the anthracene ring as described in Formula 1,
Ar¹ = 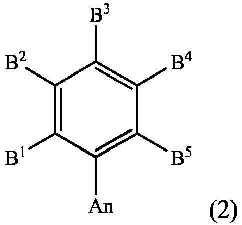 wherein Ar² has a structure represented by Formula 3, where An shows the attachment to the anthracene ring as described in Formula 1,
Ar² = 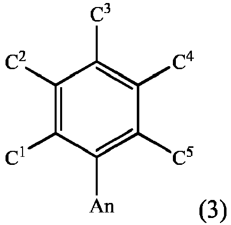 wherein each of A¹, A², A³, A⁴, A⁵, A⁶, A⁷, and A⁸ are selected from the group consisting of hydrogen and fluorine;
wherein at least two of the substituents, B¹, B², B³, B⁴, and B⁵, as shown in Formula 2, are not hydrogen, wherein each of B¹, B², B⁴, and B⁵ are selected from the group consisting of hydrogen and fluorine, and B³ is selected from the group consisting of hydrogen, fluorine, trifluoromethyl, and an alkoxy group with 1-10 carbons; and
wherein at least two of the substituents, C¹, C², C³, C⁴, and C⁵, as shown in Formula 3, are not hydrogen, wherein each of C¹, C², C⁴, and C⁵ are selected from the group consisting of hydrogen and fluorine, and C³ is selected from the group consisting of hydrogen, fluorine, trifluoromethyl, and an alkoxy group with 1-10 carbons,
wherein the composition is for reacting with a hydrogen peroxide solution to provide a chemiluminescent system that produces a blue/violet chemiluminescent light in the region where the emission maximum is from 390 nm to less than 438 nm.

Fluorescer compounds based upon symmetrical and asymmetrical 9,10-diphenylanthracene derivatives or Formula (1) are provided, where the substituents on the anthracene labeled A¹, A², A³, A⁴, A⁵, A⁶, A⁷, and A⁸ are either fluorine or hydrogen.

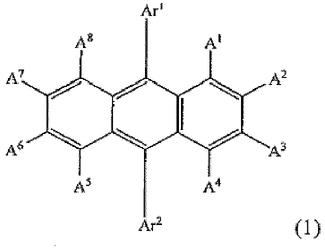

Furthermore, if Ar¹ and Ar² are the same, at least two of the substituents, B¹, B², B³, B⁴, and B⁵, as shown in Figure (2), are not hydrogen, wherein B¹, B², B⁴, and B⁵ are either hydrogen or fluorine and B³ is either hydrogen, fluorine, trifluoromethyl, or an alkoxy group with 1-10 carbons.
Ar¹ =

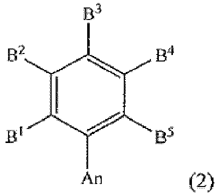

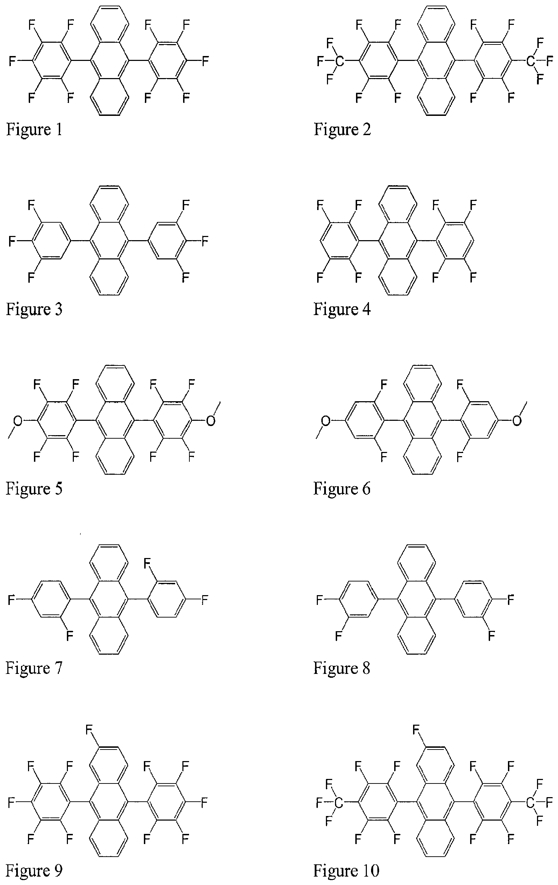

Additionally, if Ar¹ and Ar² are not the same, Ar¹ will meet the conditions described above and the second ring, as shown in Figure 3, may be completely substituted with hydrogen or wherein at least one of the substituents, C¹, C², C³, C⁴, and C⁵ which are not hydrogen, wherein C¹, C², C⁴, and C⁵ are either hydrogen or fluorine and C³ is either hydrogen, fluorine, trifluoromethyl, or an alkoxy group with 1-10 carbons.
Ar² =

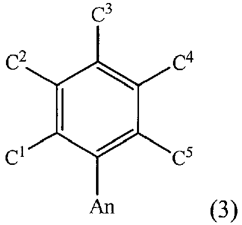

It is a further objective of the instant invention to provide a chemiluminescent system comprising the composition of the invention, and a hydrogen peroxide solution, wherein the chemiluminescent system is for producing a blue/violet chemiluminescent light in the region where the emission maximum is from 390 nm to less then 438 nm, and in a more specific embodiment, a chemiluminescent system which produces light having an emission maximum in the region from about 400 nm to about 420 nm.

In accordance with an embodiment of the present invention, the oxalate compound is a bis(phenyl) oxalate ester having the formula: 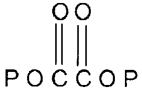 in which the phenyl groups(P) are substituted by at least one carbalkoxy group of the formula 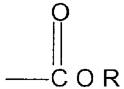 in which R is (a) an alkyl group (1-18 carbons, straight chain, branched chain, cyclic) or (b) a substituted alkyl group, where said substituents are selected from the group consisting of fluoro, chloro, trifluoromethyl, alkoxy, cyano, carbalkoxy, and phenyl; and in which (2) the phenyl groups P, are substituted by at least two additional substituents selected from the group consisting of fluoro, chloro, bromo, cyano, trifluoromethyl, carbalkoxy, nitro, alkoxy, alkoxy methyl, methyl, and higher alkyl, said additional substituents being selected so that the sum of their Hammett sigma constants for phenols lies between 1.0 and 2.

In a preferred embodiment the oxalate is bis(2,4,5-trichloro-6-carbopentoxyphenyl)oxalate.

### BRIEF DESCRIPTION OF THE FIGURES

As follows, Figures 1-88 illustrate anthracene derivatives with the same or symmetrically substituted aryl rings in the 9 and 10 positions, and Figures 89 - 660 illustrate anthracene derivatives with different or asymmetric aryl rings in the 9 and 10 positions.
Figure 1 illustrates a 9,10-bis(2,3,4,5,6-pentafluorophenyl)anthracene;
Figure 2 illustrates a 9,10-bis[2,3,5,6-tetrafluoro-4-(trifluoromethyl)phenyl]anthracene;
Figure 3 illustrates a 9,10-bis(3,4,5-trifluorophenyl)anthracene
Figure 4 illustrates a 9,10-bis(2,3,5,6-tetrafluorophenyl)anthracene;
Figure 5 illustrates a 9,10-bis(2,3,5,6-tetrafluoro-4-methoxypheny)anthracene;
Figure 6 illustrates a 9,10-bis(2,6-difluoro-4-methoxyphenyl)anthracene;
Figure 7 illustrates a 9,10-bis(2,4-difluorophenyl)anthracene
Figure 8 illustrates a 9,10-bis(3,4-difluorophenyl)anthracene;
Figure 9 illustrates a 2-fluoro-9,10-bis(2,3,4,5,6-pentafluorophenyl)anthracene;
Figure 10 illustrates a 2-fluoro-9,10-bis[2,3,5,6-tetrafluoro-4-(trifluoromethyl)phenyl] anthracene;
Figure 11 illustrates a 2-fluoro-9,10-bis(3,4,5-trifluorophenyl)anthracene
Figure 12 illustrates a 2-fluoro-9,10-bis(2,3,5,6-tetrafluorophenyl)anthracene;
Figure 13 illustrates a 2-fluoro-9,10-bis(2,3,5,6-tetrafluoro-4-methoxyphenyl)anthracene;
Figure 14 illustrates a 9,10-bis(2,6-difluoro-4-methoxyphenyl)-2-fluoroanthracene;
Figure 15 illustrates a 9,10-bis(2,4-difluorophenyl)-2-fluoroanthracene;
Figure 16 illustrates a 9,10-bis(3,4-difluorophenyl)-2-fluoroanthracene anthracene;
Figure 17 illustrates a 1-fluoro-9,10-bis(2,3,4,5,6-pentafluorophenyl) anthracene;
Figure 18 illustrates a 1-fluoro-9,10-bis[2,3,5,6-tetrafluoro-4-(trifluoromethyl)phenyl]anthracene;
Figure 19 illustrates a 1-fluoro-9,10-bis(3,4,5-trifluorophenyl)anthracene
Figure 20 illustrates a 1-fluoro-9,10-bis(2,3,5,6-tetrafluorophenyl)anthracene;
Figure 21 illustrates a 1-fluoro-9,10-bis(2,3,5,6-tetrafluoro-4-methoxyphenyl)anthracene;
Figure 22 illustrates a 9,10-bis(2,6-difluoro-4-methoxyphenyl)-1-fluoroanthracene;
Figure 23 illustrates a 9,10-bis(2,4-difluorophenyl)1-fluoroanthracene;
Figure 24 illustrates a 9,10-bis(3,4-difluorophenyl)1-fluoroanthracene;
Figure 25 illustrates a 1,4-difluoro-9,10-bis(2,3,4,5,6-pentafluorophenyl)anthracene;
Figure 26 illustrates a 1,4-difluoro-9,10-bis[2,3,5,6-tetrafluoro-4-(trifluoromethyl)phenyl]anthracene;
Figure 27 illustrates a 1,4-difluoro-9,10-bis(3,4,5-trifluorophenyl)anthracene;
Figure 28 illustrates a 1,4-difluoro-9,10-bis(2,3,5,6-tetrafluorophenyl)anthracene;
Figure 29 illustrates a 1,4-difluoro-9,10-bis(2,3,5,6-tetrafluoro-4-methoxyphenyl)anthracene;
Figure 30 illustrates a 9,10-bis(2,6-difluoro-4-methoxyphenyl)-1,4-difluoroanthracene;
Figure 31 illustrates a 9,10-bis(2,4-difluorophenyl)-1,4-difluoroanthracene;
Figure 32 illustrates a 9,10-bis(3,4-difluorophenyl)-1,4-difluoroanthracene;
Figure 33 illustrates a 2,3-difluoro-9,10-bis(2,3,4,5,6-pentafluorophenyl)anthracene;
Figure 34 illustrates a 2,3-difluoro-9,10-bis[2,3,5,6-tetrafluoro-4-(trifluoromethyl)phenyl]anthracene;
Figure 35 illustrates a 2,3-difluoro-9,10-bis(3,4,5-trifluorophenyl)anthracene;
Figure 36 illustrates a 2,3-difluoro-9,10-bis(2,3,5,6-tetrafluorophenyl) anthracene;
Figure 37 illustrates a 2,3-difluoro9,10-bis(2,3,5,6-tetrafluoro-4-methoxyphenyl)anthracene;
Figure 38 illustrates a 9,10-bis(2,6-difluoro-4-methoxyphenyl)-2,3-difluoroanthracene;
Figure 39 illustrates a 9,10-bis(2,4-difluorophenyl)-2,3-difluoroanthracene;
Figure 40 illustrates a 9,10-bis(3,4-difluorophenyl)-2,3-difluoroanthracene;
Figure 41 illustrates a 1,4,5-trifluoro-9,10-bis(2,3,4,5,6-pentafluorophenyl)anthracene;
Figure 42 illustrates a 1,4,5-trifluoro-9,10-bis[2,3,5,6-tetrafluoro-4-(trifluoromethyl)phenyl]anthracene;
Figure 43 illustrates a 1,4,5-trifluoro-9,10-bis(3,4,5-trifluorophenyl)anthracene;
Figure 44 illustrates a 1,4,5-trifluoro-9,10-bis(2,3,5,6-tetrafluorophenyl)anthracene;
Figure 45 illustrates a 1,4,5-trifluoro-9,10-bis(2,3,5,6-tetrafluoro-4-methoxyphenyl)anthracene;
Figure 46 illustrates a 9,10-bis(2,6-difluoro-4-methoxyphenyl)-1,4,5-trifluoroanthracene;
Figure 47 illustrates a 9,10-bis(2,4-difluorophenyl)-1,4,5trifluoroanthracene;
Figure 48 illustrates a 9,10-bis(3,4-difluorophenyl)-1,4,5-trifluoroanthracene;
Figure 49 illustrates a 1,4,6-trifluoro-9,10-bis(2,3,4,5,6-pentafluorophenyl)anthracene;
Figure 50 illustrates a 1,4,6-trifluoro-9,10-bis[2,3,5,6-tetrafluoro-4-(trifluoromethyl)phenyl]anthracene;
Figure 51 illustrates a 1,4,6-trifluoro-9,10-bis(3,4,5-trifluorophenyl)anthracene;
Figure 52 illustrates a 1,4,6-trifluoro-9,10-bis(2,3,5,6-tetrafluorophenyl)anthracene;
Figure 53 illustrates a 1,4,6-trifluoro-9,10-bis(2,3,5,6-tetrafluoro-4-methoxyphenyl)anthracene;
Figure 54 illustrates a 9,10-bis(2,6-difluoro-4-methoxyphenyl)-1,4,6-trifluoroanthracene;
Figure 55 illustrates a 9,10-bis(2,4-difluorophenyl)-1,4,6-trifluoroanthracene;
Figure 56 illustrates a 9,10-bis(3,4-difluorophenyl)-1,4,6-trifluoroanthracene;
Figure 57 illustrates a 1,2,3,4-tetrafluoro-9,10-bis(2,3,4,5,6-pentafluorophenyl)anthracene;
Figure 58 illustrates a 1,2,3,4-tetrafluoro-9,10-bis[2,3,5,6-tetrafluoro-4-(trifluoromethyl)phenyl] anthracene;
Figure 59 illustrates a 1,2,3,4-tetrafluoro-9,10-bis(3,4,5-trifluorophenyl)anthracene
Figure 60 illustrates a 1,2,3,4-tetrafluoro-9,10-bis(2,3,5,6-tetrafluorophenyl)anthracene;
Figure 61 illustrates a 1,2,3,4-tetrafluoro-9,10-bis(2,3,5,6-tetrafluoro-4-methoxyphenyl)anthracene;
Figure 62 illustrates a 9,10-bis(2,6-difluoro-4-methoxyphenyl)-1,2,3,4-tetrafluoroanthracene;
Figure 63 illustrates a 9,10-bis(2,4-difluorophenyl)-1,2,3,4-tetrafluoroanthracene;
Figure 64 illustrates a 9,10-bis(3,4-difluorophenyl)-1,2,3,4-tetrafluoroanthracene;
Figure 65 illustrates a 1,2,3,4,5-pentafluoro-9,10-bis(2,3,4,5,6-pentafluorophenyl)anthracene;
Figure 66 illustrates a 1,2,3,4,5-pentafluoro-9,10-bis[2,3,5,6-tetrafluoro-4-(trifluoromethyl)phenyl]anthracene;
Figure 67 illustrates a 1,2,3,4,5-pentafluoro-9,10-bis(3,4,5-trifluorophenyl)anthracene
Figure 68 illustrates a 1,2,3,4,5-pentafluoro-9,10-bis(2,3,5,6-tetrafluorophenyl)anthracene;
Figure 69 illustrates a 1,2,3,4,5-pentafluoro-9,10-bis(2,3,5,6-tetrafluoro-4-methoxyphenyl)anthracene;
Figure 70 illustrates a 9,10-bis(2,6-difluoro-4-methoxyphenyl)-1,2,3,4,5-pentafluoroanthracene;
Figure 71 illustrates a 9,10-bis(2,4-difluorophenyl)-1,2,3,4,5-pentafluoroanthracene;
Figure 72 illustrates a 9,10-bis(3,4-difluorophenyl)-1,2,3,4,5-pentafluoroanthracene;
Figure 73 illustrates a 1,2,3,4,6-pentafluoro-9,10-bis(2,3,4,5,6-pentafluorophenyl)anthracene;
Figure 74 illustrates a 1,2,3,4,6-pentafluoro-9,10-bis[2,3,5,6-tetrafluoro-4-(trifluoromethyl)phenyl]anthracene;
Figure 75 illustrates a 1,2,3,4,6-pentafluoro-9,10-bis(3,4,5-trifluorophenyl)anthracene:
Figure 76 illustrates a 1,2,3,4,6-pentafluoro-9,10-bis(2,3,5,6-tetrafluorophenyl)anthracene;
Figure 77 illustrates a 1,2,3,4,6-pentafluoro-9,10-bis(2,3,5,6-tetrafluoro-4-methoxyphenyl)anthracene;
Figure 78 illustrates a 9,10-bis(2,6-difluoro-4-methoxyphenyl)-1,2,3,4,6-pentafluoroanthracene;
Figure 79 illustrates a 9,10-bis(2,4-difluorophenyl)-1,2,3,4,6-pentafluoroanthracene;
Figure 80 illustrates a 9,10-bis(3,4-difluorophenyl)-1,2,3,4,6-pentafluoroanthracene;
Figure 81 illustrates a 1,2,3,4,5,6,7,8-octafluoro-9,10-bis(2,3,4,5,6-pentafluorophenyl)anthracene;
Figure 82 illustrates a 1,2,3,4,5,6,7,8-octafluoro-9,10-bis[2,3,5,6-tetrafluoro-4-(trifluoromethyl)phenyl]anthracene;
Figure 83 illustrates a 1,2,3,4,5,6,7,8-octafluoro-9,10-bis(3,4,5-trifluorophenyl)anthracene
Figure 84 illustrates a 1,2,3,4,5,6,7,8-octafluoro-9,10-bis(2,3,5,6-tetrafluorophenyl)anthracene;
Figure 85 illustrates a 1,2,3,4,5,6,7,8-octafluoro-9,10-bis(2,3,5,6-tetrafluoro-4-methoxyphenyl)anthracene;
Figure 86 illustrates a 9,10-bis(2,6-difluoro-4-methoxyphenyl)-1,2,3,4,5,6,7,8-octafluoroanthracene;
Figure 87 illustrates a 9,10-bis(2,4-difluorophenyl)-1,2,3,4,5,6,7,8-octafluoroanthracene;
Figure 88 illustrates a 9,10-bis(3,4-difluorophenyl)-1,2,3,4,5,6,7,8-octafluoroanthracene;
Figure 89 illustrates a 9-(2,3,4,5,6-pentafluorophenyl)-10-phenylanthracene;
Figure 90 illustrates a 9-(4-methoxyphenyl)-10-(2,3,4,5,6-pentafluorophenyl)anthracene;
Figure 91 illustrates a 9-(2,3,4,5,6-pentafluorophenyl)-10-(2,3,5,6-tetrafluoropheny)lanthracene;
Figure 92 illustrates a 9-(2,3,4,5,6-pentafluorophenyl)-10-(2,3,5,6-tetrafluoro-4-methoxyphenyl)anthracene;

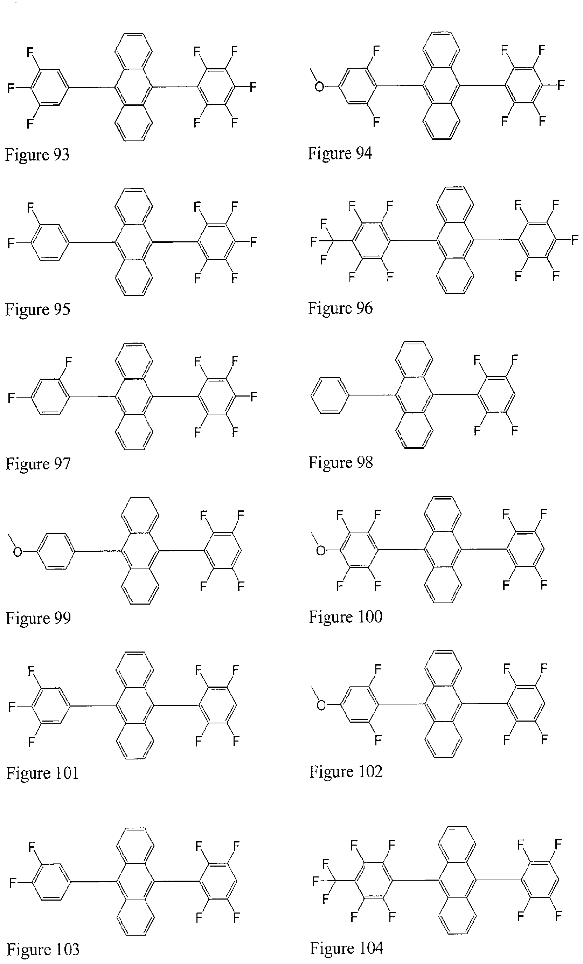

Figure 93 illustrates a 9-(2,3,4,5,6-pentafluorophenyl)-10-(3,4,5-trifluoro)anthracene;
Figure 94 illustrates a 9-(2,6-difluoro-4-methoxyphenyl)-10-(2,3,4,5,6-pentafluorophenyl)anthracene
Figure 95 illustrates a 9-(3,4-difluorophenyl)-10-(2,3,4,5,6-pentafluorophenyl)anthracene;
Figure 96 illustrates a 9-(2,3,4,5,6-pentafluorophenyl)-10-[2,3,5,6-tetrafluoro-4-(trifluoromethyl)phenyl]anthracene;
Figure 97 illustrates a 9-(2,4-difluorophenyl)-10-(2,3,4,5,6-pentafluorophenyl)anthracene;
Figure 98 illustrates a 9-phenyl-10-(2,3,5,6-tetrafluorophenyl)anthracene;
Figure 99 illustrates a 9-(4-methoxyphenyl)-10-(2,3,5,6-tetrafluorophenyl)anthracene;
Figure 100 illustrates a 9-(2,3,5,6-tetrafluorophenyl)-10-(2,3,5,6-tetrafluoro-4-methoxyphenyl)anthracene;
Figure 101 illustrates a 9-(2,3,5,6-tetrafluorophenyl)-10-(3,4,5-trifluorophenyl)anthracene;
Figure 102 illustrates a 9-(2,6-difluoro-4-methoxyphenyl)-10-(2,3,5,6-tetrafluorophenyl)anthracene;
Figure 103 illustrates a 9-(3,4-difluorophenyl)-10-(2,3,5,6-tetrafluorophenyl)anthracene;
Figure 104 illustrates a 9-[2,3,5,6-tetrafluoro-4-(trifluoromethyl)phenyl]-10-(2,3,,5,6-tetrafluorophenyl)anthracene;
Figure 105 illustrates a 9-(3,4-difluorophenyl)-10-(2,3,5,6-tetrafluorophenyl)anthracene;
Figure 106 illustrates a 9-phenyl-10-(2,3,5,6-tetrafluoro-4-methoxyphenyl)anthracene;
Figure 107 illustrates a 9-(4-methoxyphenyl)-10-(2,3,5,6-tetrafluoro-4-methoxyphenyl)anthracene;
Figure 108 illustrates a 9-(2,3,5,6-tetrafluoro-4-methoxyphenyl)-10-(3,4,5-trifluorophenyl)anthracene;
Figure 109 illustrates a 9-(2,6-difluoro-4-methoxyphenyl)-10-(2,3,5,6-tetrafluoro-4-methoxyphenyl)anthracene;
Figure 110 illustrates a 9-(3,4-difluorophenyl)-10-(2,3,5,6-tetrafluoro-4-methoxyphenyl)anthracene;
Figure 111 illustrates a 9-[2,3,5,6-tetrafluoro-4-(trifluoromethyl)phenyl]-10-(2,3,5,6-tetrafluoro-4-methoxyphenyl)anthracene;
Figure 112 illustrates a 9-(2,4-difluorophenyl)-10-(2,3,5,6-tetrafluoro-4-methoxyphenyl)anthracene;
Figure 113 illustrates a 9-phenyl-10-(3,4,5-trifluorophenyl)anthracene;
Figure 114 illustrates a 9-(4-methoxyphenyl)-10-(3,4,5-trifluorophenyl)anthracene;
Figure 115 illustrates a 9-(2,6-difluoro-4-methoxyphenyl)-10-(3,4,5-trifluorophenyl)anthracene;
Figure 116 illustrates a 9-(3,4-difluorophenyl)-10-(3,4,5-trifluorophenyl)anthracene;

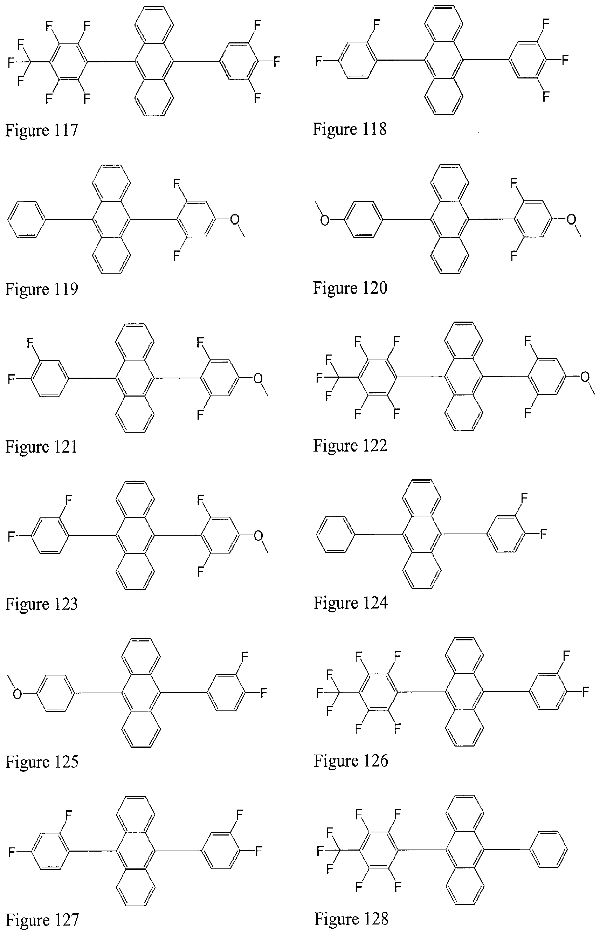

Figure 117 illustrates a 9-[2,3,5,6-tetrafluoro-4-(trifluoromethyl)phenyl]-10-(3,4,5-trifluorophenyl)anthracene;
Figure 118 illustrates a 9-(2,4-difluorophenyl)-10-(3,4,5-trifluorophenyl)anthracene;
Figure 119 illustrates a 9-(2,6-difluoro-4-methoxyphenyl)-10-phenyl anthracene:
Figure 120 illustrates a 9-(2,6-difluoro-4-methoxyphenyl)-10-(4-methoxyphenyl)anthracene:
Figure 121 illustrates a 9-(2,6-difluoro-4-methoxyphenyl)-10-(3,4-difluorophenyl)anthracene;
Figure 122 illustrates a 9-(2,6-difluoro-4-methoxyphenyl)-10-[2,3,5,6-tetrafluoro-4-(trifluoromethyl)phenyl]anthracene;
Figure 123 illustrates a 9-(2,6-difluoro-4-methoxyphenyl)-10-(2,4-difluorophenyl)anthracene;
Figure 124 illustrates a 9-(3,4-difluorophenyl)-10-phenylanthracene;
Figure 125 illustrates a 9-(3,4-difluorophenyl)-10-(4-methoxyphenyl)anthracene;
Figure 126 illustrates a 9-(3,4-difluorophenyl)-10-[2,3,5,6-tetrafluoro-4-(trifluoromethyl)phenyl]anthracene;
Figure 127 illustrates a 9-(2,4-difluorophenyl)-10-(3,4-difluorophenyl)anthracene;
Figure 128 illustrates a 9-phenyl-10-[2,3,5,6-tetrafluoro-4-(trifluoromethyl)phenyl]anthracene;

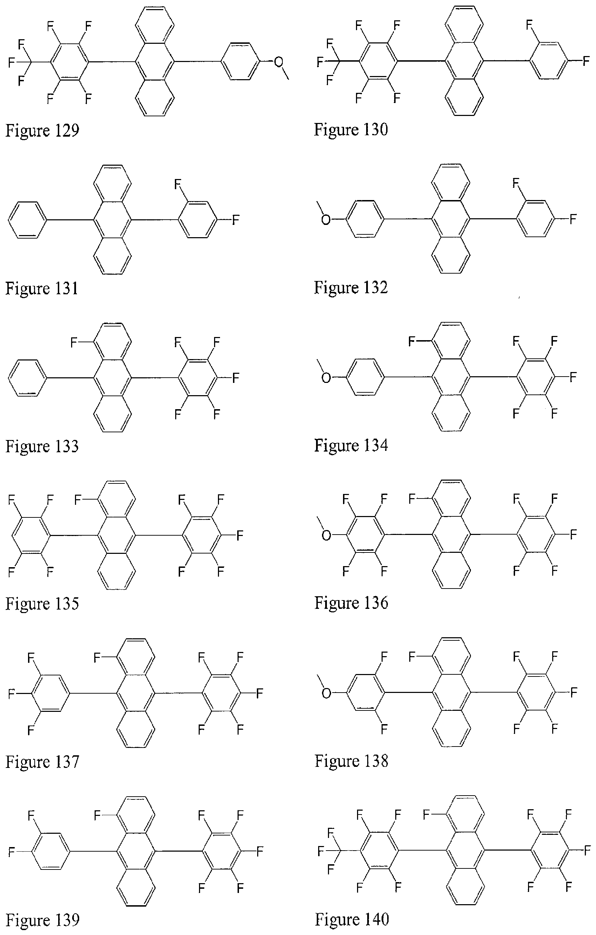

Figure 129 illustrates a 9-(4-methoxyphenyl)-10-[2,3,5,6-tetrafluoro-4-(trifluoromethyl)phenyl]anthracene;
Figure 130 illustrates a 9-(2,4-difluorophenyl)-10-[2,3,5,6-tetrafluoro-4-(trifluoromethyl)phenyl]anthracene;
Figure 131 illustrates a 9-(2,4-difluorophenyl)-10-phenylanthracene;
Figure 132 illustrates a 9-(2,4-difluorophenyl)-10-(4-methoxyphenyl)anthracene;
Figure 133 illustrates a 1-fluoro-9-(2,3,4,5,6-pentafluorophenyl)-10-phenylanthracene;
Figure 134 illustrates a 1-fluoro-9-(4-methoxyphenyl)-10-(2,3,4,5,6-pentafluorophenyl)anthracene;
Figure 135 illustrates a 1-fluoro-9-(2,3,4,5,6-pentafluorophenyl)-10-(2,3,5,6-tetrafluoropheny)lanthracene;
Figure 136 illustrates a 1-fluoro-9-(2,3,4,5,6-pentafluorophenyl)-10-(2,3,5,6-tetrafluoro-4-methoxyphenyl)anthracene;
Figure 137 illustrates a 1-fluoro-9-(2,3,4,5,6-pentafluorophenyl)-10-(3,4,5-trifluoro)anthracene;
Figure 138 illustrates a 9-(2,6-difluoro-4-methoxyphenyl)-1-fluoro-10-(2,3,4,5,6-pentafluorophenyl)anthracene
Figure 139 illustrates a 9-(3,4-difluorophenyl)-1-fluoro-10-(2,3,4,5,6-pentafluorophenyl)anthracene;
Figure 140 illustrates a 1-fluoro-9-(2,3,4,5,6-pentafluorophenyl)-10-[2,3,5,6-tetrafluoro-4-(trifluoromethyl)phenyl]anthracene;

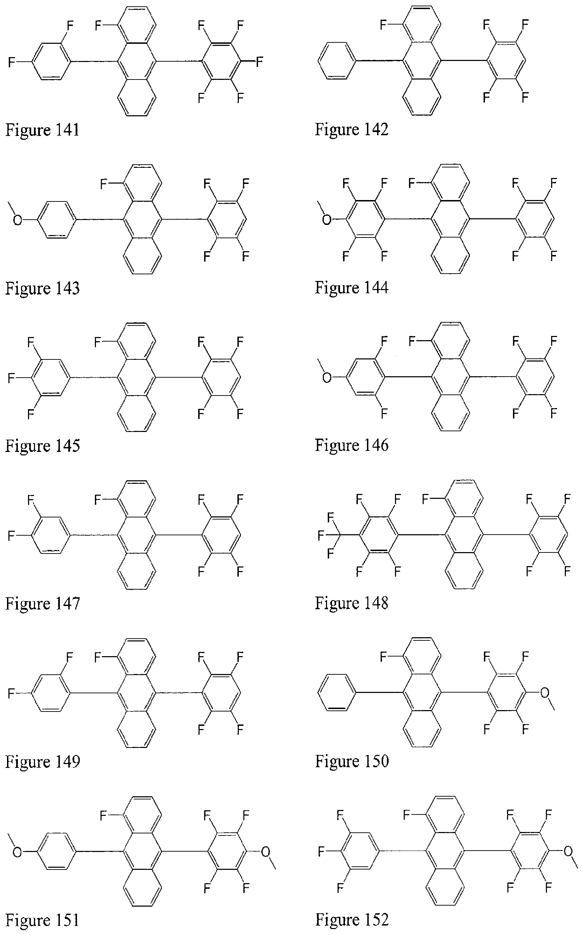

Figure 141 illustrates a 9-(2,4-difluorophenyl)-1-fluoro-10-(2,3,4,5,6-pentafluorophenyl)anthracene;
Figure 142 illustrates a 1-fluoro-9-phenyl-10-(2,3,5,6-tetrafluorophenyl)anthracene;
Figure 143 illustrates a 1-fluoro-9-(4-methoxyphenyl)-10-(2,3,5,6-tetrafluorophenyl)anthracene;
Figure 144 illustrates a 1-fluoro-9-(2,3,5,6-tetrafluorophenyl)-10-(2,3,5,6-tetrafluoro-4-methoxyphenyl)anthracene;
Figure 145 illustrates a 1-fluoro-9-(2,3,5,6-tetrafluorophenyl)-10-(3,4,5-trifluorophenyl)anthracene;
Figure 146 illustrates a 9-(2,6-difluoro-4-methoxyphenyl)-1-fluoro-10-(2,3,5,6-tetrafluorophenyl)anthracene;
Figure 147 illustrates a 9-(3,4-difluorophenyl)-1-fluoro-10-(2,3,5,6-tetrafluorophenyl)anthracene;
Figure 148 illustrates a 1-fluoro-9-[2,3,5,6-tetrafluoro-4-(trifluoromethyl)phenyl]-10-(2,3,,5,6-tetrafluorophenyl)anthracene;
Figure 149 illustrates a 9-(3,4-difluorophenyl)-1-fluoro-10-(2,3,5,6-tetrafluorophenyl)anthracene;
Figure 150 illustrates a 1-fluoro-9-phenyl-10-(2,3,5,6-tetrafluoro-4-methoxyphenyl)anthracene;
Figure 151 illustrates a 1-fluoro-9-(4-methoxyphenyl)-10-(2,3,5,6-tetrafluoro-4-methoxyphenyl)anthracene;
Figure 152 illustrates a 1-fluoro-9-(2,3,5,6-tetrafluoro-4-methoxyphenyl)-10-(3,4,5-trifluorophenyl)anthracene;

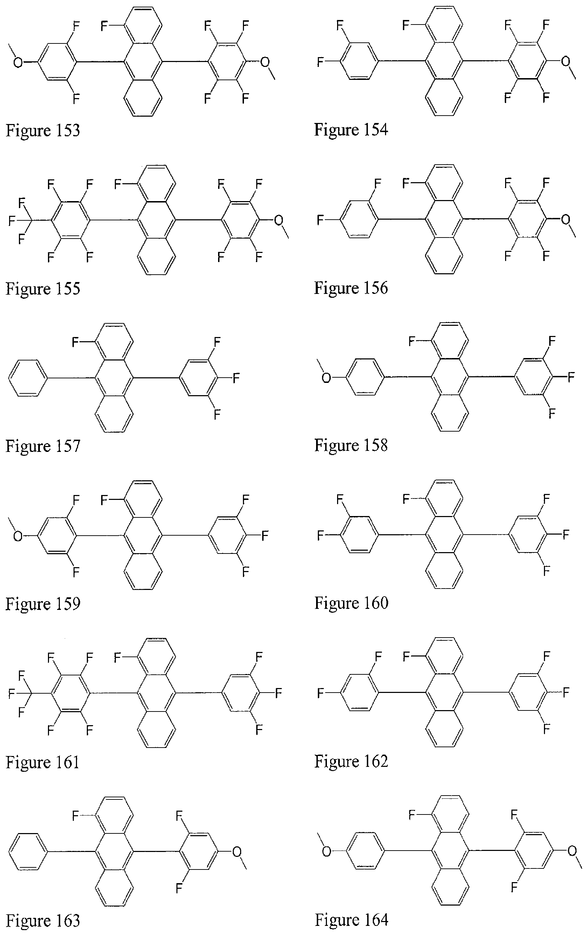

Figure 153 illustrates a 9-(2,6-difluoro-4-methoxyphenyl)-1-fluoro-10-(2,3,5,6-tetrafluoro-4-methoxyphenyl)anthracene;
Figure 154 illustrates a 9-(3,4-difluorophenyl)-1-fluoro-10-(2,3,5,6-tetrafluoro-4-methoxyphenyl)anthracene;
Figure 155 illustrates a 1-fluoro-9-[2,3,5,6-tetrafluoro-4-(trifluoromethyl)phenyl]-10-(2,3,5,6-tetrafluoro-4-methoxyphenyl)anthracene;
Figure 156 illustrates a 9-(2,4-difluorophenyl)-1-fluoro-10-(2,3,5,6-tetrafluoro-4-methoxyphenyl)anthracene;
Figure 157 illustrates a 1-fluoro-9-phenyl-10-(3,4,5-trifluorophenyl)anthracene;
Figure 158 illustrates a 1-fluoro-9-(4-methoxyphenyl)-10-(3,4,5-trifluorophenyl)anthracene;
Figure 159 illustrates a 9-(2,6-difluoro-4-methoxyphenyl)-1-fluoro-10-(3,4,5-trifluorophenyl)anthracene;
Figure 160 illustrates a 9-(3,4-difluorophenyl)-1-fluoro-10-(3,4,5-trifluorophenyl)anthracene;
Figure 161 illustrates a 1-fluoro-9-[2,3,5,6-tetrafluoro-4-(trifluoromethyl)phenyl]-10-(3,4,5-trifluorophenyl)anthracene;
Figure 162 illustrates a 9-(2,4-difluorophenyl)-1-fluoro-10-(3,4,5-trifluorophenyl)anthracene;
Figure 163 illustrates a 9-(2,6-difluoro-4-methoxyphenyl)-1-fluoro-10-phenyl anthracene:
Figure 164 illustrates a 9-(2,6-difluoro-4-methoxyphenyl)-1-fluoro-10-(4-methoxyphenyl)anthracene:

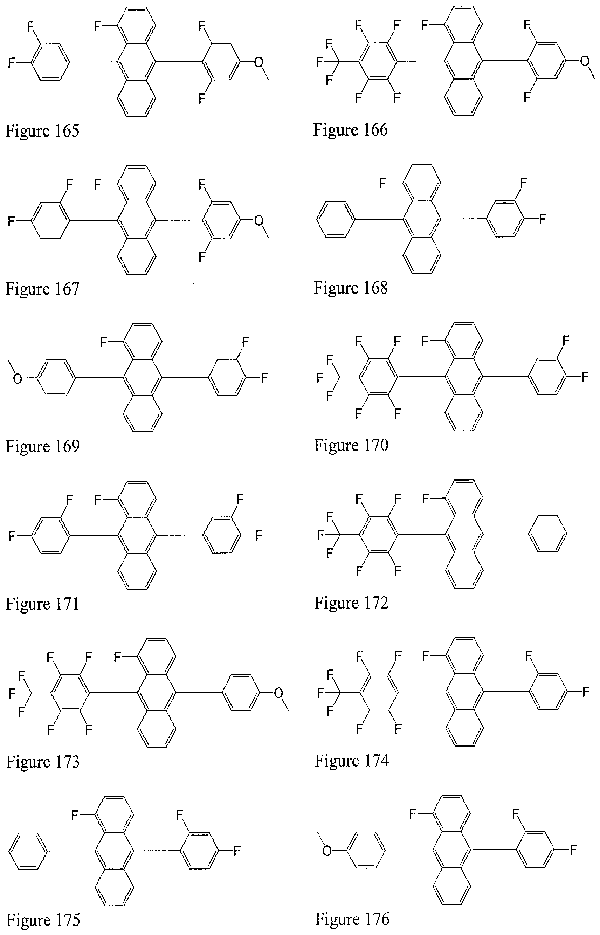

Figure 165 illustrates a 9-(2,6-difluoro-4-methoxyphenyl)-10-(3,4-difluorophenyl)-1-fluoroanthracene;
Figure 166 illustrates a 9-(2,6-difluoro-4-methoxyphenyl)-1-fluoro-10-[2,3,5,6-tetrafluoro-4-(trifluoromethyl)phenyl] anthracene;
Figure 167 illustrates a 9-(2,6-difluoro-4-methoxyphenyl)-1-fluoro-10-(2,4-difluorophenyl)anthracene;
Figure 168 illustrates a 9-(3,4-difluorophenyl)-1-fluoro-10-phenylanthracene;
Figure 169 illustrates a 9-(3,4-difluorophenyl)-1-fluoro-10-(4-methoxyphenyl)anthracene;
Figure 170 illustrates a 9-(3,4-difluorophenyl)-1-fluoro-10-[2,3,5,6-tetrafluoro-4-(trifluoromethyl)phenyl]anthracene;
Figure 171 illustrates a 9-(2,4-difluorophenyl)-1-fluoro-10-(3,4-difluorophenyl)anthracene;
Figure 172 illustrates a 1-fluoro-9-phenyl-10-[2,3,5,6-tetrafluoro-4-(trifluoromethyl)phenyl]anthracene;
Figure 173 illustrates a 1-fluoro-9-(4-methoxyphenyl)-10-[2,3,5,6-tetrafluoro-4-(trifluoromethyl)phenyl]anthracene;
Figure 174 illustrates a 9-(2,4-difluorophenyl)-1-fluoro-10-[2,3,5,6-tetrafluoro-4-(trifluoromethyl)phenyl]anthracene;
Figure 175 illustrates a 9-(2,4-difluorophenyl)-1-fluoro-10-phenylanthracene;
Figure 176 illustrates a 9-(2,4-difluorophenyl)-1-fluoro-10-(4-methoxyphenyl)anthracene;

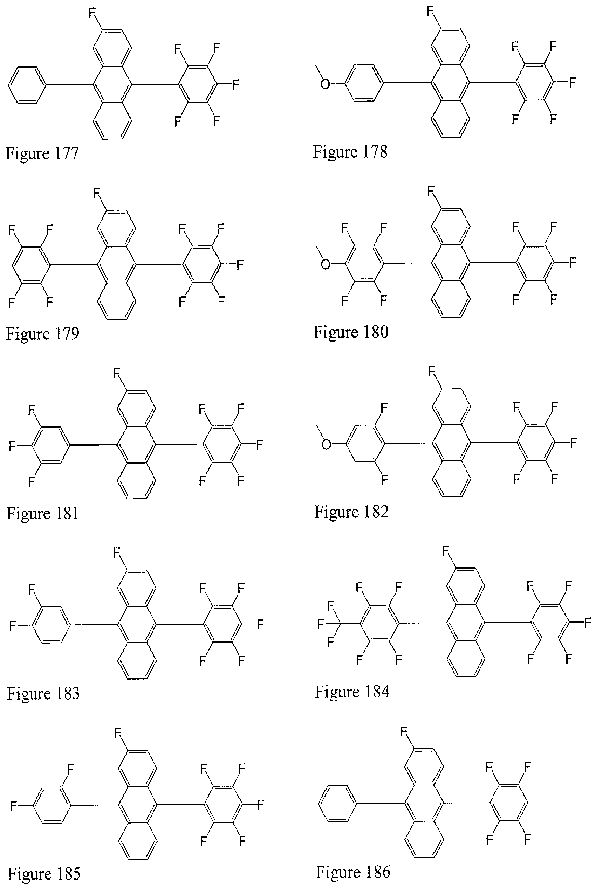

Figure 177 illustrates a 2-fluoro-9-(2,3,4,5,6-pentafluorophenyl)-10-phenylanthracene;
Figure 178 illustrates a 2-fluoro-9-(4-methoxyphenyl)-10-(2,3,4,5,6-pentafluorophenyl)anthracene;
Figure 179 illustrates a 2-fluoro-9-(2,3,4,5,6-pentafluorophenyl)-10-(2,3,5,6-tetrafluoropheny)lanthracene;
Figure 180 illustrates a 2-fluoro-9-(2,3,4,5,6-pentafluorophenyl)-10-(2,3,5,6-tetrafluoro-4-methoxyphenyl)anthracene;
Figure 181 illustrates a 2-fluoro-9-(2,3,4,5,6-pentafluorophenyl)-10-(3,4,5-trifluoro)anthracene;
Figure 182 illustrates a 9-(2,6-difluoro-4-methoxyphenyl)-2-fluoro-10-(2,3,4,5,6-pentafluorophenyl)anthracene
Figure 183 illustrates a 9-(3,4-difluorophenyl)-2-fluoro-10-(2,3,4,5,6-pentafluorophenyl)anthracene;
Figure 184 illustrates a 2-fluoro-9-(2,3,4,5,6-pentafluorophenyl)-10-[2,3,5,6-tetrafluoro-4-(trifluoromethyl)phenyl]anthracene;
Figure 185 illustrates a 9-(2,4-difluorophenyl)-2-fluoro-10-(2,3,4,5,6-pentafluorophenyl)anthracene;
Figure 186 illustrates a 2-fluoro-9-phenyl-10-(2,3,5,6-tetrafluorophenyl)anthracene;

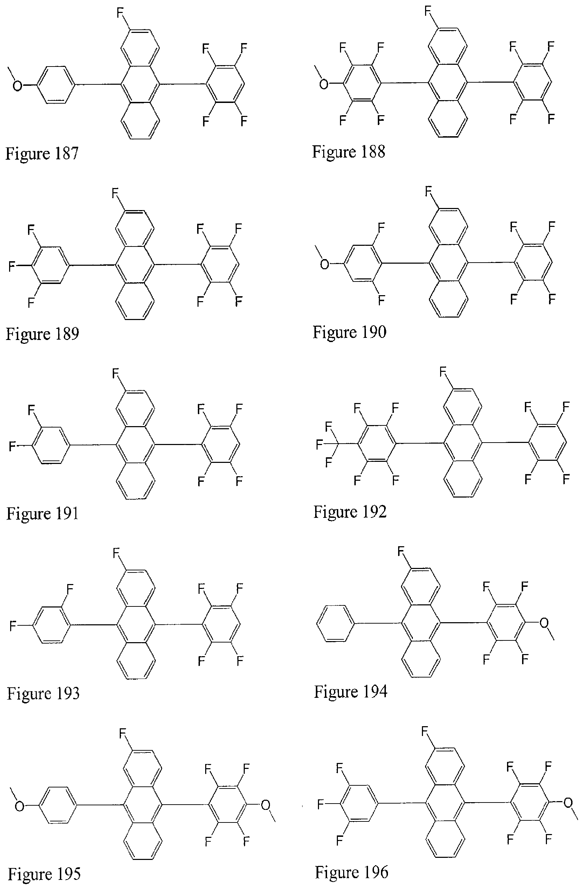

Figure 187 illustrates a 2-fluoro-9-(4-methoxyphenyl)-10-(2,3,5,6-tetrafluorophenyl)anthracene;
Figure 188 illustrates a 2-fluoro-9-(2,3,5,6-tetrafluorophenyl)-10-(2,3,5,6-tetrafluoro-4-methoxyphenyl)anthracene;
Figure 189 illustrates a 2-fluoro-9-(2,3,5,6-tetrafluorophenyl)-10-(3,4,5-trifluorophenyl)anthracene;
Figure 190 illustrates a 9-(2,6-difluoro-4-methoxyphenyl)-2-fluoro-10-(2,3,5,6-tetrafluorophenyl)anthracene;
Figure 191 illustrates a 9-(3,4-difluorophenyl)-2-fluoro-10-(2,3,5,6-tetrafluorophenyl)anthracene;
Figure 192 illustrates a 2-fluoro-9-[2,3,5,6-tetrafluoro-4-(trifluoromethyl)phenyl]-10-(2,3,,5,6-tetrafluorophenyl)anthracene;
Figure 193 illustrates a 9-(3,4-difluorophenyl)-2-fluoro-10-(2,3,5,6-tetrafluorophenyl)anthracene;
Figure 194 illustrates a 2-fluoro-9-phenyl-10-(2,3,5,6-tetrafluoro-4-methoxyphenyl)anthracene;
Figure 195 illustrates a 2-fluoro-9-(4-methoxyphenyl)-10-(2,3,5,6-tetrafluoro-4-methoxyphenyl)anthracene;
Figure 196 illustrates a 2-fluoro-9-(2,3,5,6-tetrafluoro-4-methoxyphenyl)-10-(3,4,5-trifluorophenyl)anthracene;

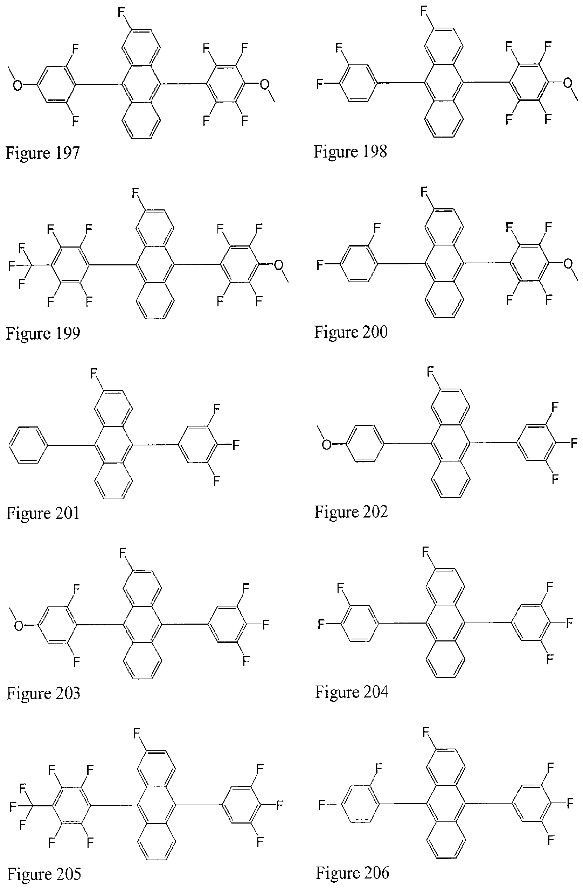

Figure 197 illustrates a 9-(2,6-difluoro-4-methoxyphenyl)-2-fluoro-10-(2,3,5,6-tetrafluoro-4-methoxyphenyl)anthracene;
Figure 198 illustrates a 9-(3,4-difluorophenyl)-2-fluoro-10-(2,3,5,6-tetrafluoro-4-methoxyphenyl)anthracene;
Figure 199 illustrates a 2-fluoro-9-[2,3,5,6-tetrafluoro-4-(trifluoromethyl)phenyl]-10-(2,3,5,6-tetrafluoro-4-methoxyphenyl)anthracene;
Figure 200 illustrates a 9-(2,4-difluorophenyl)-2-fluoro-10-(2,3,5,6-tetrafluoro-4-methoxyphenyl)anthracene;
Figure 201 illustrates a 2-fluoro-9-phenyl-10-(3,4,5-trifluorophenyl)anthracene;
Figure 202 illustrates a 2-fluoro-9-(4-methoxyphenyl)-10-(3,4,5-trifluorophenyl)anthracene;
Figure 203 illustrates a 9-(2,6-difluoro-4-methoxyphenyl)-2-fluoro-10-(3,4,5-trifluorophenyl)anthracene;
Figure 204 illustrates a 9-(3,4-difluorophenyl)-2-fluoro-10-(3,4,5-trifluorophenyl)anthracene;
Figure 205 illustrates a 2-fluoro-9-[2,3,5,6-tetrafluoro-4-(trifluoromethyl)phenyl]-10-(3,4,5-trifluorophenyl)anthracene;
Figure 206 illustrates a 9-(2,4-difluorophenyl)-2-fluoro-10-(3,4,5-trifluorophenyl)anthracene;

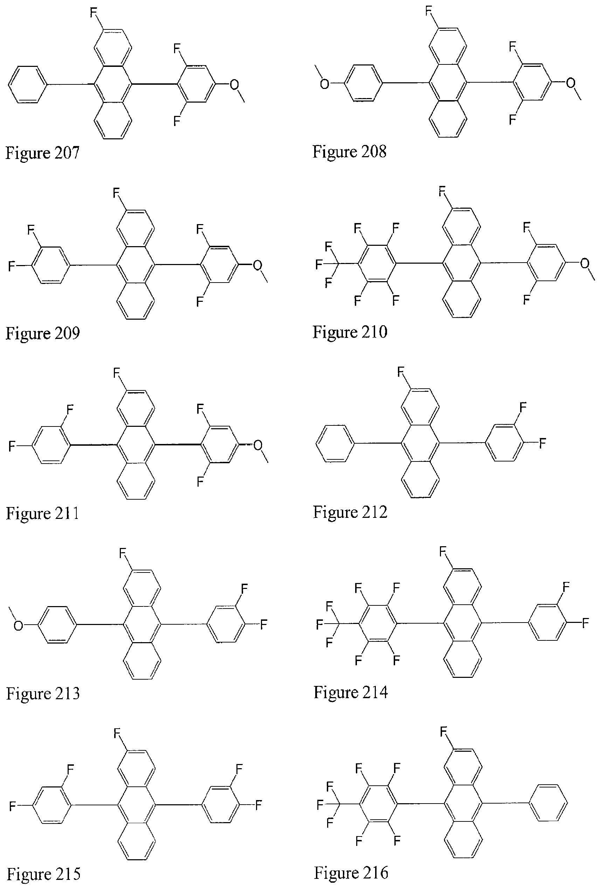

Figure 207 illustrates a 9-(2,6-difluoro-4-methoxyphenyl)-2-fluoro-10-phenylanthracene:
Figure 208 illustrates a 9-(2,6-difluoro-4-methoxyphenyl)-2-fluoro-10-(4-methoxyphenyl) anthracene:
Figure 209 illustrates a 9-(2,6-difluoro-4-methoxyphenyl)-10-(3,4-difluorophenyl)-2-fluoroanthracene;
Figure 210 illustrates a 9-(2,6-difluoro-4-methoxyphenyl)-2-fluoro-10-[2,3,5,6-tetrafluoro-4-(trifluoromethyl)phenyl]anthracene;
Figure 211 illustrates a 9-(2,6-difluoro-4-methoxyphenyl)-2-fluoro-10-(2,4-difluorophenyl)anthracene;
Figure 212 illustrates a 9-(3,4-difluorophenyl)-2-fluoro-10-phenylanthracene;
Figure 213 illustrates a 9-(3,4-difluorophenyl)-2-fluoro-10-(4-methoxyphenyl)anthracene;
Figure 214 illustrates a 9-(3,4-difluorophenyl)-2-fluoro-10-[2,3,5,6-tetrafluoro-4-(trifluoromethyl)phenyl]anthracene;
Figure 215 illustrates a 9-(2,4-difluorophenyl)-2-fluoro-10-(3,4-difluorophenyl)anthracene;
Figure 216 illustrates a 2-fluoro-9-phenyl-10-[2,3,5,6-tetrafluoro-4-(trifluoromethyl)phenyl]anthracene;

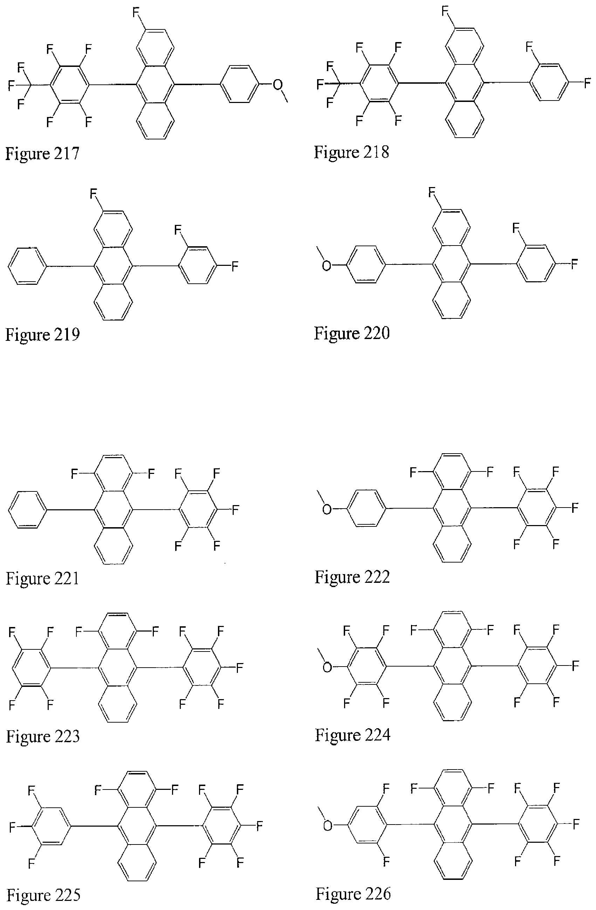

Figure 217 illustrates a 2-fluoro-9-(4-methoxyphenyl)-10-[2,3,5,6-tetrafluoro-4-(trifluoromethyl)phenyl]anthracene;
Figure 218 illustrates a 9-(2,4-difluorophenyl)-2-fluoro-10-[2,3,5,6-tetrafluoro-4-(trifluoromethyl)phenyl]anthracene;
Figure 219 illustrates a 9-(2,4-difluorophenyl)-2-fluoro-10-phenylanthracene;
Figure 220 illustrates a 9-(2,4-difluorophenyl)-2-fluoro-10-(4-methoxyphenyl)anthracene;
Figure 221 illustrates a 1,4-difluoro-9-(2,3,4,5,6-pentafluorophenyl)-10-phenylanthracene;
Figure 222 illustrates a 1,4-difluoro-9-(4-methoxyphenyl)-10-(2,3,4,5,6-pentafluorophenyl)anthracene;
Figure 223 illustrates a 1,4-difluoro-9-(2,3,4,5,6-pentafluorophenyl)-10-(2,3,5,6-tetrafluoropheny)lanthracene;
Figure 224 illustrates a 1,4-difluoro-9-(2,3,4,5,6-pentafluorophenyl)-10-(2,3,5,6-tetrafluoro-4-methoxyphenyl)anthracene;
Figure 225 illustrates a 1,4-difluoro-9-(2,3,4,5,6-pentafluorophenyl)-10-(3,4,5-trifluoro)anthracene;
Figure 226 illustrates a 9-(2,6-difluoro-4-methoxyphenyl)-1,4-difluoro-10-(2,3,4,5,6-pentafluorophenyl)anthracene

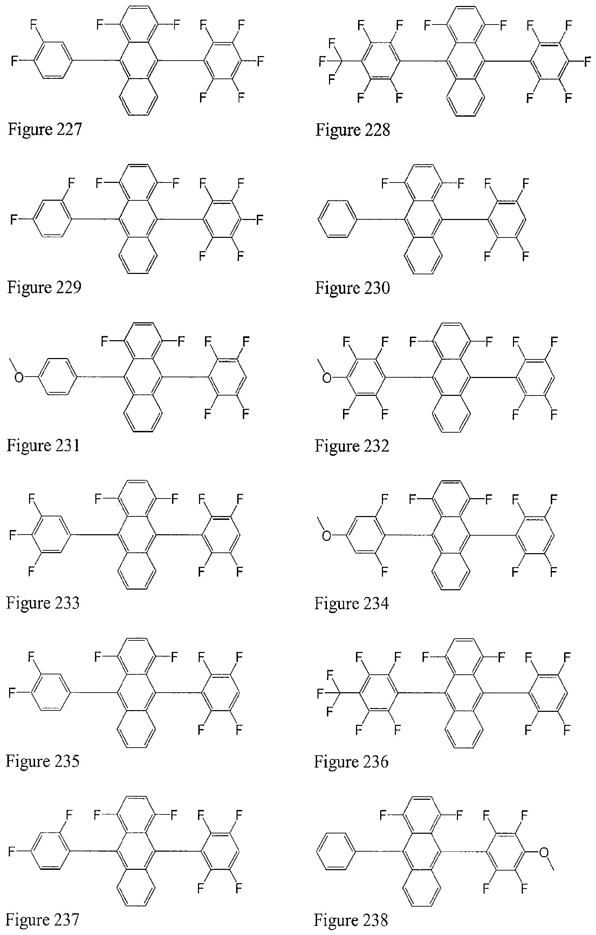

Figure 227 illustrates a 9-(3,4-difluorophenyl)-1,4-difluoro-10-(2,3,4,5,6-pentafluorophenyl)anthracene;
Figure 228 illustrates a 1,4-difluoro-9-(2,3,4,5,6-pentafluorophenyl)-10-[2,3,5,6-tetrafluoro-4-(trifluoromethyl)phenyl]anthracene;
Figure 229 illustrates a 9-(2,4-difluorophenyl)-1,4-difluoro-10-(2,3,4,5,6-pentafluorophenyl)anthracene;
Figure 230 illustrates a 1,4-difluoro-9-phenyl-10-(2,3,5,6-tetrafluorophenyl)anthracene;
Figure 231 illustrates a 1,4-difluoro-9-(4-methoxyphenyl)-10-(2,3,5,6-tetrafluorophenyl)anthracene;
Figure 232 illustrates a 1,4-difluoro-9-(2,3,5,6-tetrafluorophenyl)-10-(2,3,5,6-tetrafluoro-4-methoxyphenyl)anthracene;
Figure 233 illustrates a 1,4-difluoro-9-(2,3,5,6-tetrafluorophenyl)-10-(3,4,5-trifluorophenyl)anthracene;
Figure 234 illustrates a 9-(2,6-difluoro-4-methoxyphenyl)-1,4-difluoro-10-(2,3,5,6-tetrafluorophenyl)anthracene;
Figure 235 illustrates a 9-(3,4-difluorophenyl)-1,4-difluoro-10-(2,3,5,6-tetrafluorophenyl)anthracene;
Figure 236 illustrates a 1,4-difluoro-9-[2,3,5,6-tetrafluoro-4-(trifluoromethyl)phenyl]-10-(2,3,,5,6-tetrafluorophenyl)anthracene;
Figure 237 illustrates a 9-(3,4-difluorophenyl)-1,4-difluoro-10-(2,3,5,6-tetrafluorophenyl)anthracene;
Figure 238 illustrates a 1,4-difluoro-9-phenyl-10-(2,3,5,6-tetrafluoro-4-methoxyphenyl)anthracene;

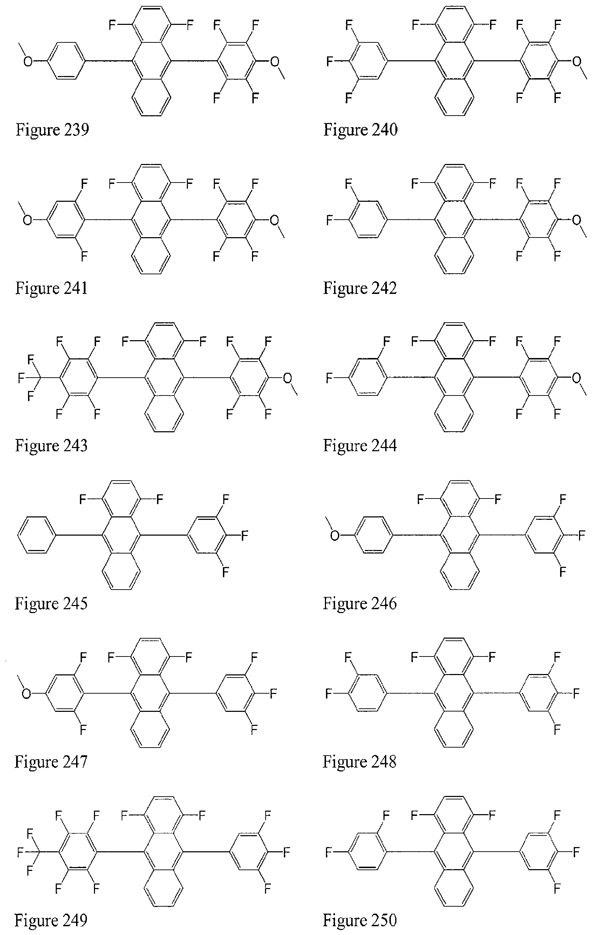

Figure 239 illustrates a 1,4-difluoro-9-(4-methoxyphenyl)-10-(2,3,5,6-tetrafluoro-4-methoxyphenyl)anthracene;
Figure 240 illustrates a 1,4-difluoro-9-(2,3,5,6-tetrafluoro-4-methoxyphenyl)-10-(3,4,5-trifluorophenyl)anthracene;
Figure 241 illustrates a 9-(2,6-difluoro-4-methoxyphenyl)-1,4-difluoro-10-(2,3,5,6-tetrafluoro-4-methoxyphenyl)anthracene;
Figure 242 illustrates a 9-(3,4-difluorophenyl)-1,4-difluoro-10-(2,3,5,6-tetrafluoro-4-methoxyphenyl)anthracene;
Figure 243 illustrates a 1,4-difluoro-9-[2,3,5,6-tetrafluoro-4-(trifluoromethyl)phenyl]-10-(2,3,5,6-tetrafluoro-4-methoxyphenyl)anthracene;
Figure 244 illustrates a 9-(2,4-difluorophenyl)-1,4-difluoro-10-(2,3,5,6-tetrafluoro-4-methoxyphenyl)anthracene;
Figure 245 illustrates a 1,4-difluoro-9-phenyl-10-(3,4,5-trifluorophenyl)anthracene;
Figure 246 illustrates a 1,4-difluoro-9-(4-methoxyphenyl)-10-(3,4,5-trifluorophenyl)anthracene;
Figure 247 illustrates a 9-(2,6-difluoro-4-methoxyphenyl)-1,4-difluoro-10-(3,4,5-trifluorophenyl)anthracene;
Figure 248 illustrates a 9-(3,4-difluorophenyl)-1,4-difluoro-10-(3,4,5-trifluorophenyl)anthracene;
Figure 249 illustrates a 1,4-difluoro-9-[2,3,5,6-tetrafluoro-4-(trifluoromethyl)phenyl]-10-(3,4,5-trifluorophenyl)anthracene;
Figure 250 illustrates a 9-(2,4-difluorophenyl)-1,4-difluoro-10-(3,4,5-trifluorophenyl)anthracene;

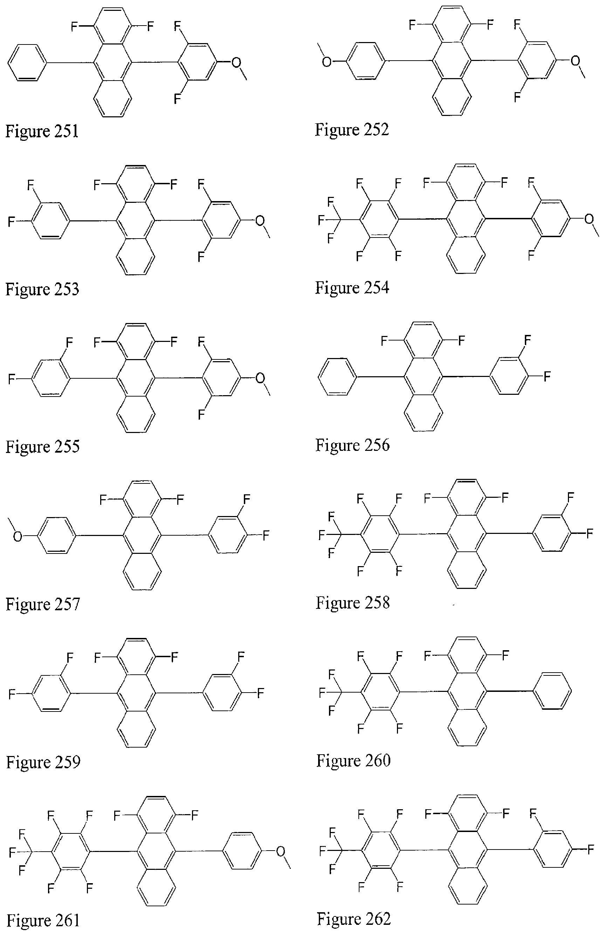

Figure 251 illustrates a 9-(2,6-difluoro-4-methoxyphenyl)-1,4-difluoro-10-phenylanthracene:
Figure 252 illustrates a 9-(2,6-difluoro-4-methoxyphenyl)-1,4-difluoro-10-(4-methoxyphenyl)anthracene:
Figure 253 illustrates a 9-(2,6-difluoro-4-methoxyphenyl)-10-(3,4-difluorophenyl)-1,4-difluoroanthracene;
Figure 254 illustrates a 9-(2,6-difluoro-4-methoxyphenyl)-1,4-difluoro-10-[2,3,5,6-tetrafluoro-4-(trifluoromethyl)phenyl]anthracene;
Figure 255 illustrates a 9-(2,6-difluoro-4-methoxyphenyl)-1,4-difluoro-10-(2,4-difluorophenyl)anthracene;
Figure 256 illustrates a 9-(3,4-difluorophenyl)-1,4-difluoro-10-phenyl anthracene;
Figure 257 illustrates a 9-(3,4-difluorophenyl)-1,4-difluoro-10-(4-methoxyphenyl)anthracene;
Figure 258 illustrates a 9-(3,4-difluorophenyl)-1,4-difluoro-10-[2,3,5,6-tetrafluoro-4-(trifluoromethyl)phenyl]anthracene;
Figure 259 illustrates a 9-(2,4-difluorophenyl)-1,4-difluoro-10-(3,4-difluorophenyl)anthracene;
Figure 260 illustrates a 1,4-difluoro-9-phenyl-10-[2,3,5,6-tetrafluoro-4-(trifluoromethyl)phenyl]anthracene;
Figure 261 illustrates a 1,4-difluoro-9-(4-methoxyphenyl)-10-[2,3,5,6-tetrafluoro-4-(trifluoromethyl)phenyl]anthracene;
Figure 262 illustrates a 9-(2,4-difluorophenyl)-1,4-difluoro-10-[2,3,5,6-tetrafluoro-4-(trifluoromethyl)phenyl]anthracene;

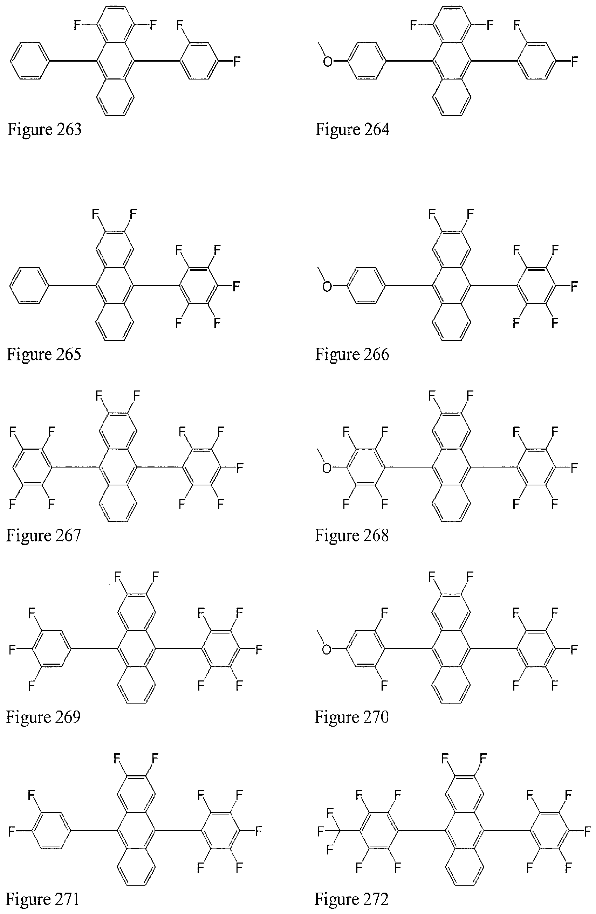

Figure 263 illustrates a 9-(2,4-difluorophenyl)-1,4-difluoro-10-phenyl anthracene;
Figure 264 illustrates a 9-(2,4-difluorophenyl)-1,4-difluoro-10-(4-methoxyphenyl)anthracene;
Figure 265 illustrates a 2,3-difluoro-9-(2,3,4,5,6-pentafluorophenyl)-10-phenyl anthracene;
Figure 266 illustrates a 2,3-difluoro-9-(4-methoxyphenyl)-10-(2,3,4,5,6-pentafluorophenyl)anthracene;
Figure 267 illustrates a2,3-difluoro-9-(2,3,4,5,6-pentafluorophenyl)-10-(2,3,5,6-tetrafluoropheny)lanthracene;
Figure 268 illustrates a 2,3-difluoro-9-(2,3,4,5,6-pentafluorophenyl)-10-(2,3,5,6-tetrafluoro-4-methoxyphenyl)anthracene;
Figure 269 illustrates a 2,3-difluoro-9-(2,3,4,5,6-pentafluorophenyl)-10-(3,4,5-trifluoro)anthracene;
Figure 270 illustrates a 9-(2,6-difluoro-4-methoxyphenyl)-2,3-difluoro-10-(2,3,4,5,6-pentafluorophenyl)anthracene
Figure 271 illustrates a 9-(3,4-difluorophenyl)-2,3-difluoro-10-(2,3,4,5,6-pentafluorophenyl)anthracene;
Figure 272 illustrates a 2,3-difluoro-9-(2,3,4,5,6-pentafluorophenyl)-10-[2,3,5,6-tetrafluoro-4-(trifluoromethyl)phenyl]anthracene;

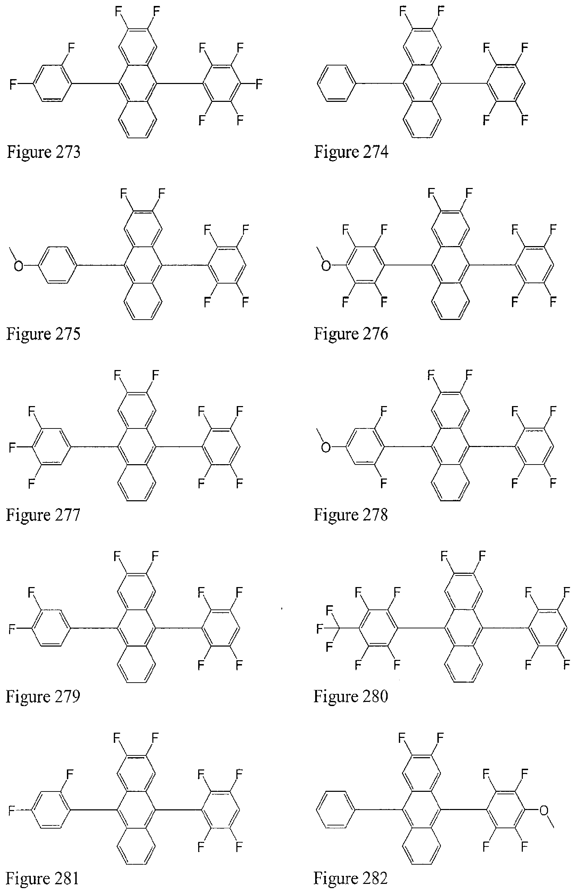

Figure 273 illustrates a 9-(2,4-difluorophenyl)-2,3-difluoro-10-(2,3,4,5,6-pentafluorophenyl)anthracene;
Figure 274 illustrates a 2,3-difluoro-9-phenyl-10-(2,3,5,6-tetrafluorophenyl)anthracene;
Figure 275 illustrates a 2,3-difluoro-9-(4-methoxyphenyl)-10-(2,3,5,6-tetrafluorophenyl)anthracene;
Figure 276 illustrates a 2,3-difluoro-9-(2,3,5,6-tetrafluorophenyl)-10-(2,3,5,6-tetrafluoro-4-methoxyphenyl)anthracene;
Figure 277 illustrates a 2,3-difluoro-9-(2,3,5,6-tetrafluorophenyl)-10-(3,4,5-trifluorophenyl)anthracene;
Figure 278 illustrates a 9-(2,6-difluoro-4-methoxyphenyl)-2,3-difluoro-10-(2,3,5,6-tetrafluorophenyl)anthracene;
Figure 279 illustrates a 9-(3,4-difluorophenyl)-2,3-difluoro-10-(2,3,5,6-tetrafluorophenyl)anthracene;
Figure 280 illustrates a 2,3-difluoro-9-[2,3,5,6-tetrafluoro-4-(trifluoromethyl)phenyl]-10-(2,3,,5,6-tetrafluorophenyl)anthracene;
Figure 281 illustrates a 9-(3,4-difluorophenyl)-2,3-difluoro-10-(2,3,5,6-tetrafluorophenyl)anthracene;
Figure 282 illustrates a 2,3-difluoro-9-phenyl-10-(2,3,5,6-tetrafluoro-4-methoxyphenyl)anthracene;

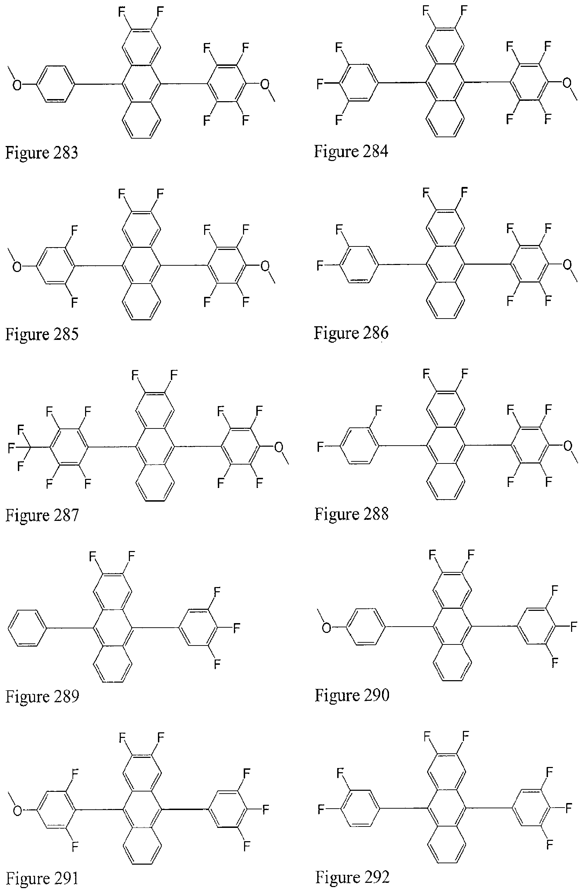

Figure 283 illustrates a 2,3-difluoro-9-(4-methoxyphenyl)-10-(2,3,5,6-tetrafluoro-4-methoxyphenyl)anthracene;
Figure 284 illustrates a 2,3-difluoro-9-(2,3,5,6-tetrafluoro-4-methoxyphenyl)-10-(3,4,5-trifluorophenyl)anthracene;
Figure 285 illustrates a 9-(2,6-difluoro-4-methoxyphenyl)-2,3-difluoro-10-(2,3,5,6-tetrafluoro-4-methoxyphenyl)anthracene;
Figure 286 illustrates a 9-(3,4-difluorophenyl)-2,3-difluoro-10-(2,3,5,6-tetrafluoro-4-methoxyphenyl)anthracene;
Figure 287 illustrates a 2,3-difluoro-9-[2,3,5,6-tetrafluoro-4-(trifluoromethyl)phenyl]-10-(2,3,5,6-tetrafluoro-4-methoxyphenyl)anthracene;
Figure 288 illustrates a 9-(2,4-difluorophenyl)-2,3-difluoro-10-(2,3,5,6-tetrafluoro-4-methoxyphenyl)anthracene;
Figure 289 illustrates a 2,3-difluoro-9-phenyl-10-(3,4,5-tri fluorophenyl) anthracene;
Figure 290 illustrates a 2,3-difluoro-9-(4-methoxyphenyl)-10-(3,4,5-trifluorophenyl)anthracene;
Figure 291 illustrates a 9-(2,6-difluoro-4-methoxyphenyl)-2,3-difluoro-10-(3,4,5-trifluorophenyl)anthracene;
Figure 292 illustrates a 9-(3,4-difluorophenyl)-2,3-difluoro-10-(3,4,5-trifluorophenyl)anthracene;

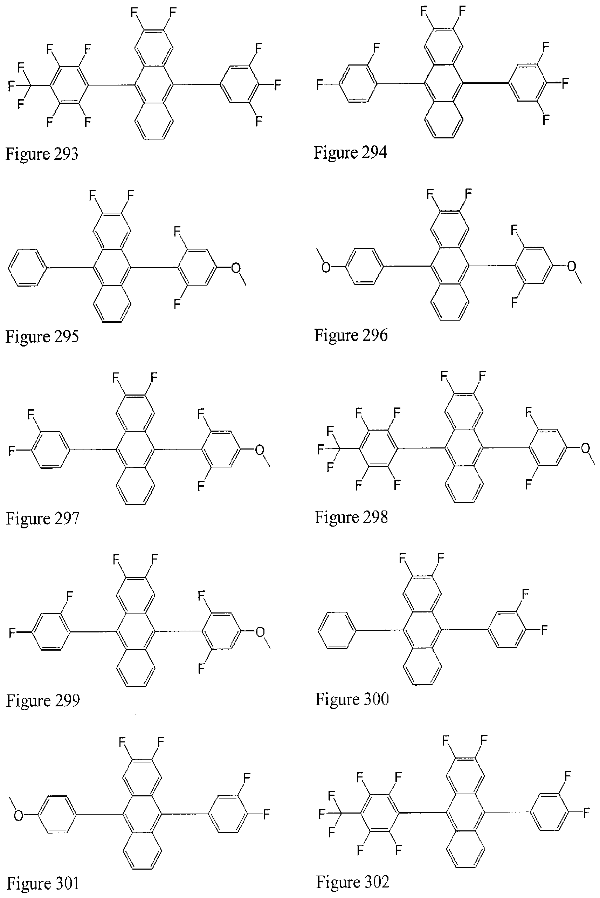

Figure 293 illustrates a 2,3-difluoro-9-[2,3,5,6-tetrafluoro-4-(trifluoromethyl)phenyl]-10-(3,4,5-trifluorophenyl)anthracene;
Figure 294 illustrates a 9-(2,4-difluorophenyl)-2,3-difluoro-10-(3,4,5-trifluorophenyl)anthracene;
Figure 295 illustrates a 9-(2,6-difluoro-4-methoxyphenyl)-2,3-difluoro-10-phenylanthracene:
Figure 296 illustrates a 9-(2,6-difluoro-4-methoxyphenyl)-2,3-difluoro-10-(4-methoxyphenyl)anthracene:
Figure 297 illustrates a 9-(2,6-difluoro-4-methoxyphenyl)-10-(3,4-difluorophenyl)-2,3-difluoroanthracene;
Figure 298 illustrates a 9-(2,6-difluoro-4-methoxyphenyl)-2,3-difluoro-10-[2,3,5,6-tetrafluoro-4-(trifluoromethyl)phenyl] anthracene;
Figure 299 illustrates a 9-(2,6-difluoro-4-methoxyphenyl)-2,3-difluoro-10-(2,4-difluorophenyl)anthracene;
Figure 300 illustrates a 9-(3,4-difluorophenyl)-2,3-difluoro-10-phenylanthracene;
Figure 301 illustrates a 9-(3,4-difluorophenyl)-2,3-difluoro-10-(4-methoxyphenyl)anthracene;
Figure 302 illustrates a 9-(3,4-difluorophenyl)-2,3-difluoro-10-[2,3,5,6-tetrafluoro-4-(trifluoromethyl)phenyl]anthracene;

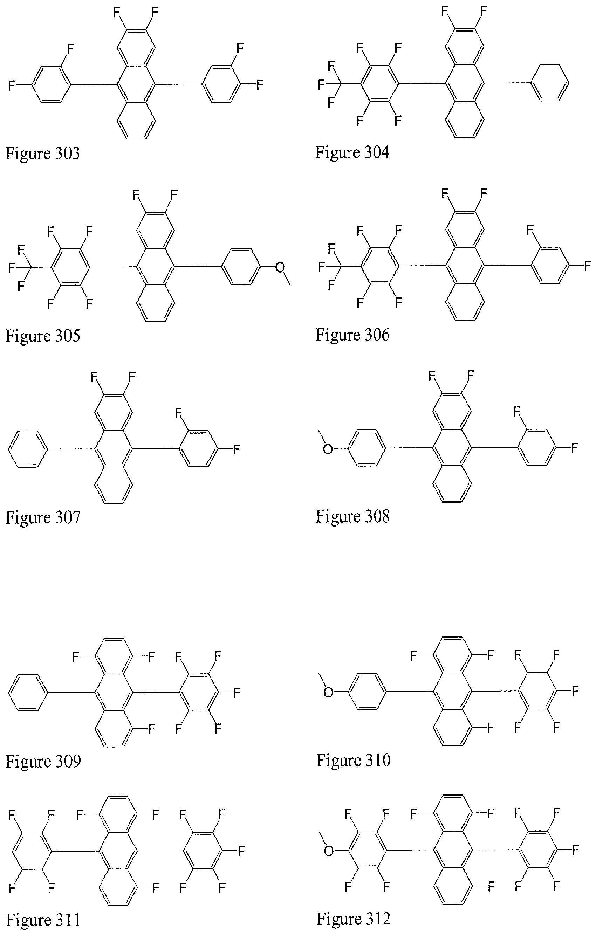

Figure 303 illustrates a 9-(2,4-difluorophenyl)-2,3-difluoro-10-(3,4-difluorophenyl)anthracene;
Figure 304 illustrates a 2,3-difluoro-9-phenyl-10-[2,3,5,6-tetrafluoro-4-(trifluoromethyl)phenyl]anthracene;
Figure 305 illustrates a 2,3-difluoro-9-(4-methoxyphenyl)-10-[2,3,5,6-tetrafluoro-4-(trifluoromethyl)phenyl]anthracene;
Figure 306 illustrates a 9-(2,4-difluorophenyl)-2,3-difluoro-10-[2,3,5,6-tetrafluoro-4-(trifluoromethyl)phenyl]anthracene;
Figure 307 illustrates a 9-(2,4-difluorophenyl)-2,3-difluoro-10-phenylanthracene;
Figure 308 illustrates a 9-(2,4-difluorophenyl)-2,3-difluoro-10-(4-methoxyphenyl)anthracene;
Figure 309 illustrates a 1,4,5-trifluoro-9-(2,3,4,5,6-pentafluorophenyl)-10-phenyl anthracene;
Figure 310 illustrates a 1,4,5-trifluoro-9-(4-methoxyphenyl)-10-(2,3,4,5,6-pentafluorophenyl)anthracene;
Figure 311 illustrates a 1,4,5-trifluoro-9-(2,3,4,5,6-pentafluorophenyl)-10-(2,3,5,6-tetrafluoropheny)lanthracene;
Figure 312 illustrates a 1,4,5-trifluoro-9-(2,3,4,5,6-pentafluorophenyl)-10-(2,3,5,6-tetrafluoro-4-methoxyphenyl)anthracene;

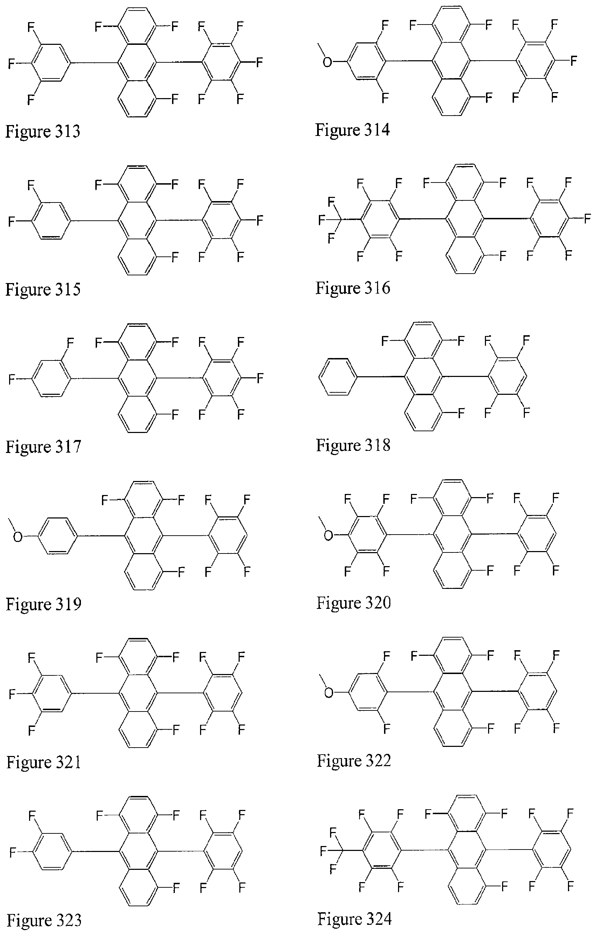

Figure 313 illustrates a 1,4,5-trifluoro-9-(2,3,4,5,6-pentafluorophenyl)-10-(3,4,5-trifluoro)anthracene;
Figure 314 illustrates a 9-(2,6-difluoro-4-methoxyphenyl)-1,4,5-trifluoro-10-(2,3,4,5,6-pentafluorophenyl)anthracene
Figure 315 illustrates a 9-(3,4-difluorophenyl)-1,4,5-trifluoro-10-(2,3,4,5,6-pentafluorophenyl)anthracene;
Figure 316 illustrates a 1,4,5-trifluoro-9-(2,3,4,5,6-pentafluorophenyl)-10-[2,3,5,6-tetrafluoro-4-(trifluoromethyl)phenyl] anthracene;
Figure 317 illustrates a 9-(2,4-difluorophenyl)-1,4,5-trifluoro-10-(2,3,4,5,6-pentafluorophenyl)anthracene;
Figure 318 illustrates a 1,4,5-trifluoro-9-phenyl-10-(2,3,5,6-tetrafluorophenyl)anthracene;
Figure 319 illustrates a 1,4,5-trifluoro-9-(4-methoxyphenyl)-10-(2,3,5,6-tetrafluorophenyl)anthracene;
Figure 320 illustrates a 1,4,5-trifluoro-9-(2,3,5,6-tetrafluorophenyl)-10-(2,3,5,6-tetrafluoro-4-methoxyphenyl)anthracene;
Figure 321 illustrates a 1,4,5-trifluoro-9-(2,3,5,6-tetrafluorophenyl)-10-(3,4,5-trifluorophenyl)anthracene;
Figure 322 illustrates a 9-(2,6-difluoro-4-methoxyphenyl)-1,4,5-trifluoro-10-(2,3,5,6-tetrafluorophenyl)anthracene;
Figure 323 illustrates a 9-(3,4-difluorophenyl)-1,4,5-trifluoro-10-(2,3,5,6-tetrafluorophenyl)anthracene;
Figure 324 illustrates a 1,4,5-trifluoro-9-[2,3,5,6-tetrafluoro-4-(trifluoromethyl)phenyl]-10-(2,3,,5,6-tetrafluorophenyl)anthracene;

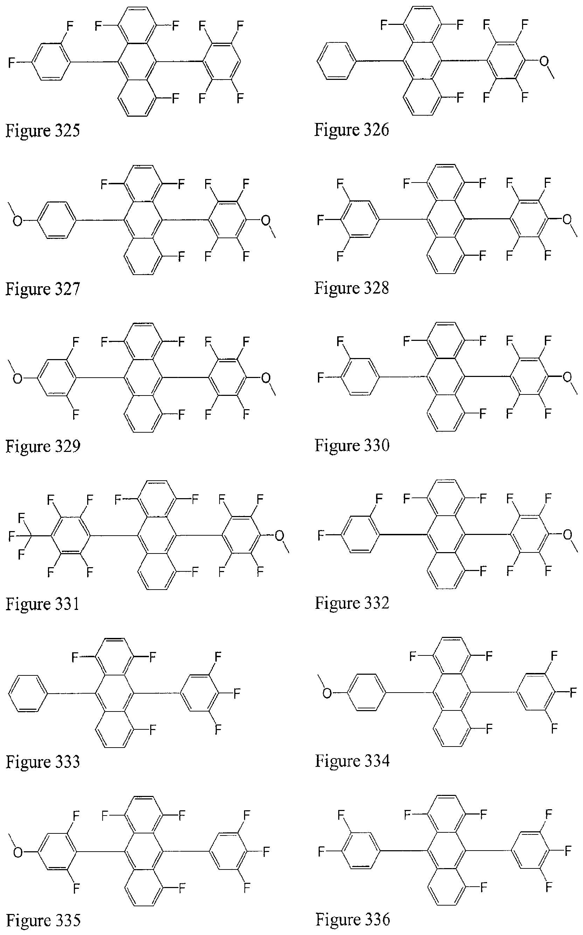

Figure 325 illustrates a 9-(3,4-difluorophenyl)-1,4,5-trifluoro-10-(2,3,5,6-tetrafluorophenyl)anthracene;
Figure 326 illustrates a 1,4,5-trifluoro-9-phenyl-10-(2,3,5,6-tetrafluoro-4-methoxyphenyl)anthracene;
Figure 327 illustrates a 1,4,5-trifluoro-9-(4-methoxyphenyl)-10-(2,3,5,6-tetrafluoro-4-methoxyphenyl)anthracene;
Figure 328 illustrates a 1,4,5-trifluoro-9-(2,3,5,6-tetrafluoro-4-methoxyphenyl)-10-(3,4,5-trifluorophenyl)anthracene;
Figure 329 illustrates a 9-(2,6-difluoro-4-methoxyphenyl)-1,4,5-trifluoro-10-(2,3,5,6-tetrafluoro-4-methoxyphenyl)anthracene;
Figure 330 illustrates a 9-(3,4-difluorophenyl)-1,4,5-trifluoro-10-(2,3,5,6-tetrafluoro-4-methoxyphenyl)anthracene;
Figure 331 illustrates a 1,4,5-trifluoro-9-[2,3,5,6-tetrafluoro-4-(trifluoromethyl)phenyl]-10-(2,3,5,6-tetrafluoro-4-methoxyphenyl)anthracene;
Figure 332 illustrates a 9-(2,4-difluorophenyl)-1,4,5-trifluoro-10-(2,3,5,6-tetrafluoro-4-methoxyphenyl)anthracene;
Figure 333 illustrates a 1,4,5-trifluoro-9-phenyl-10-(3,4,5-trifluorophenyl) anthracene;
Figure 334 illustrates a 1,4,5-trifluoro-9-(4-methoxyphenyl)-10-(3,4,5-trifluorophenyl)anthracene;
Figure 335 illustrates a 9-(2,6-difluoro-4-methoxyphenyl)-1,4,5-trifluoro-10-(3,4,5-trifluorophenyl)anthracene;
Figure 336 illustrates a 9-(3,4-difluorophenyl)-1,4,5-trifluoro-10-(3,4,5-trifluorophenyl)anthracene;

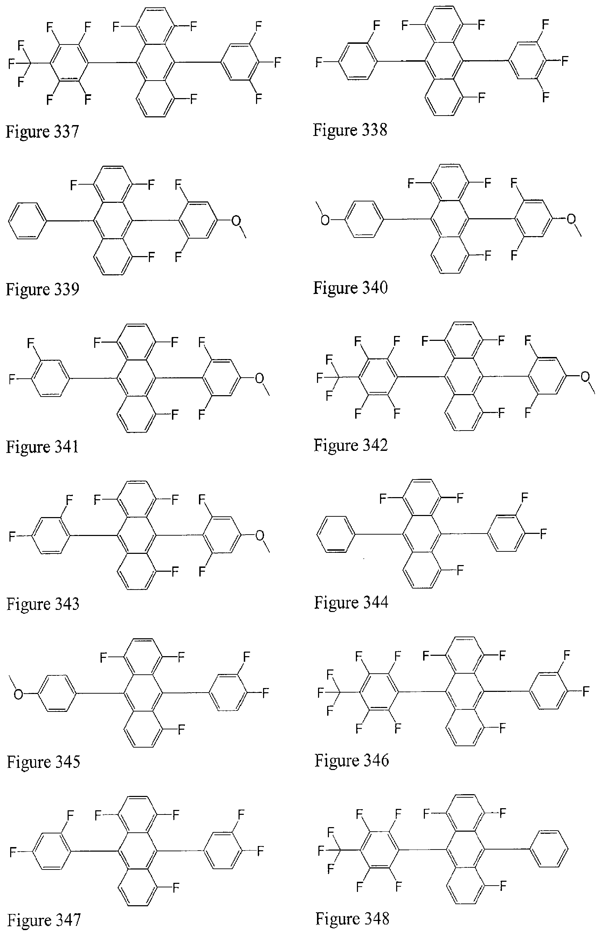

Figure 337 illustrates a 1,4,5-trifluoro-9-[2,3,5,6-tetrafluoro-4-(trifluoromethyl)phenyl]-10-(3,4,5-trifluorophenyl)anthracene;
Figure 338 illustrates a 9-(2,4-difluorophenyl)-1,4,5-trifluoro-10-(3,4,5-trifluorophenyl)anthracene;
Figure 339 illustrates a 9-(2,6-difluoro-4-methoxyphenyl)-1,4,5-trifluoro-10-phenylanthracene:
Figure 340 illustrates a 9-(2,6-difluoro-4-methoxyphenyl)-1,4,5-trifluoro-10-(4-methoxyphenyl)anthracene:
Figure 341 illustrates a 9-(2,6-difluoro-4-methoxyphenyl)-10-(3,4-difluorophenyl)-1,4,5-trifluoroanthracene;
Figure 342 illustrates a 9-(2,6-difluoro-4-methoxyphenyl)-1,4,5-trifluoro-10-[2,3,5,6-tetrafluoro-4-(trifluoromethyl)phenyl]anthracene;
Figure 343 illustrates a 9-(2,6-difluoro-4-methoxyphenyl)-1,4,5-trifluoro-10-(2,4-difluorophenyl)anthracene;
Figure 344 illustrates a 9-(3,4-difluorophenyl)-1,4,5-trifluoro-10-phenylanthracene;
Figure 345 illustrates a 9-(3,4-difluorophenyl)-1,4,5-trifluoro-10-(4-methoxyphenyl)anthracene;
Figure 346 illustrates a 9-(3,4-difluorophenyl)-1,4,5-trifluoro-10-[2,3,5,6-tetrafluoro-4-(trifluoromethyl)phenyl]anthracene;
Figure 347 illustrates a 9-(2,4-difluorophenyl)-1,4,5-trifluoro-10-(3,4-difluorophenyl)anthracene;
Figure 348 illustrates a 1,4,5-trifluoro-9-phenyl-10-[2,3,5,6-tetrafluoro-4-(trifluoromethyl)phenyl]anthracene;

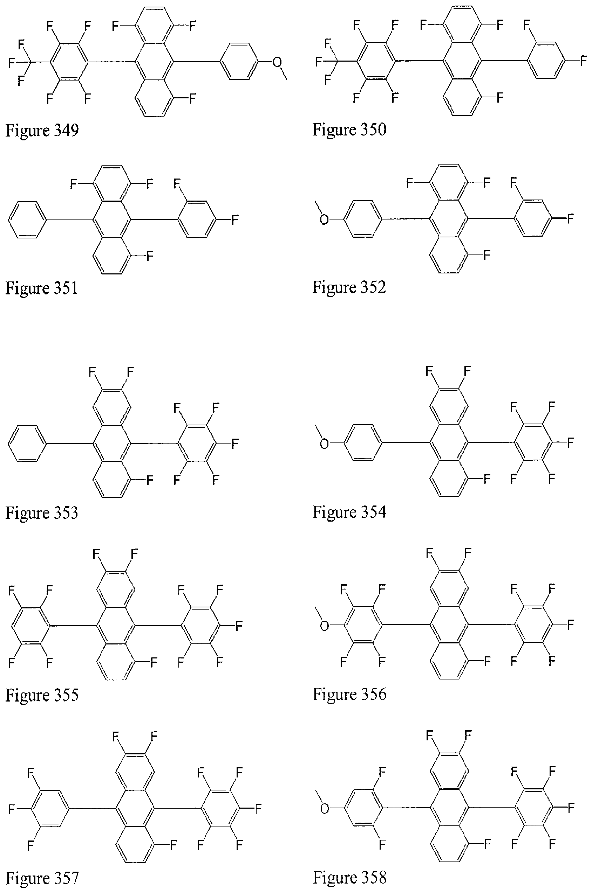

Figure 349 illustrates a 1,4,5-trifluoro-9-(4-methoxyphenyl)-10-[2,3,5,6-tetrafluoro-4-(trifluoromethyl)phenyl]anthracene;
Figure 350 illustrates a 9-(2,4-difluorophenyl)-1,4,5-trifluoro-10-[2,3,5,6-tetrafluoro-4-(trifluoromethyl)phenyl]anthracene;
Figure 351 illustrates a 9-(2,4-difluorophenyl)-1,4,5-trifluoro-10-phenylanthracene;
Figure 352 illustrates a 9-(2,4-difluorophenyl)-1,4,5-trifluoro-10-(4-methoxyphenyl)anthracene;
Figure 353 illustrates a 2,3,5-trifluoro-9-(2,3,4,5,6-pentafluorophenyl)-10-phenyl anthracene;
Figure 354 illustrates a 2,3,5-trifluoro-9-(4-methoxyphenyl)-10-(2,3,4,5,6-pentafluorophenyl)anthracene;
Figure 355 illustrates a 2,3,5-trifluoro-9-(2,3,4,5,6-pentafluorophenyl)-10-(2,3,5,6-tetrafluoropheny)lanthracene;
Figure 356 illustrates a 2,3,5-trifluoro-9-(2,3,4,5,6-pentafluorophenyl)-10-(2,3,5,6-tetrafluoro-4-methoxyphenyl)anthracene;
Figure 357 illustrates a 2,3,5-trifluoro-9-(2,3,4,5,6-pentafluorophenyl)-10-(3,4,5-trifluoro)anthracene;
Figure 358 illustrates a 9-(2,6-difluoro-4-methoxyphenyl)-2,3,5-trifluoro-10-(2,3,4,5,6-pentafluorophenyl)anthracene

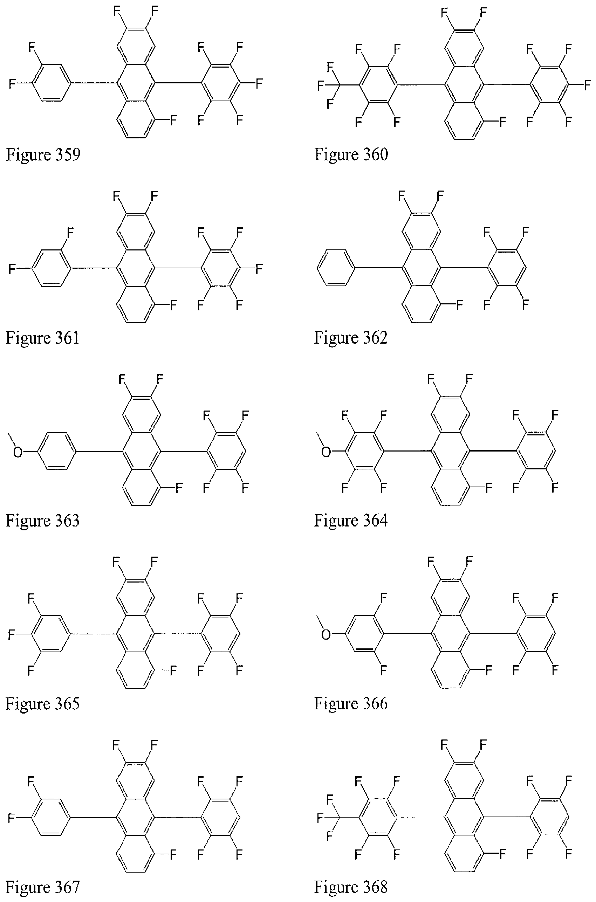

Figure 359 illustrates a 9-(3,4-difluorophenyl)-2,3,5-trifluoro-10-(2,3,4,5,6-pentafluorophenyl)anthracene;
Figure 360 illustrates a 2,3,5-trifluoro-9-(2,3,4,5,6-pentafluorophenyl)-10-[2,3,5,6-tetrafluoro-4-(trifluoromethyl)phenyl]anthracene;
Figure 361 illustrates a 9-(2,4-difluorophenyl)-2,3,5-trifluoro-10-(2,3,4,5,6-pentafluorophenyl)anthracene;
Figure 362 illustrates a 2,3,5-trifluoro-9-phenyl-10-(2,3,5,6-tetrafluorophenyl)anthracene;
Figure 363 illustrates a 2,3,5-trifluoro-9-(4-methoxyphenyl)-10-(2,3,5,6-tetrafluorophenyl)anthracene;
Figure 364 illustrates a 2,3,5-trifluoro-9-(2,3,5,6-tetrafluorophenyl)-10-(2,3,5,6-tetrafluoro-4-methoxyphenyl)anthracene;
Figure 365 illustrates a 2,3,5-trifluoro-9-(2,3,5,6-tetrafluorophenyl)-10-(3,4,5-trifluorophenyl)anthracene;
Figure 366 illustrates a 9-(2,6-difluoro-4-methoxyphenyl)-2,3,5-trifluoro-10-(2,3,5,6-tetrafluorophenyl)anthracene;
Figure 367 illustrates a 9-(3,4-difluorophenyl)-2,3,5-trifluoro-10-(2,3,5,6-tetrafluorophenyl)anthracene;
Figure 368 illustrates a 2,3,5-trifluoro-9-[2,3,5,6-tetrafluoro-4-(trifluoromethyl)phenyl]-10-(2,3,,5,6-tetrafluorophenyl)anthracene;

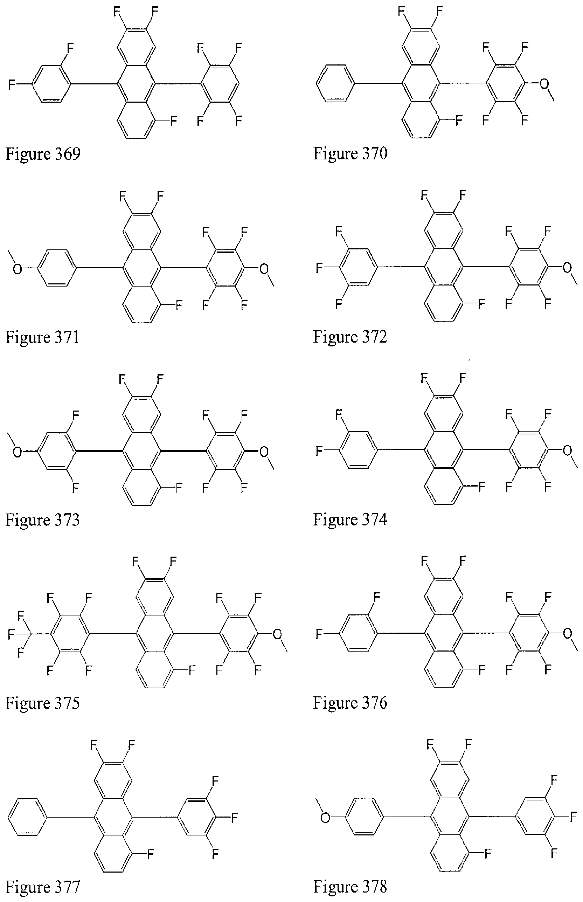

Figure 369 illustrates a 9-(3,4-difluorophenyl)-2,3,5-trifluoro-10-(2,3,5,6-tetrafluorophenyl)anthracene;
Figure 370 illustrates a 2,3,5-trifluoro-9-phenyl-10-(2,3,5,6-tetrafluoro-4-methoxyphenyl)anthracene;
Figure 371 illustrates a 2,3,5-trifluoro-9-(4-methoxyphenyl)-10-(2,3,5,6-tetrafluoro-4-methoxyphenyl)anthracene;
Figure 372 illustrates a 2,3,5-trifluoro-9-(2,3,5,6-tetrafluoro-4-methoxyphenyl)-10-(3,4,5-trifluorophenyl)anthracene;
Figure 373 illustrates a 9-(2,6-difluoro-4-methoxyphenyl)-2,3,5-trifluoro-10-(2,3,5,6-tetrafluoro-4-methoxyphenyl)anthracene;
Figure 374 illustrates a 9-(3,4-difluorophenyl)-2,3,5-trifluoro-10-(2,3,5,6-tetrafluoro-4-methoxyphenyl)anthracene;
Figure 375 illustrates a 2,3,5-trifluoro-9-[2,3,5,6-tetrafluoro-4-(trifluoromethyl)phenyl]-10-(2,3,5,6-tetrafluoro-4-methoxyphenyl)anthracene;
Figure 376 illustrates a 9-(2,4-difluorophenyl)-2,3,5-trifluoro-10-(2,3,5,6-tetrafluoro-4-methoxyphenyl)anthracene;
Figure 377 illustrates a 2,3,5-trifluoro-9-phenyl-10-(3,4,5-trifluorophenyl)anthracene;
Figure 378 illustrates a 2,3,5-trifluoro-9-(4-methoxyphenyl)-10-(3,4,5-trifluorophenyl)anthracene;

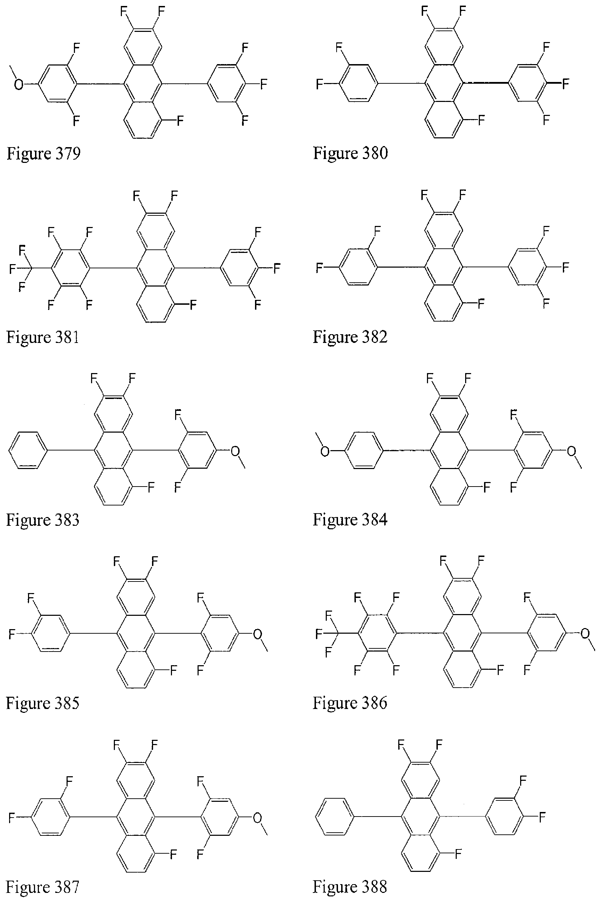

Figure 379 illustrates a 9-(2,6-difluoro-4-methoxyphenyl)-2,3,5-trifluoro-10-(3,4,5-trifluorophenyl)anthracene;
Figure 380 illustrates a 9-(3,4-difluorophenyl)-2,3,5-trifluoro-10-(3,4,5-trifluorophenyl)anthracene;
Figure 381 illustrates a 2,3,5-trifluoro-9-[2,3,5,6-tetrafluoro-4-(trifluoromethyl)phenyl]-10-(3,4,5-trifluorophenyl)anthracene;
Figure 382 illustrates a 9-(2,4-difluorophenyl)-2,3,5-trifluoro-10-(3,4,5-trifluorophenyl)anthracene;
Figure 383 illustrates a 9-(2,6-difluoro-4-methoxyphenyl)-2,3,5-trifluoro-10-phenylanthracene:
Figure 384 illustrates a 9-(2,6-difluoro-4-methoxyphenyl)-2,3,5-trifluoro-10-(4-methoxyphenyl)anthracene:
Figure 385 illustrates a 9-(2,6-difluoro-4-methoxyphenyl)-10-(3,4-difluorophenyl)-2,3,5-trifluoroanthracene;
Figure 386 illustrates a 9-(2,6-difluoro-4-methoxyphenyl)-2,3,5-trifluoro-10-[2,3,5,6-tetrafluoro-4-(trifluoromethyl)phenyl]anthracene;
Figure 387 illustrates a 9-(2,6-difluoro-4-methoxyphenyl)-2,3,5-trifluoro-10-(2,4-difluorophenyl)anthracene;
Figure 388 illustrates a 9-(3,4-difluorophenyl)-2,3,5-trifluoro-10-phenylanthracene;

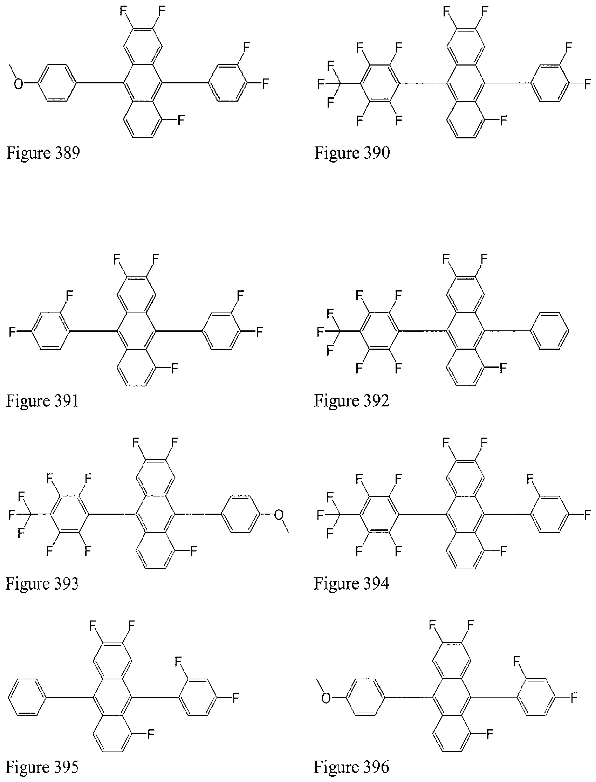

Figure 389 illustrates a 9-(3,4-difluorophenyl)-2,3,5-trifluoro-10-(4-methoxyphenyl)anthracene;
Figure 390 illustrates a 9-(3,4-difluorophenyl)-2,3,5-trifluoro-10-[2,3,5,6-tetrafluoro-4-(trifluoromethyl)phenyl]anthracene;
Figure 391 illustrates a 9-(2,4-difluorophenyl)-2,3,5-trifluoro-10-(3,4-difluorophenyl)anthracene;
Figure 392 illustrates a 2,3,5-trifluoro-9-phenyl-10-[2,3,5,6-tetrafluoro-4-(tri fluoromethyl)phenyl] anthracene;
Figure 393 illustrates a 2,3,5-trifluoro-9-(4-methoxyphenyl)-10-[2,3,5,6-tetrafluoro-4-(trifluoromethyl)phenyl]anthracene;
Figure 394 illustrates a 9-(2,4-difluorophenyl)-2,3,5-trifluoro-10-[2,3,5,6-tetrafluoro-4-(trifluoromethyl)phenyl]anthracene;
Figure 395 illustrates a 9-(2,4-difluorophenyl)-2,3,5-trifluoro-10-phenylanthracene;
Figure 396 illustrates a 9-(2,4-difluorophenyl)-2,3,5-trifluoro-10-(4-methoxyphenyl)anthracene;

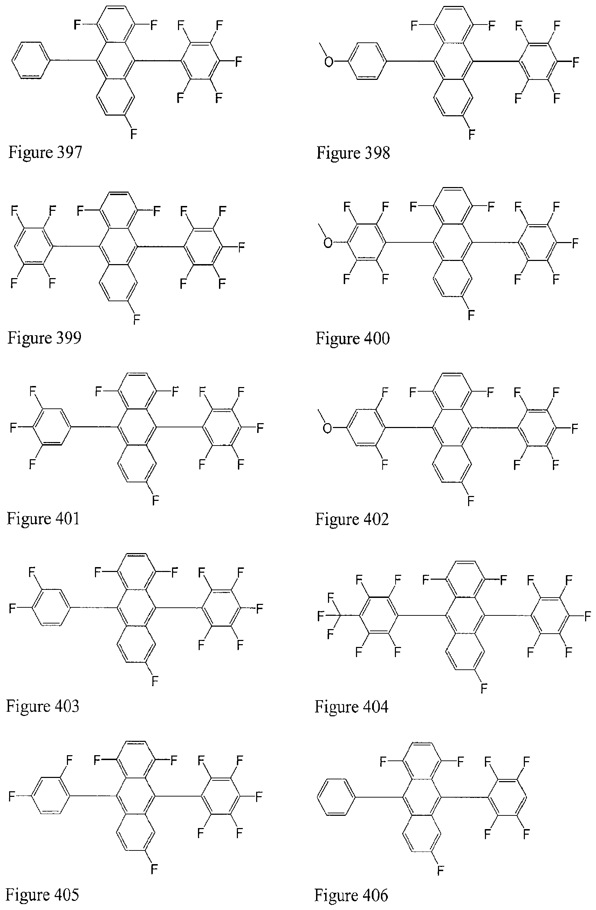

Figure 397 illustrates a 1,4,6-trifluoro-9-(2,3,4,5,6-pentafluorophenyl)-10-phenylanthracene;
Figure 398 illustrates a 1,4,6-trifluoro-9-(4-methoxyphenyl)-10-(2,3,4,5,6-pentafluorophenyl)anthracene;
Figure 399 illustrates a 1,4,6-trifluoro-9-(2,3,4,5,6-pentafluorophenyl)-10-(2,3,5,6-tetrafluoropheny)lanthracene;
Figure 400 illustrates a 1,4,6-trifluoro-9-(2,3,4,5,6-pentafluorophenyl)-10-(2,3,5,6-tetrafluoro-4-methoxyphenyl)anthracene;
Figure 401 illustrates a 1,4,6-trifluoro-9-(2,3,4,5,6-pentafluorophenyl)-10-(3,4,5-trifluoro)anthracene;
Figure 402 illustrates a 9-(2,6-difluoro-4-methoxyphenyl)-1,4,6-trifluoro-10-(2,3,4,5,6-pentafluorophenyl)anthracene
Figure 403 illustrates a 9-(3,4-difluorophenyl)-1,4,6-trifluoro-10-(2,3,4,5,6-pentafluorophenyl)anthracene;
Figure 404 illustrates a 1,4,6-trifluoro-9-(2,3,4,5,6-pentafluorophenyl)-10-[2,3,5,6-tetrafluoro-4-(trifluoromethyl)phenyl]anthracene;
Figure 405 illustrates a 9-(2,4-difluorophenyl)-1,4,6-trifluoro-10-(2,3,4,5,6-pentafluorophenyl)anthracene;
Figure 406 illustrates a 1,4,6-trifluoro-9-phenyl-10-(2,3,5,6-tetrafluorophenyl)anthracene;

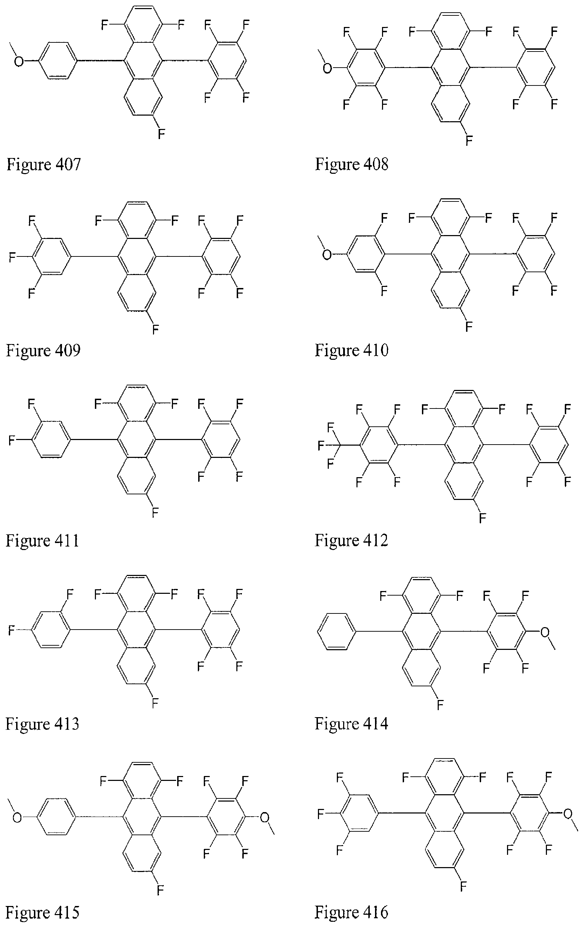

Figure 407 illustrates a 1,4,6-trifluoro-9-(4-methoxyphenyl)-10-(2,3,5,6-tetrafluorophenyl)anthracene;
Figure 408 illustrates a 1,4,6-trifluoro-9-(2,3,5,6-tetrafluorophenyl)-10-(2,3,5,6-tetrafluoro-4-methoxyphenyl)anthracene;
Figure 409 illustrates a 1,4,6-trifluoro-9-(2,3,5,6-tetrafluorophenyl)-10-(3,4,5-trifluorophenyl)anthracene;
Figure 410 illustrates a 9-(2,6-difluoro-4-methoxyphenyl)-1,4,6-trifluoro-10-(2,3,5,6-tetrafluorophenyl)anthracene;
Figure 411 illustrates a 9-(3,4-difluorophenyl)-1,4,6-trifluoro-10-(2,3,5,6-tetrafluorophenyl)anthracene;
Figure 412 illustrates a 1,4,6-trifluoro-9-[2,3,5,6-tetrafluoro-4-(trifluoromethyl)phenyl]-10-(2,3,,5,6-tetrafluorophenyl)anthracene;
Figure 413 illustrates a 9-(3,4-difluorophenyl)-1,4,6-trifluoro-10-(2,3,5,6-tetrafluorophenyl)anthracene;
Figure 414 illustrates a 1,4,6-trifluoro-9-phenyl-10-(2,3,5,6-tetrafluoro-4-methoxyphenyl)anthracene;
Figure 415 illustrates a 1,4,6-trifluoro-9-(4-methoxyphenyl)-10-(2,3,5,6-tetrafluoro-4-methoxyphenyl)anthracene;
Figure 416 illustrates a 1,4,6-trifluoro-9-(2,3,5,6-tetrafluoro-4-methoxyphenyl)-10-(3,4,5-trifluorophenyl)anthracene;

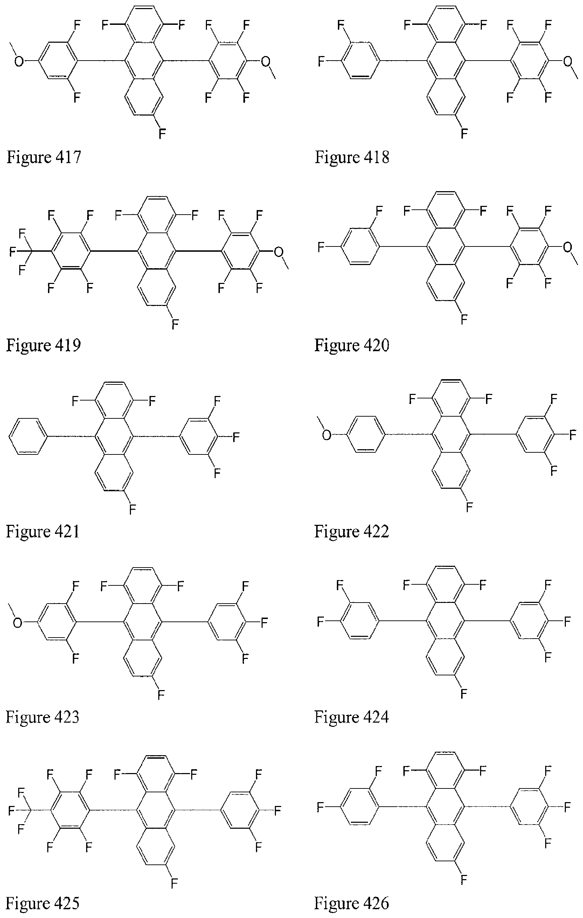

Figure 417 illustrates a 9-(2,6-difluoro-4-methoxyphenyl)-1,4,6-trifluoro-10-(2,3,5,6-tetrafluoro-4-methoxyphenyl)anthracene;
Figure 418 illustrates a 9-(3,4-difluorophenyl)-1,4,6-trifluoro-10-(2,3,5,6-tetrafluoro-4-methoxyphenyl)anthracene;
Figure 419 illustrates a 1,4,6-trifluoro-9-[2,3,5,6-tetrafluoro-4-(trifluoromethyl)phenyl]-10-(2,3,5,6-tetrafluoro-4-methoxyphenyl)anthracene;
Figure 420 illustrates a 9-(2,4-difluorophenyl)-1,4,6-trifluoro-10-(2,3,5,6-tetrafluoro-4-methoxyphenyl)anthracene;
Figure 421 illustrates a 1,4,6-trifluoro-9-phenyl-10-(3,4,5-trifluorophenyl)anthracene;
Figure 422 illustrates a 1,4,6-trifluoro-9-(4-methoxyphenyl)-10-(3,4,5-trifluorophenyl)anthracene;
Figure 423 illustrates a 9-(2,6-difluoro-4-methoxyphenyl)-1,4,6-trifluoro-10-(3,4,5-trifluorophenyl)anthracene;
Figure 424 illustrates a 9-(3,4-difluorophenyl)-1,4,6-trifluoro-10-(3,4,5-trifluorophenyl)anthracene;
Figure 425 illustrates a 1,4,6-trifluoro-9-[2,3,5,6-tetrafluoro-4-(trifluoromethyl)phenyl]-10-(3,4,5-trifluorophenyl)anthracene;
Figure 426 illustrates a 9-(2,4-difluorophenyl)-1,4,6-trifluoro-10-(3,4,5-trifluorophenyl)anthracene;

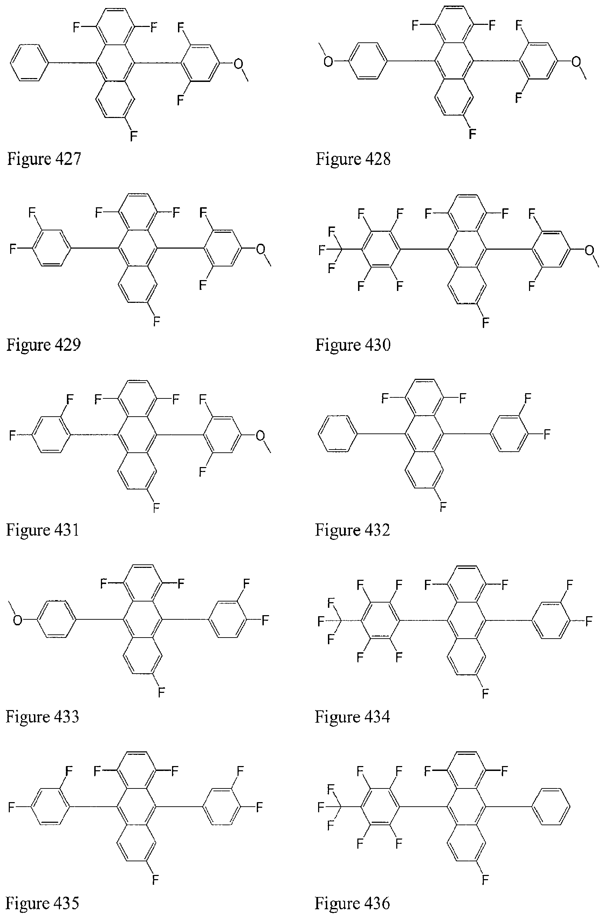

Figure 427 illustrates a 9-(2,6-difluoro-4-methoxyphenyl)-1,4,6-trifluoro-10-phenylanthracene:
Figure 428 illustrates a 9-(2,6-difluoro-4-methoxyphenyl)-1,4,6-trifluoro-10-(4-methoxyphenyl)anthracene:
Figure 429 illustrates a 9-(2,6-difluoro-4-methoxyphenyl)-10-(3,4-difluorophenyl)-1,4,6-trifluoroanthracene;
Figure 430 illustrates a 9-(2,6-difluoro-4-methoxyphenyl)-1,4,6-trifluoro-10-[2,3,5,6-tetrafluoro-4-(trifluoromethyl)phenyl]anthracene;
Figure 431 illustrates a 9-(2,6-difluoro-4-methoxyphenyl)-1,4,6-trifluoro-10-(2,4-difluorophenyl)anthracene;
Figure 432 illustrates a 9-(3,4-difluorophenyl)-1,4,6-trifluoro-10-phenylanthracene;
Figure 433 illustrates a 9-(3,4-difluorophenyl)-1,4,6-trifluoro-10-(4-methoxyphenyl)anthracene;
Figure 434 illustrates a 9-(3,4-difluorophenyl)-1,4,6-trifluoro-10-[2,3,5,6-tetrafluoro-4-(trifluoromethyl)phenyl]anthracene;
Figure 435 illustrates a 9-(2,4-difluorophenyl)-1,4,6-trifluoro-10-(3,4-difluorophenyl)anthracene;
Figure 436 illustrates a 1,4,6-trifluoro-9-phenyl-10-[2,3,5,6-tetrafluoro-4-(trifluoromethyl)phenyl]anthracene;

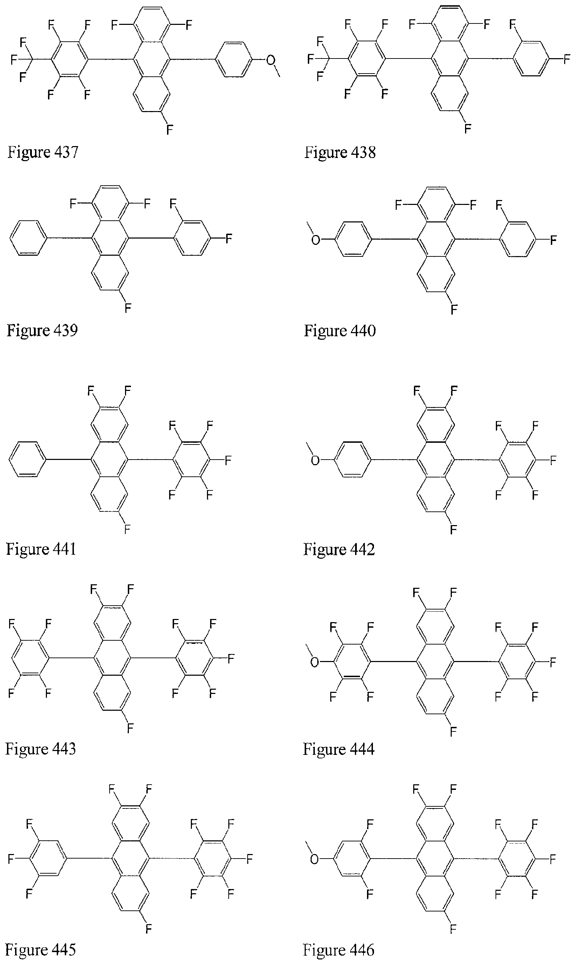

Figure 437 illustrates a 1,4,6-trifluoro-9-(4-methoxyphenyl)-10-[2,3,5,6-tetrafluoro-4-(trifluoromethyl)phenyl]anthracene;
Figure 438 illustrates a 9-(2,4-difluorophenyl)-1,4,6-trifluoro-10-[2,3,5,6-tetrafluoro-4-(trifluoromethyl)phenyl]anthracene;
Figure 439 illustrates a 9-(2,4-difluorophenyl)-1,4,6-trifluoro-10-phenylanthracene;
Figure 440 illustrates a 9-(2,4-difluorophenyl)-1,4,6-trifluoro-10-(4-methoxyphenyl)anthracene;
Figure 441 illustrates a 2,3,6-trifluoro-9-(2,3,4,5,6-pentafluorophenyl)-10-phenylanthracene;
Figure 442 illustrates a 2,3,6-trifluoro-9-(4-methoxyphenyl)-10-(2,3,4,5,6-pentafluorophenyl)anthracene;
Figure 443 illustrates a 2,3,6-trifluoro-9-(2,3,4,5,6-pentafluorophenyl)-10-(2,3,5,6-tetrafluoropheny)lanthracene;
Figure 444 illustrates a 2,3,6-trifluoro-9-(2,3,4,5,6-pentafluorophenyl)-10-(2,3,5,6-tetrafluoro-4-methoxyphenyl)anthracene;
Figure 445 illustrates a 2,3,6-trifluoro-9-(2,3,4,5,6-pentafluorophenyl)-10-(3,4,5-trifluoro)anthracene;
Figure 446 illustrates a 9-(2,6-difluoro-4-methoxyphenyl)-2,3,6-trifluoro-10-(2,3,4,5,6-pentafluorophenyl)anthracene

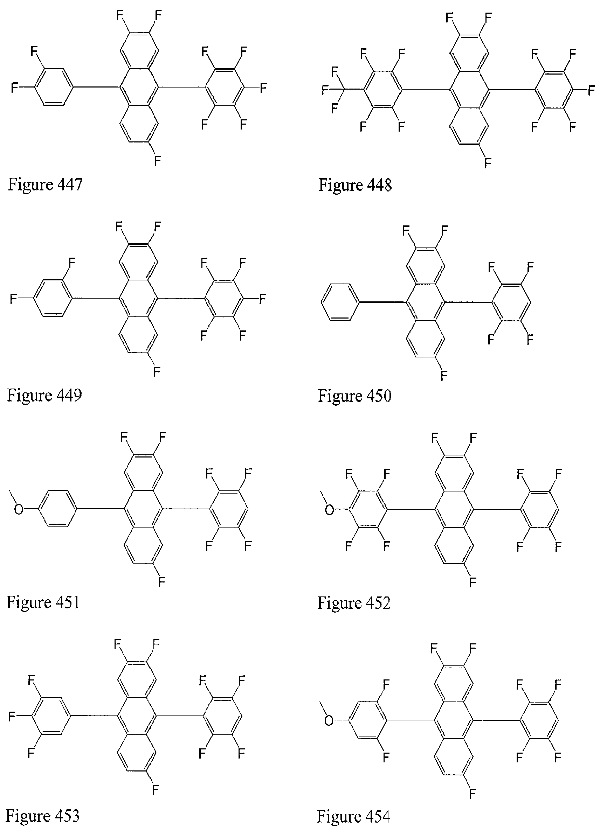

Figure 447 illustrates a 9-(3,4-difluorophenyl)-2,3,6-trifluoro-10-(2,3,4,5,6-pentafluorophenyl)anthracene;
Figure 448 illustrates a 2,3,6-trifluoro-9-(2,3,4,5,6-pentafluorophenyl)-10-[2,3,5,6-tetrafluoro-4-(trifluoromethyl)phenyl]anthracene;
Figure 449 illustrates a 9-(2,4-difluorophenyl)-2,3,6-trifluoro-10-(2,3,4,5,6-pentafluorophenyl)anthracene;
Figure 450 illustrates a 2,3,6-trifluoro-9-phenyl-10-(2,3,5,6-tetrafluorophenyl)anthracene;
Figure 451 illustrates a 2,3,6-trifluoro-9-(4-methoxyphenyl)-10-(2,3,5,6-tetrafluorophenyl)anthracene;
Figure 452 illustrates a 2,3,6-trifluoro-9-(2,3,5,6-tetrafluorophenyl)-10-(2,3,5,6-tetrafluoro-4-methoxyphenyl)anthracene;
Figure 453 illustrates a 2,3,6-trifluoro-9-(2,3,5,6-tetrafluorophenyl)-10-(3,4,5-trifluorophenyl)anthracene;
Figure 454 illustrates a 9-(2,6-difluoro-4-methoxyphenyl)-2,3,6-trifluoro-10-(2,3,5,6-tetrafluorophenyl)anthracene;

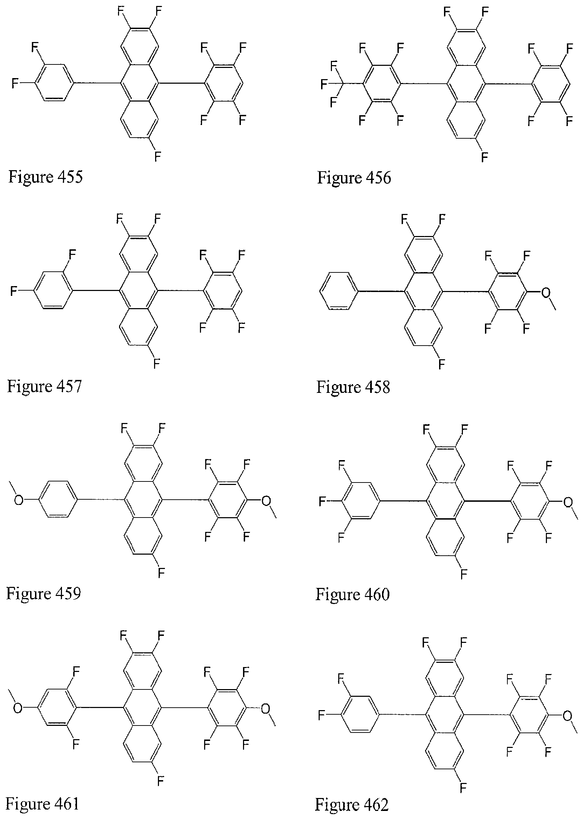

Figure 455 illustrates a 9-(3,4-difluorophenyl)-2,3,6-trifluoro-10-(2,3,5,6-tetrafluorophenyl)anthracene;
Figure 456 illustrates a 2,3,6-trifluoro-9-[2,3,5,6-tetrafluoro-4-(trifluoromethyl)phenyl]-10-(2,3,,5,6-tetrafluorophenyl)anthracene;
Figure 457 illustrates a 9-(3,4-difluorophenyl)-2,3,6-trifluoro-10-(2,3,5,6-tetrafluorophenyl)anthracene;
Figure 458 illustrates a 2,3,6-trifluoro-9-phenyl-10-(2,3,5,6-tetrafluoro-4-methoxyphenyl)anthracene;
Figure 459 illustrates a 2,3,6-trifluoro-9-(4-methoxyphenyl)-10-(2,3,5,6-tetrafluoro-4-methoxyphenyl)anthracene;
Figure 460 illustrates a 2,3,6-trifluoro-9-(2,3,5,6-tetrafluoro-4-methoxyphenyl)-10-(3,4,5-trifluorophenyl)anthracene;
Figure 461 illustrates a 9-(2,6-difluoro-4-methoxyphenyl)-2,3,6-trifluoro-10-(2,3,5,6-tetrafluoro-4-methoxyphenyl)anthracene;
Figure 462 illustrates a 9-(3,4-difluorophenyl)-2,3,6-trifluoro-10-(2,3,5,6-tetrafluoro-4-methoxyphenyl)anthracene;

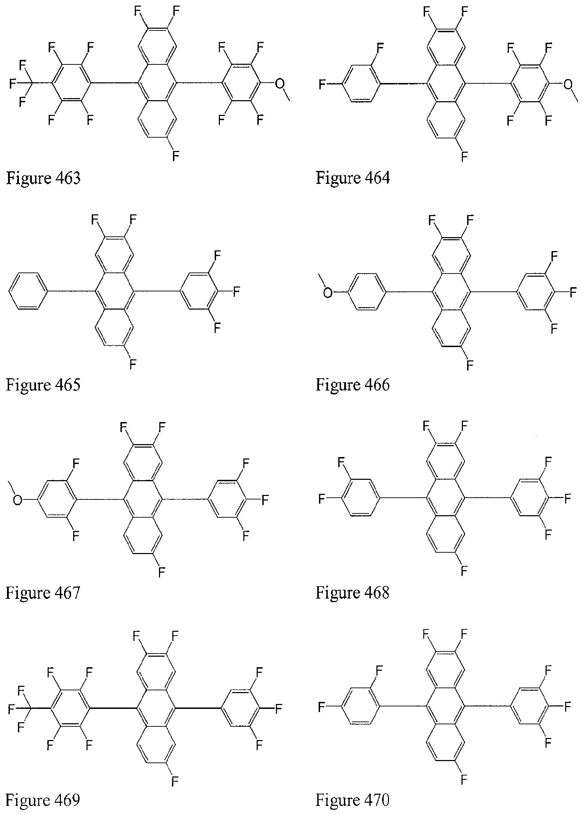

Figure 463 illustrates a 2,3,6-trifluoro-9-[2,3,5,6-tetrafluoro-4-(trifluoromethyl)phenyl]-10-(2,3,5,6-tetrafluoro-4-methoxyphenyl)anthracene;
Figure 464 illustrates a 9-(2,4-difluorophenyl)-2,3,6-trifluoro-10-(2,3,5,6-tetrafluoro-4-methoxyphenyl)anthracene;
Figure 465 illustrates a 2,3,6-trifluoro-9-phenyl-10-(3,4,5-trifluorophenyl)anthracene;
Figure 466 illustrates a 2,3,6-trifluoro-9-(4-methoxyphenyl)-10-(3,4,5-trifluorophenyl)anthracene;
Figure 467 illustrates a 9-(2,6-difluoro-4-methoxyphenyl)-2,3,6-trifluoro-10-(3,4,5-trifluorophenyl)anthracene;
Figure 468 illustrates a 9-(3,4-difluorophenyl)-2,3,6-trifluoro-10-(3,4,5-trifluorophenyl)anthracene;
Figure 469 illustrates a 2,3,6-trifluoro-9-[2,3,5,6-tetrafluoro-4-(trifluoromethyl)phenyl]-10-(3,4,5-trifluorophenyl)anthracene;
Figure 470 illustrates a 9-(2,4-difluorophenyl)-2,3,6-trifluoro-10-(3,4,5-trifluorophenyl)anthracene;

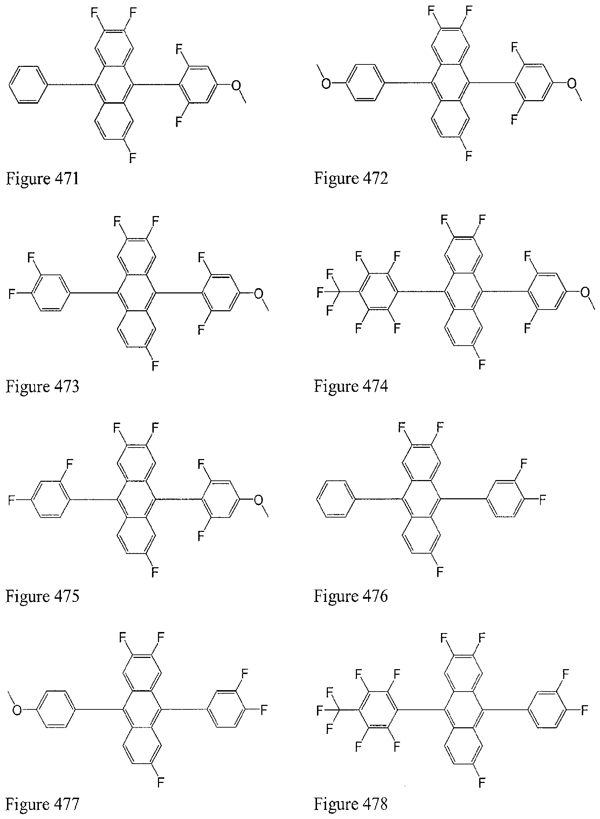

Figure 471 illustrates a 9-(2,6-difluoro-4-methoxyphenyl)-2,3,6-trifluoro-10-phenyl anthracene:
Figure 472 illustrates a 9-(2,6-difluoro-4-methoxyphenyl)-2,3,6-trifluoro-10-(4-methoxyphenyl)anthracene:
Figure 473 illustrates a 9-(2,6-difluoro-4-methoxyphenyl)-10-(3,4-difluorophenyl)-2,3,6-trifluoroanthracene;
Figure 474 illustrates a 9-(2,6-difluoro-4-methoxyphenyl)-2,3,6-trifluoro-10-[2,3,5,6-tetrafluoro-4-(trifluoromethyl)phenyl]anthracene;
Figure 475 illustrates a 9-(2,6-difluoro-4-methoxyphenyl)-2,3,6-trifluoro-10-(2,4-difluorophenyl)anthracene;
Figure 476 illustrates a 9-(3,4-difluorophenyl)-2,3,6-trifluoro-10-phenylanthracene;
Figure 477 illustrates a 9-(3,4-difluorophenyl)-2,3,6-trifluoro-10-(4-methoxyphenyl)anthracene;
Figure 478 illustrates a 9-(3,4-difluorophenyl)-2,3,6-trifluoro-10-[2,3,5,6-tetrafluoro-4-(trifluoromethyl)phenyl]anthracene;

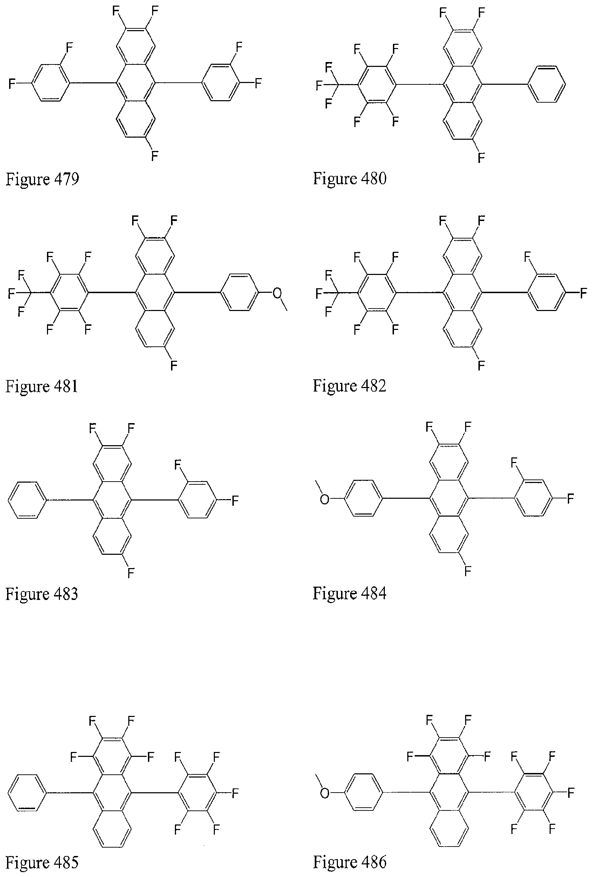

Figure 479 illustrates a 9-(2,4-difluorophenyl)-2,3,6-trifluoro-10-(3,4-difluorophenyl)anthracene;
Figure 480 illustrates a 2,3,6-trifluoro-9-phenyl-10-[2,3,5,6-tetrafluoro-4-(trifluoromethyl)phenyl]anthracene;
Figure 481 illustrates a 2,3,6-trifluoro-9-(4-methoxyphenyl)-10-[2,3,5,6-tetrafluoro-4-(trifluoromethyl)phenyl]anthracene;
Figure 482 illustrates a 9-(2,4-difluorophenyl)-2,3,6-trifluoro-10-[2,3,5,6-tetrafluoro-4-(trifluoromethyl)phenyl]anthracene;
Figure 483 illustrates a 9-(2,4-difluorophenyl)-2,3,6-trifluoro-10-phenylanthracene;
Figure 484 illustrates a 9-(2,4-difluorophenyl)-2,3,6-trifluoro-10-(4-methoxyphenyl)anthracene;
Figure 485 illustrates a 1,2,3,4-tetrafluoro-9-(2,3,4,5,6-pentafluorophenyl)-10-phenylanthracene;
Figure 486 illustrates a 1,2,3,4-tetrafluoro-9-(4-methoxyphenyl)-10-(2,3,4,5,6-pentafluorophenyl)anthracene;

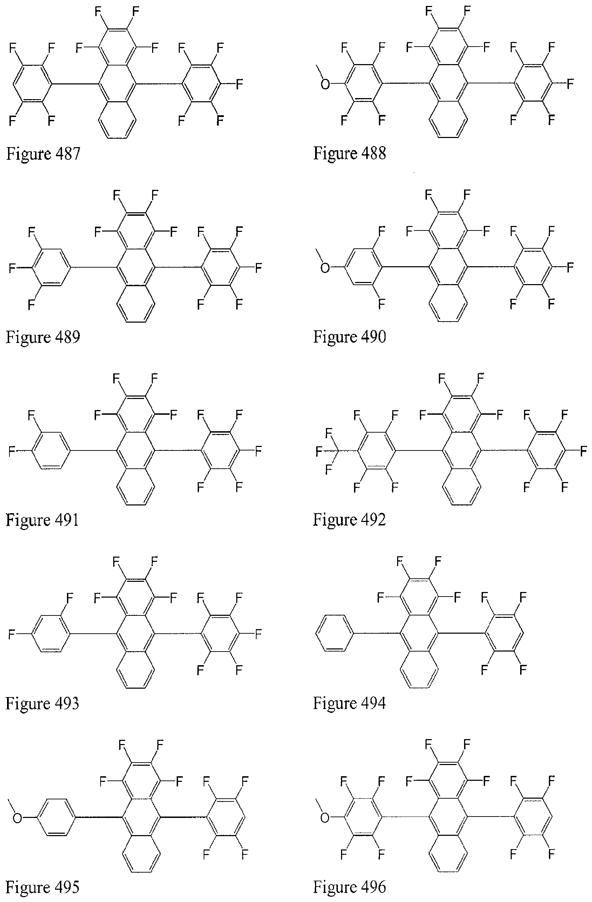

Figure 487 illustrates a 1,2,3,4-tetrafluoro-9-(2,3,4,5,6-pentafluorophenyl)-10-(2,3,5,6-tetrafluoropheny)lanthracene;
Figure 488 illustrates a 1,2,3,4-tetrafluoro-9-(2,3,4,5,6-pentafluorophenyl)-10-(2,3,5,6-tetrafluoro-4-methoxyphenyl)anthracene;
Figure 489 illustrates a 1,2,3,4-tetrafluoro-9-(2,3,4,5,6-pentafluorophenyl)-10-(3,4,5-trifluoro)anthracene;
Figure 490 illustrates a 9-(2,6-difluoro-4-methoxyphenyl)-1,2,3,4-tetrafluoro-10-(2,3,4,5,6-pentafluorophenyl)anthracene
Figure 491 illustrates a 9-(3,4-difluorophenyl)-1,2,3,4-tetrafluoro-10-(2,3,4,5,6-pentafluorophenyl)anthracene;
Figure 492 illustrates a 1,2,3,4-tetrafluoro-9-(2,3,4,5,6-pentafluorophenyl)-10-[2,3,5,6-tetrafluoro-4-(trifluoromethyl)phenyl]anthracene;
Figure 493 illustrates a 9-(2,4-difluorophenyl)-1,2,3,4-tetrafluoro-10-(2,3,4,5,6-pentafluorophenyl)anthracene;
Figure 494 illustrates a 1,2,3,4-tetrafluoro-9-phenyl-10-(2,3,5,6-tetrafluorophenyl)anthracene;
Figure 495 illustrates a 1,2,3,4-tetrafluoro-9-(4-methoxyphenyl)-10-(2,3,5,6-tetrafluorophenyl)anthracene;
Figure 496 illustrates a 1,2,3,4-tetrafluoro-9-(2,3,5,6-tetrafluorophenyl)-10-(2,3,5,6-tetrafluoro-4-methoxyphenyl)anthracene;

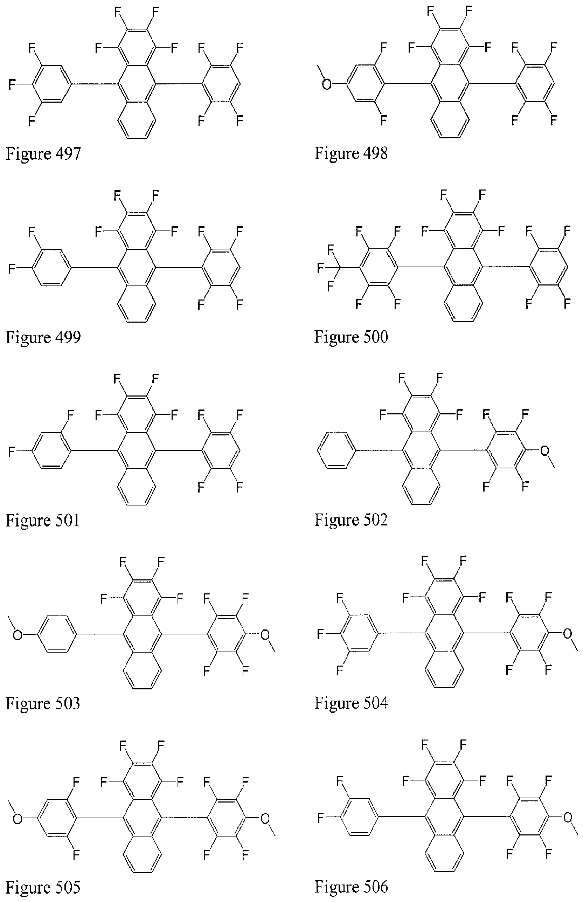

Figure 497 illustrates a 1,2,3,4-tetrafluoro-9-(2,3,5,6-tetrafluorophenyl)-10-(3,4,5-trifluorophenyl)anthracene;
Figure 498 illustrates a 9-(2,6-difluoro-4-methoxyphenyl)-1,2,3,4-tetrafluoro-10-(2,3,5,6-tetrafluorophenyl)anthracene;
Figure 499 illustrates a 9-(3,4-difluorophenyl)-1,2,3,4-tetrafluoro-10-(2,3,5,6-tetrafluorophenyl)anthracene;
Figure 500 illustrates a 1,2,3,4-tetrafluoro-9-[2,3,5,6-tetrafluoro-4-(trifluoromethyl)phenyl]-10-(2,3,,5,6-tetrafluorophenyl)anthracene;
Figure 501 illustrates a 9-(3,4-difluorophenyl)-1,2,3,4-tetrafluoro-10-(2,3,5,6-tetrafluorophenyl)anthracene;
Figure 502 illustrates a 1,2,3,4-tetrafluoro-9-phenyl-10-(2,3,5,6-tetrafluoro-4-methoxyphenyl)anthracene;
Figure 503 illustrates a 1,2,3,4-tetrafluoro-9-(4-methoxyphenyl)-10-(2,3,5,6-tetrafluoro-4-methoxyphenyl)anthracene;
Figure 504 illustrates a 1,2,3,4-tetrafluoro-9-(2,3,5,6-tetrafluoro-4-methoxyphenyl)-10-(3,4,5-trifluorophenyl)anthracene;
Figure 505 illustrates a 9-(2,6-difluoro-4-methoxyphenyl)-1,2,3,4-tetrafluoro-10-(2,3,5,6-tetrafluoro-4-methoxyphenyl)anthracene;
Figure 506 illustrates a 9-(3,4-difluorophenyl)-1,2,3,4-tetrafluoro-10-(2,3,5,6-tetrafluoro-4-methoxyphenyl)anthracene;

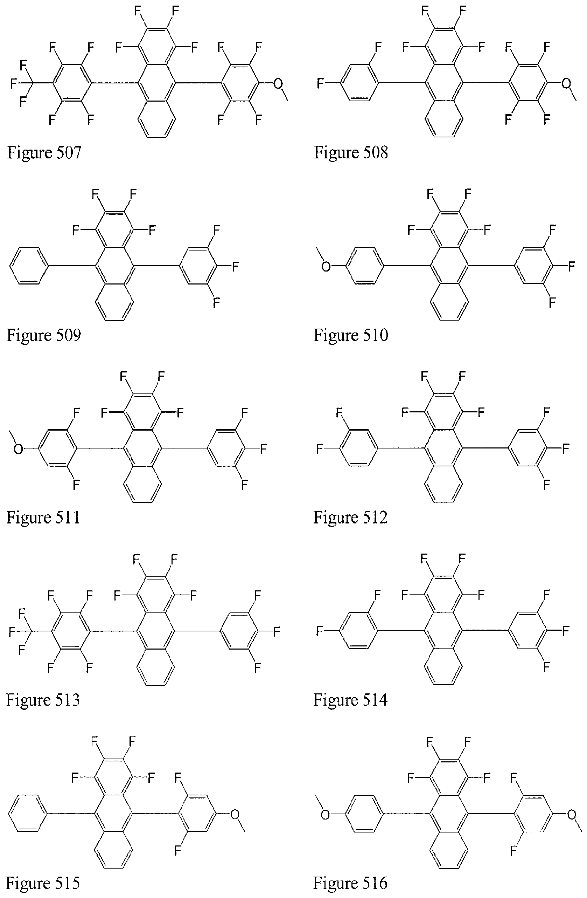

Figure 507 illustrates a 1,2,3,4-tetrafluoro-9-[2,3,5,6-tetrafluoro-4-(trifluoromethyl)phenyl]-10-(2,3,5,6-tetrafluoro-4-methoxyphenyl)anthracene;
Figure 508 illustrates a 9-(2,4-difluorophenyl)-1,2,3,4-tetrafluoro-10-(2,3,5,6-tetrafluoro-4-methoxyphenyl)anthracene;
Figure 509 illustrates a 1,2,3,4-tetrafluoro-9-phenyl-10-(3,4,5-trifluorophenyl)anthracene;
Figure 510 illustrates a 1,2,3,4-tetrafluoro-9-(4-methoxyphenyl)-10-(3,4,5-trifluorophenyl)anthracene;
Figure 511 illustrates a 9-(2,6-difluoro-4-methoxyphenyl)-1,2,3,4-tetrafluoro-10-(3,4,5-trifluorophenyl)anthracene;
Figure 512 illustrates a 9-(3,4-difluorophenyl)-1,2,3,4-tetrafluoro-10-(3,4,5-trifluorophenyl)anthracene;
Figure 513 illustrates a 1,2,3,4-tetrafluoro-9-[2,3,5,6-tetrafluoro-4-(trifluoromethyl)phenyl]-10-(3,4,5-trifluorophenyl)anthracene;
Figure 514 illustrates a 9-(2,4-difluorophenyl)-1,2,3,4-tetrafluoro-10-(3,4,5-trifluorophenyl)anthracene;
Figure 515 illustrates a 9-(2,6-difluoro-4-methoxyphenyl)-1,2,3,4-tetrafluoro-10-phenylanthracene:
Figure 516 illustrates a 9-(2,6-difluoro-4-methoxyphenyl)-1,2,3,4-tetrafluoro-10-(4-methoxyphenyl)anthracene:

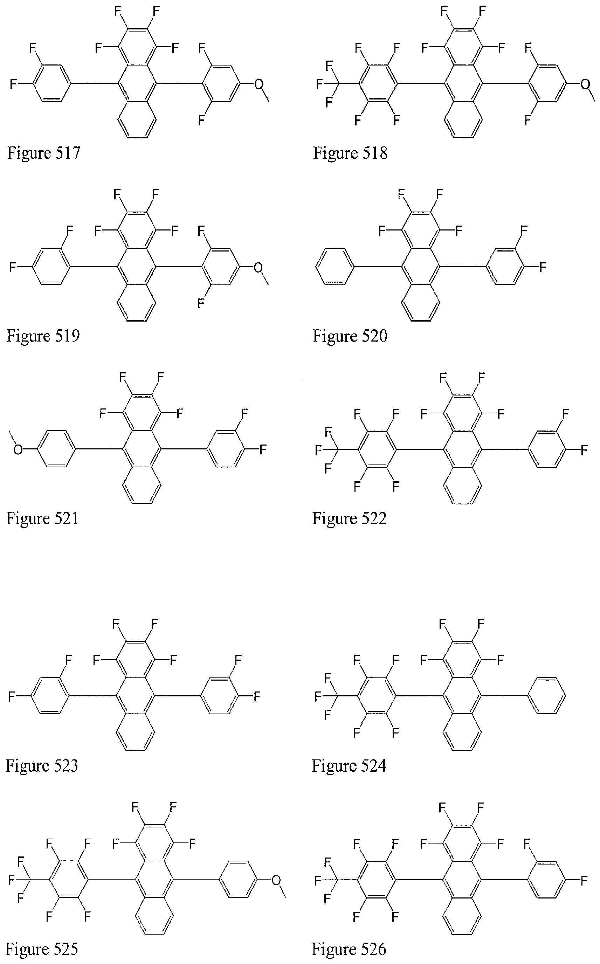

Figure 517 illustrates a 9-(2,6-difluoro-4-methoxyphenyl)-10-(3,4-difluorophenyl)-1,2,3,4-tetrafluoroanthracene;
Figure 518 illustrates a 9-(2,6-difluoro-4-methoxyphenyl)-1,2,3,4-tetrafluoro-10-[2,3,5,6-tetrafluoro-4-(trifluoromethyl)phenyl]anthracene;
Figure 519 illustrates a 9-(2,6-difluoro-4-methoxyphenyl)-1,2,3,4-tetrafluoro-10-(2,4-difluorophenyl)anthracene;
Figure 520 illustrates a 9-(3,4-difluorophenyl)-1,2,3,4-tetrafluoro-10-phenyl anthracene;
Figure 521 illustrates a 9-(3,4-difluorophenyl)-1,2,3,4-tetrafluoro-10-(4-methoxyphenyl)anthracene;
Figure 522 illustrates a 9-(3,4-difluorophenyl)-1,2,3,4-tetrafluoro-10-[2,3,5,6-tetrafluoro-4-(trifluoromethyl)phenyl]anthracene;
Figure 523 illustrates a 9-(2,4-difluorophenyl)-1,2,3,4-tetrafluoro-10-(3,4-difluorophenyl)anthracene;
Figure 524 illustrates a 1,2,3,4-tetrafluoro-9-phenyl-10-[2,3,5,6-tetrafluoro-4-(trifluoromethyl)phenyl]anthracene;
Figure 525 illustrates a 1,2,3,4-tetrafluoro-9-(4-methoxyphenyl)-10-[2,3,5,6-tetrafluoro-4-(trifluoromethyl)phenyl]anthracene;
Figure 526 illustrates a 9-(2,4-difluorophenyl)-1,2,3,4-tetrafluoro-10-[2,3,5,6-tetrafluoro-4-(trifluoromethyl)phenyl]anthracene;

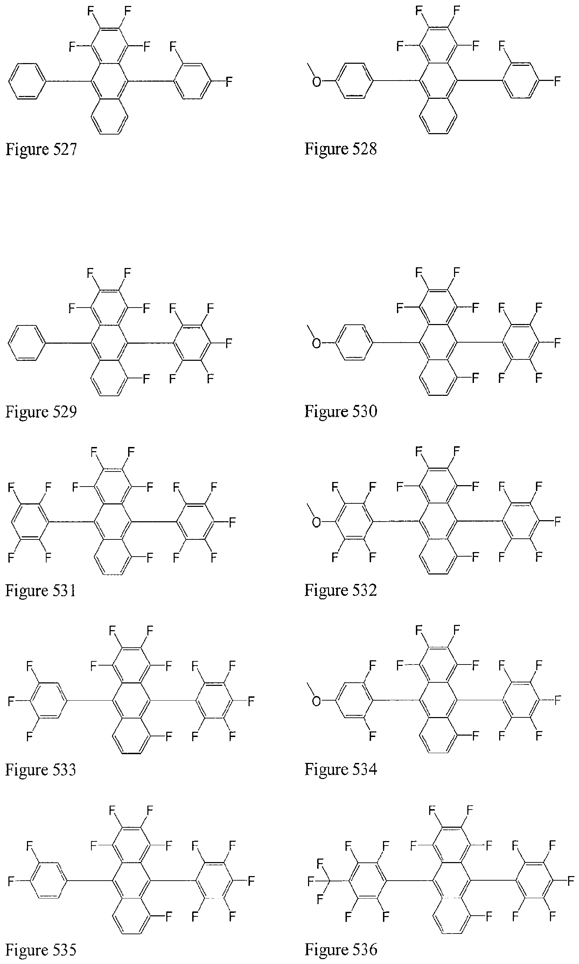

Figure 527 illustrates a 9-(2,4-difluorophenyl)-1,2,3,4-tetrafluoro-10-phenyl anthracene;
Figure 528 illustrates a 9-(2,4-difluorophenyl)-1,2,3,4-tetrafluoro-10-(4-methoxyphenyl)anthracene;
Figure 529 illustrates a 1,2,3,4,5-pentafluoro-9-(2,3,4,5,6-pentafluorophenyl)-10-phenylanthracene;
Figure 530 illustrates a 1,2,3,4,5-pentafluoro-9-(4-methoxyphenyl)-10-(2,3,4,5,6-pentafluorophenyl)anthracene;
Figure 531 illustrates a 1,2,3,4,5-pentafluoro-9-(2,3,4,5,6-pentafluorophenyl)-10-(2,3,5,6-tetrafluoropheny)lanthracene;
Figure 532 illustrates a 1,2,3,4,5-pentafluoro-9-(2,3,4,5,6-pentafluorophenyl)-10-(2,3,5,6-tetrafluoro-4-methoxyphenyl)anthracene;
Figure 533 illustrates a 1,2,3,4,5-pentafluoro-9-(2,3,4,5,6-pentafluorophenyl)-10-(3,4,5-trifluoro)anthracene;
Figure 534 illustrates a 9-(2,6-difluoro-4-methoxyphenyl)-1,2,3,4,5-pentafluoro-10-(2,3,4,5,6-pentafluorophenyl)anthracene
Figure 535 illustrates a 9-(3,4-difluorophenyl)-1,2,3,4,5-pentafluoro-10-(2,3,4,5,6-pentafluorophenyl)anthracene;
Figure 536 illustrates a 1,2,3,4,5-pentafluoro-9-(2,3,4,5,6-pentafluorophenyl)-10-[2,3,5,6-tetrafluoro-4-(trifluoromethyl)phenyl]anthracene;

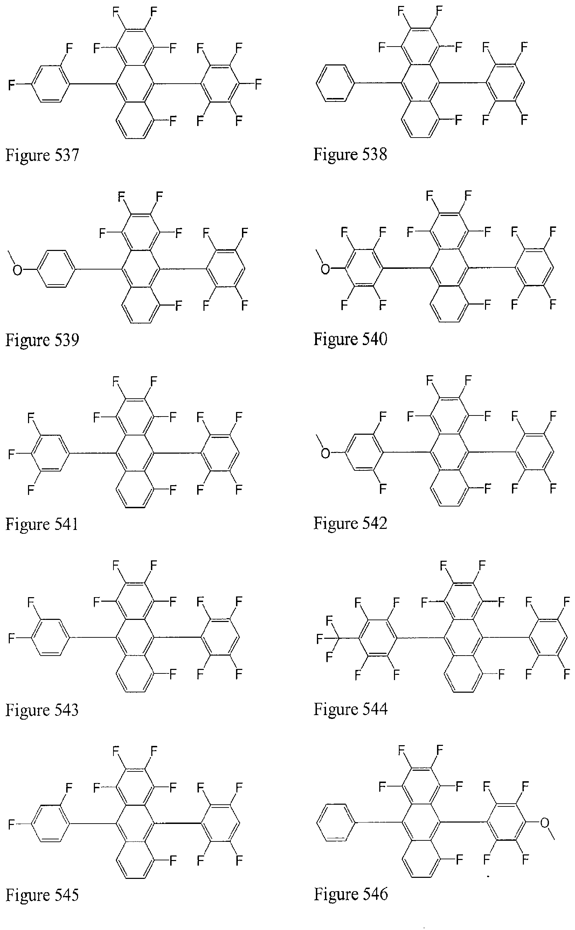

Figure 537 illustrates a 9-(2,4-difluorophenyl)-1,2,3,4,5-pentafluoro-10-(2,3,4,5,6-pentafluorophenyl)anthracene;
Figure 538 illustrates a 1,2,3,4,5-pentafluoro-9-phenyl-10-(2,3,5,6-tetrafluorophenyl)anthracene;
Figure 539 illustrates a 1,2,3,4,5-pentafluoro-9-(4-methoxyphenyl)-10-(2,3,5,6-tetrafluorophenyl)anthracene;
Figure 540 illustrates a 1,2,3,4,5-pentafluoro-9-(2,3,5,6-tetrafluorophenyl)-10-(2,3,5,6-tetrafluoro-4-methoxyphenyl)anthracene;
Figure 541 illustrates a 1,2,3,4,5-pentafluoro-9-(2,3,5,6-tetrafluorophenyl)-10-(3,4,5-trifluorophenyl)anthracene;
Figure 542 illustrates a 9-(2,6-difluoro-4-methoxyphenyl)-1,2,3,4,5-pentafluoro-10-(2,3,5,6-tetrafluorophenyl)anthracene;
Figure 543 illustrates a 9-(3,4-difluorophenyl)-1,2,3,4,5-pentafluoro-10-(2,3,5,6-tetrafluorophenyl)anthracene;
Figure 544 illustrates a 1,2,3,4,5-pentafluoro-9-[2,3,5,6-tetrafluoro-4-(trifluoromethyl)phenyl]-10-(2,3,,5,6-tetrafluorophenyl)anthracene;
Figure 545 illustrates a 9-(3,4-difluorophenyl)-1,2,3,4,5-pentafluoro-10-(2,3,5,6-tetrafluorophenyl)anthracene;
Figure 546 illustrates a 1,2,3,4,5-pentafluoro-9-phenyl-10-(2,3,5,6-tetrafluoro-4-methoxyphenyl)anthracene;

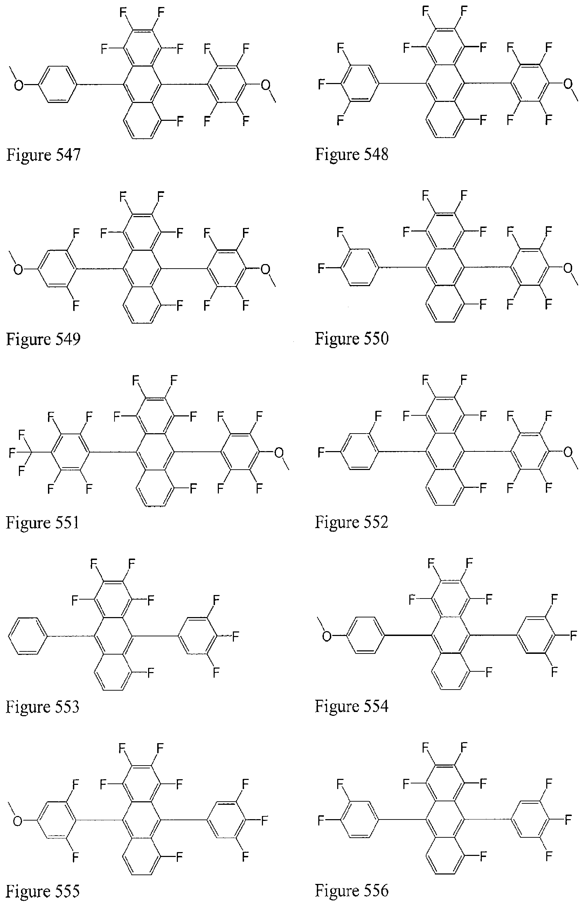

Figure 547 illustrates a 1,2,3,4,5-pentafluoro-9-(4-methoxyphenyl)-10-(2,3,5,6-tetrafluoro-4-methoxyphenyl)anthracene;
Figure 548 illustrates a 1,2,3,4,5-pentafluoro-9-(2,3,5,6-tetrafluoro-4-methoxyphenyl)-10-(3,4,5-trifluorophenyl)anthracene;
Figure 549 illustrates a 9-(2,6-difluoro-4-methoxyphenyl)-1,2,3,4,5-pentafluoro-10-(2,3,5,6-tetrafluoro-4-methoxyphenyl)anthracene;
Figure 550 illustrates a 9-(3,4-difluorophenyl)-1,2,3,4,5-pentafluoro-10-(2,3,5,6-tetrafluoro-4-methoxyphenyl)anthracene;
Figure 551 illustrates a 1,2,3,4,5-pentafluoro-9-[2,3,5,6-tetrafluoro-4-(trifluoromethyl)phenyl]-10-(2,3,5,6-tetrafluoro-4-methoxyphenyl)anthracene;
Figure 552 illustrates a 9-(2,4-difluorophenyl)-1,2,3,4,5-pentafluoro-10-(2,3,5,6-tetrafluoro-4-methoxyphenyl)anthracene;
Figure 553 illustrates a 1,2,3,4,5-pentafluoro-9-phenyl-10-(3,4,5-trifluorophenyl)anthracene;
Figure 554 illustrates a 1,2,3,4,5-pentafluoro-9-(4-methoxyphenyl)-10-(3,4,5-trifluorophenyl)anthracene;
Figure 555 illustrates a 9-(2,6-difluoro-4-methoxyphenyl)-1,2,3,4,5-pentafluoro-10-(3,4,5-trifluorophenyl)anthracene;
Figure 556 illustrates a 9-(3,4-difluorophenyl)-1,2,3,4,5-pentafluoro-10-(3,4,5-trifluorophenyl)anthracene;

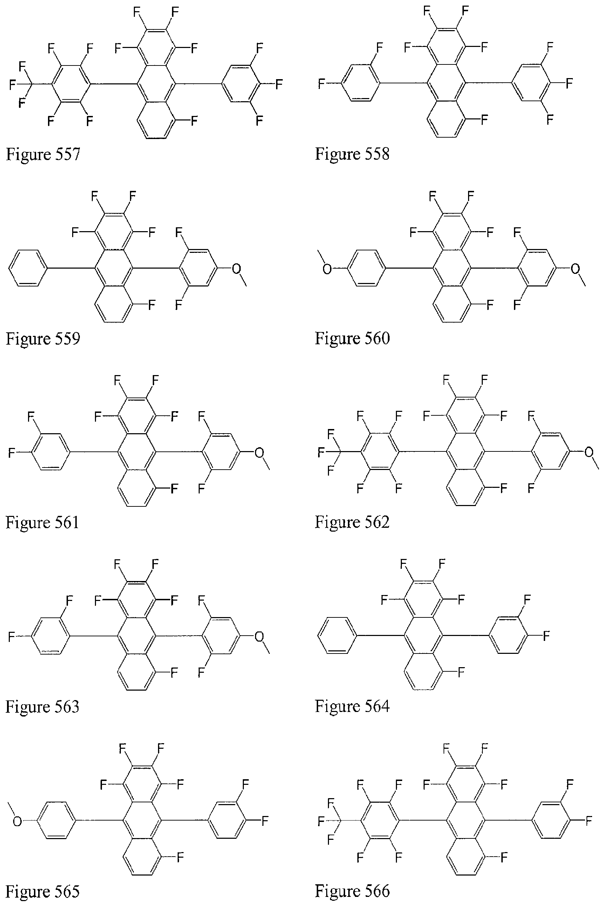

Figure 557 illustrates a 1,2,3,4,5-pentafluoro-9-[2,3,5,6-tetrafluoro-4-(trifluoromethyl)phenyl]-10-(3,4,5-trifluorophenyl)anthracene;
Figure 558 illustrates a 9-(2,4-difluorophenyl)-1,2,3,4,5-pentafluoro-10-(3,4,5-trifluorophenyl)anthracene;
Figure 559 illustrates a 9-(2,6-difluoro-4-methoxyphenyl)-1,2,3,4,5-pentafluoro-10-phenylanthracene:
Figure 560 illustrates a 9-(2,6-difluoro-4-methoxyphenyl)-1,2,3,4,5-pentafluoro-10-(4-methoxyphenyl)anthracene:
Figure 561 illustrates a 9-(2,6-difluoro-4-methoxyphenyl)-10-(3,4-difluorophenyl)-1,2,3,4,5-pentafluoroanthracene;
Figure 562 illustrates a 9-(2,6-difluoro-4-methoxyphenyl)-1,2,3,4,5-pentafluoro-10-[2,3,5,6-tetrafluoro-4-(trifluoromethyl)phenyl]anthracene;
Figure 563 illustrates a 9-(2,6-difluoro-4-methoxyphenyl)-1,2,3,4,5-pentafluoro-10-(2,4-difluorophenyl)anthracene;
Figure 564 illustrates a 9-(3,4-difluorophenyl)-1,2,3,4,5-pentafluoro-10-phenyl anthracene;
Figure 565 illustrates a 9-(3,4-difluorophenyl)-1,2,3,4,5-pentafluoro-10-(4-methoxyphenyl)anthracene;
Figure 566 illustrates a 9-(3,4-difluorophenyl)-1,2,3,4,5-pentafluoro-10-[2,3,5,6-tetrafluoro-4-(trifluoromethyl)phenyl]anthracene;

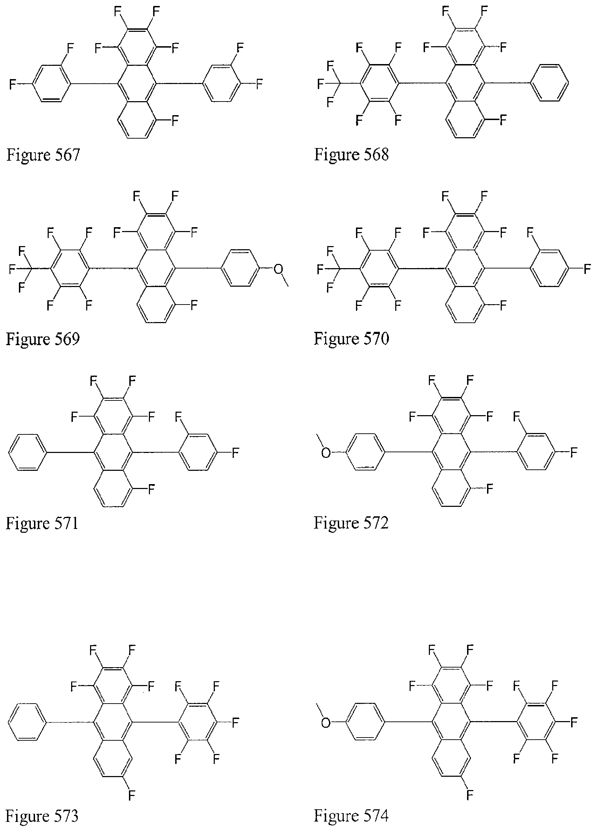

Figure 567 illustrates a 9-(2,4-difluorophenyl)-1,2,3,4,5-pentafluoro-10-(3,4-difluorophenyl)anthracene;
Figure 568 illustrates a 1,2,3,4,5-pentafluoro-9-phenyl-10-[2,3,5,6-tetrafluoro-4-(trifluoromethyl)phenyl]anthracene;
Figure 569 illustrates a 1,2,3,4,5-pentafluoro-9-(4-methoxyphenyl)-10-[2,3,5,6-tetrafluoro-4-(trifluoromethyl)phenyl]anthracene;
Figure 570 illustrates a 9-(2,4-difluorophenyl)-1,2,3,4,5-pentafluoro-10-[2,3,5,6-tetrafluoro-4-(trifluoromethyl)phenyl]anthracene;
Figure 571 illustrates a 9-(2,4-difluorophenyl)-1,2,3,4,5-pentafluoro-10-phenyl anthracene;
Figure 572 illustrates a 9-(2,4-difluorophenyl)-1,2,3,4,5-pentafluoro-10-(4-methoxyphenyl)anthracene;
Figure 573 illustrates a 1,2,3,4,6-pentafluoro-9-(2,3,4,5,6-pentafluorophenyl)-10-phenylanthracene;
Figure 574 illustrates a 1,2,3,4,6-pentafluoro-9-(4-methoxyphenyl)-10-(2,3,4,5,6-pentafluorophenyl)anthracene;

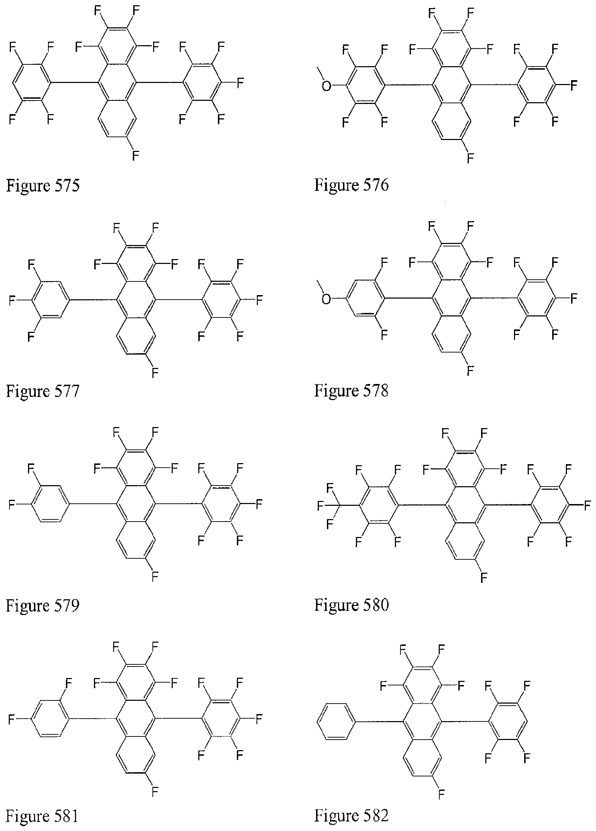

Figure 575 illustrates a 1,2,3,4,6-pentafluoro-9-(2,3,4,5,6-pentafluorophenyl)-10-(2,3,5,6-tetrafluoropheny)lanthracene;
Figure 576 illustrates a 1,2,3,4,6-pentafluoro-9-(2,3,4,5,6-pentafluorophenyl)-10-(2,3,5,6-tetrafluoro-4-methoxyphenyl)anthracene;
Figure 577 illustrates a 1,2,3,4,6-pentafluoro-9-(2,3,4,5,6-pentafluorophenyl)-10-(3,4,5-trifluoro)anthracene;
Figure 578 illustrates a 9-(2,6-difluoro-4-methoxyphenyl)-1,2,3,4,6-pentafluoro-10-(2,3,4,5,6-pentafluorophenyl)anthracene
Figure 579 illustrates a 9-(3,4-difluorophenyl)-1,2,3,4,6-pentafluoro-10-(2,3,4,5,6-pentafluorophenyl)anthracene;
Figure 580 illustrates a 1,2,3,4,6-pentafluoro-9-(2,3,4,5,6-pentafluorophenyl)-10-[2,3,5,6-tetrafluoro-4-(trifluoromethyl)phenyl]anthracene;
Figure 581 illustrates a 9-(2,4-difluorophenyl)-1,2,3,4,6-pentafluoro-10-(2,3,4,5,6-pentafluorophenyl)anthracene;
Figure 582 illustrates a 1,2,3,4,6-pentafluoro-9-phenyl-10-(2,3,5,6-tetrafluorophenyl)anthracene;

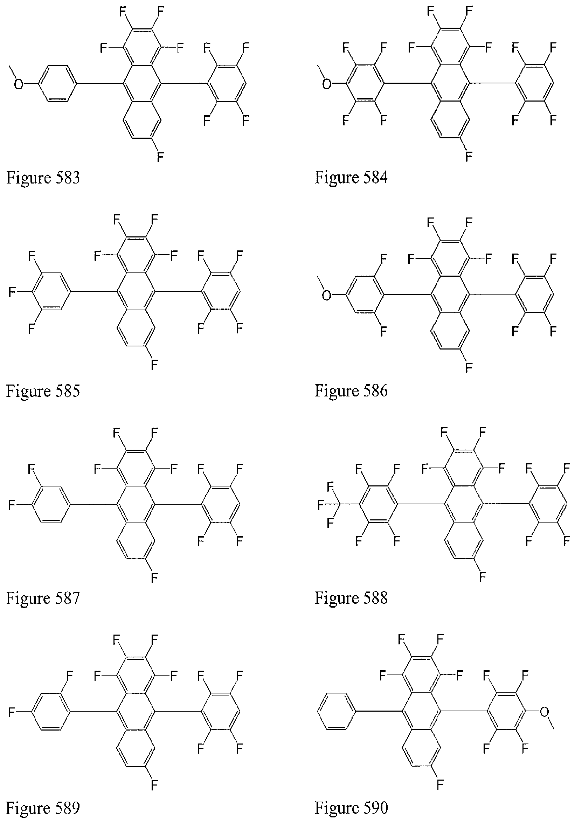

Figure 583 illustrates a 1,2,3,4,6-pentafluoro-9-(4-methoxyphenyl)-10-(2,3,5,6-tetrafluorophenyl)anthracene;
Figure 584 illustrates a 1,2,3,4,6-pentafluoro-9-(2,3,5,6-tetrafluorophenyl)-10-(2,3,5,6-tetrafluoro-4-methoxyphenyl)anthracene;
Figure 585 illustrates a 1,2,3,4,6-pentafluoro-9-(2,3,5,6-tetrafluorophenyl)-10-(3,4,5-trifluorophenyl)anthracene;
Figure 586 illustrates a 9-(2,6-difluoro-4-methoxyphenyl)-1,2,3,4,6-pentafluoro-10-(2,3,5,6-tetrafluorophenyl)anthracene;
Figure 587 illustrates a 9-(3,4-difluorophenyl)-1,2,3,4,6-pentafluoro-10-(2,3,5,6-tetrafluorophenyl)anthracene;
Figure 588 illustrates a 1,2,3,4,6-pentafluoro-9-[2,3,5,6-tetrafluoro-4-(trifluoromethyl)phenyl]-10-(2,3,,5,6-tetrafluorophenyl)anthracene;
Figure 589 illustrates a 9-(3,4-difluorophenyl)-1,2,3,4,6-pentafluoro-10-(2,3,5,6-tetrafluorophenyl)anthracene;
Figure 590 illustrates a 1,2,3,4,6-pentafluoro-9-phenyl-10-(2,3,5,6-tetrafluoro-4-methoxyphenyl)anthracene;

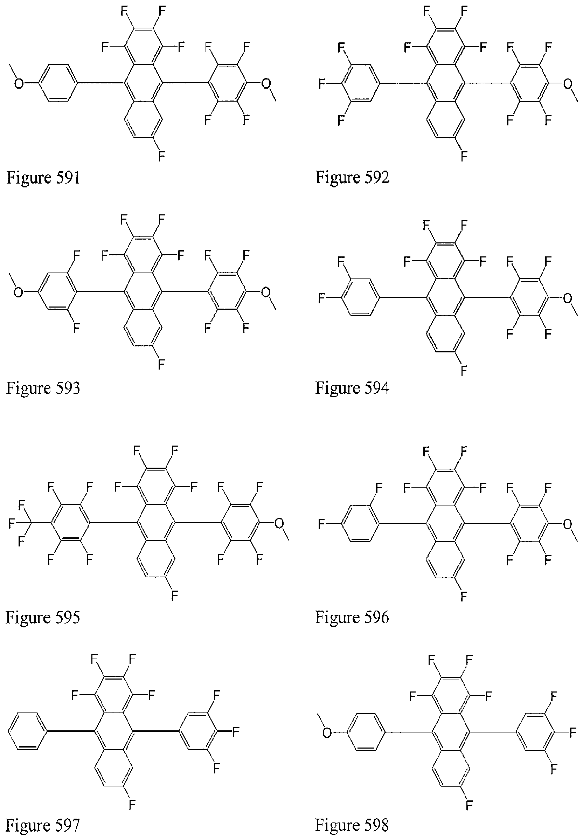

Figure 591 illustrates a 1,2,3,4,6-pentafluoro-9-(4-methoxyphenyl)-10-(2,3,5,6-tetrafluoro-4-methoxyphenyl)anthracene;
Figure 592 illustrates a 1,2,3,4,6-pentafluoro-9-(2,3,5,6-tetrafluoro-4-methoxyphenyl)-10-(3,4,5-trifluorophenyl)anthracene;
Figure 593 illustrates a 9-(2,6-difluoro-4-methoxyphenyl)-1,2,3,4,6-pentafluoro-10-(2,3,5,6-tetrafluoro-4-methoxyphenyl)anthracene;
Figure 594 illustrates a 9-(3,4-difluorophenyl)-1,2,3,4,6-pentafluoro-10-(2,3,5,6-tetrafluoro-4-methoxyphenyl)anthracene;
Figure 595 illustrates a 1,2,3,4,6-pentafluoro-9-[2,3,5,6-tetrafluoro-4-(trifluoromethyl)phenyl]-10-(2,3,5,6-tetrafluoro-4-methoxyphenyl)anthracene;
Figure 596 illustrates a 9-(2,4-difluorophenyl)-1,2,3,4,6-pentafluoro-10-(2,3,5,6-tetrafluoro-4-methoxyphenyl)anthracene;
Figure 597 illustrates a 1,2,3,4,6-pentafluoro-9-phenyl-10-(3,4,5-trifluorophenyl)anthracene;
Figure 598 illustrates a 1,2,3,4,6-pentafluoro-9-(4-methoxyphenyl)-10-(3,4,5-trifluorophenyl)anthracene;

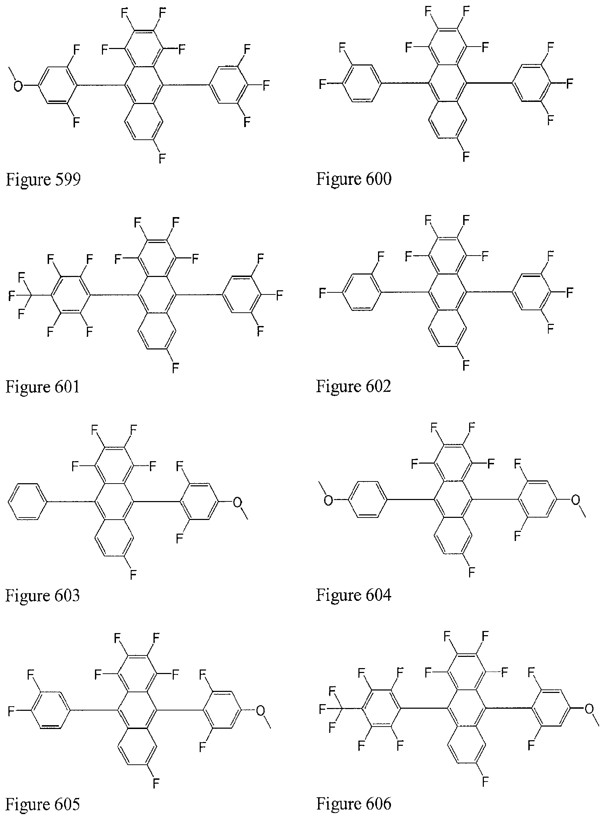

Figure 599 illustrates a 9-(2,6-difluoro-4-methoxyphenyl)-1,2,3,4,6-pentafluoro-10-(3,4,5-trifluorophenyl)anthracene;
Figure 600 illustrates a 9-(3,4-difluorophenyl)-1,2,3,4,6-pentafluoro-10-(3,4,5-trifluorophenyl)anthracene;
Figure 601 illustrates a 1,2,3,4,6-pentafluoro-9-[2,3,5,6-tetrafluoro-4-(trifluoromethyl)phenyl]-10-(3,4,5-trifluorophenyl)anthracene;
Figure 602 illustrates a 9-(2,4-difluorophenyl)-1,2,3,4,6-pentafluoro-10-(3,4,5-trifluorophenyl)anthracene;
Figure 603 illustrates a 9-(2,6-difluoro-4-methoxyphenyl)-1,2,3,4,6-pentafluoro-10-phenylanthracene:
Figure 604 illustrates a 9-(2,6-difluoro-4-methoxyphenyl)-1,2,3,4,6-pentafluoro-10-(4-methoxyphenyl)anthracene:
Figure 605 illustrates a 9-(2,6-difluoro-4-methoxyphenyl)-10-(3,4-difluorophenyl)-1,2,3,4,6-pentafluoroanthracene;
Figure 606 illustrates a 9-(2,6-difluoro-4-methoxyphenyl)-1,2,3,4,6-pentafluoro-10-[2,3,5,6-tetrafluoro-4-(trifluoromethyl)phenyl]anthracene;

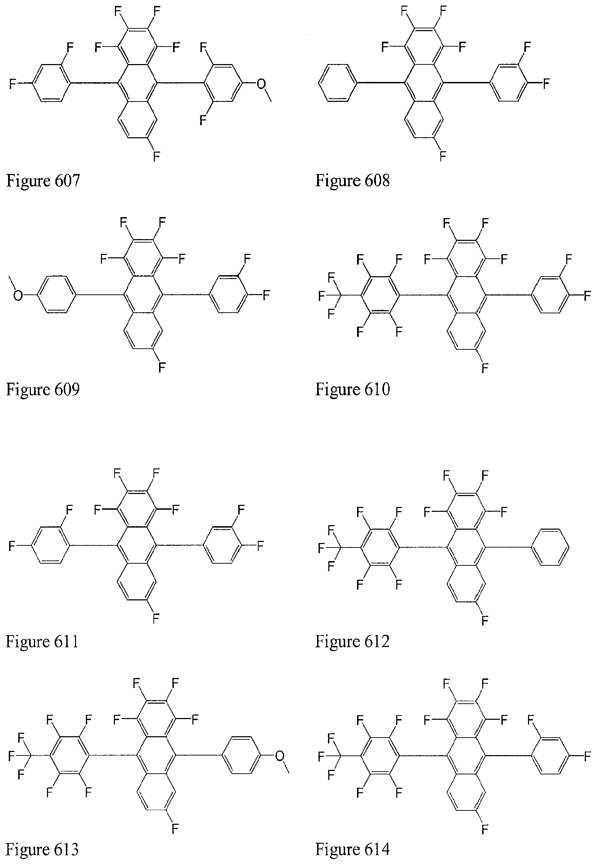

Figure 607 illustrates a 9-(2,6-difluoro-4-methoxyphenyl)-1,2,3,4,6-pentafluoro-10-(2,4-difluorophenyl)anthracene;
Figure 608 illustrates a 9-(3,4-difluorophenyl)-1,2,3,4,6-pentafluoro-10-phenylanthracene;
Figure 609 illustrates a 9-(3,4-difluorophenyl)-1,2,3,4,6-pentafluoro-10-(4-methoxyphenyl)anthracene;
Figure 610 illustrates a 9-(3,4-difluorophenyl)-1,2,3,4,6-pentafluoro-10-[2,3,5,6-tetrafluoro-4-(trifluoromethyl)phenyl]anthracene;
Figure 611 illustrates a 9-(2,4-difluorophenyl)-1,2,3,4,6-pentafluoro-10-(3,4-difluorophenyl)anthracene;
Figure 612 illustrates a 1,2,3,4,6-pentafluoro-9-phenyl-10-[2,3,5,6-tetrafluoro-4-(trifluoromethyl)phenyl]anthracene;
Figure 613 illustrates a 1,2,3,4,6-pentafluoro-9-(4-methoxyphenyl)-10-[2,3,5,6-tetrafluoro-4-(trifluoromethyl)phenyl]anthracene;
Figure 614 illustrates a 9-(2,4-difluorophenyl)-1,2,3,4,6-pentafluoro-10-[2,3,5,6-tetrafluoro-4-(trifluoromethyl)phenyl]anthracene;

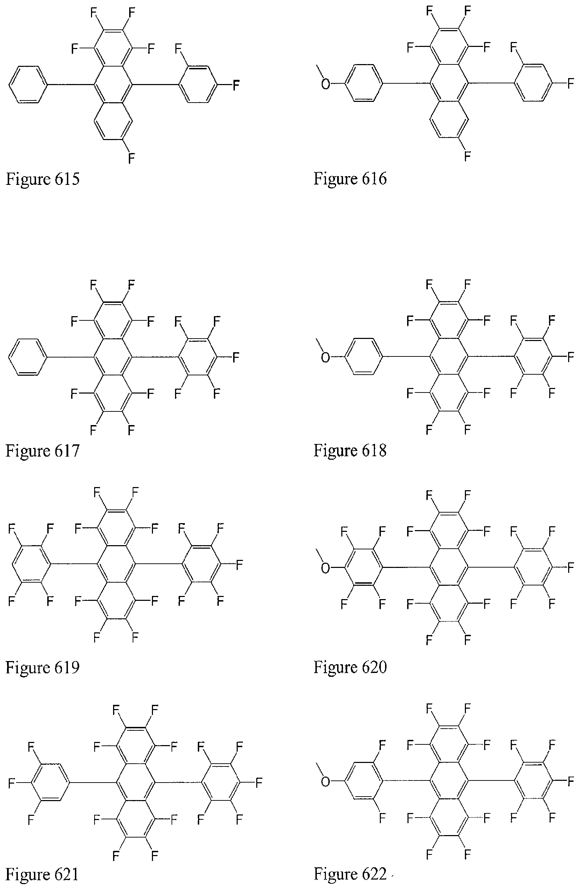

Figure 615 illustrates a 9-(2,4-difluorophenyl)-1,2,3,4,6-pentafluoro-10-phenylanthracene;
Figure 616 illustrates a 9-(2,4-difluorophenyl)-1,2,3,4,6-pentafluoro-10-(4-methoxyphenyl)anthracene;
Figure 617 illustrates a 1,2,3,4,5,6,7,8-octafluoro-9-(2,3,4,5,6-pentafluorophenyl)-10-phenylanthracene;
Figure 618 illustrates a 1,2,3,4,5,6,7,8-octafluoro-9-(4-methoxyphenyl)-10-(2,3,4,5,6-pentafluorophenyl)anthracene;
Figure 619 illustrates a 1,2,3,4,5,6,7,8-octafluoro-9-(2,3,4,5,6-pentafluorophenyl)-10-(2,3,5,6-tetrafluoropheny)lanthracene;
Figure 620 illustrates a 1,2,3,4,5,6,7,8-octafluoro-9-(2,3,4,5,6-pentafluorophenyl)-10-(2,3,5,6-tetrafluoro-4-methoxyphenyl)anthracene;
Figure 621 illustrates a 1,2,3,4,5,6,7,8-octafluoro-9-(2,3,4,5,6-pentafluorophenyl)-10-(3,4,5-trifluoro)anthracene;
Figure 622 illustrates a 9-(2,6-difluoro-4-methoxyphenyl)-1,2,3,4,5,6,7,8-octafluoro-10-(2,3,4,5,6-pentafluorophenyl)anthracene

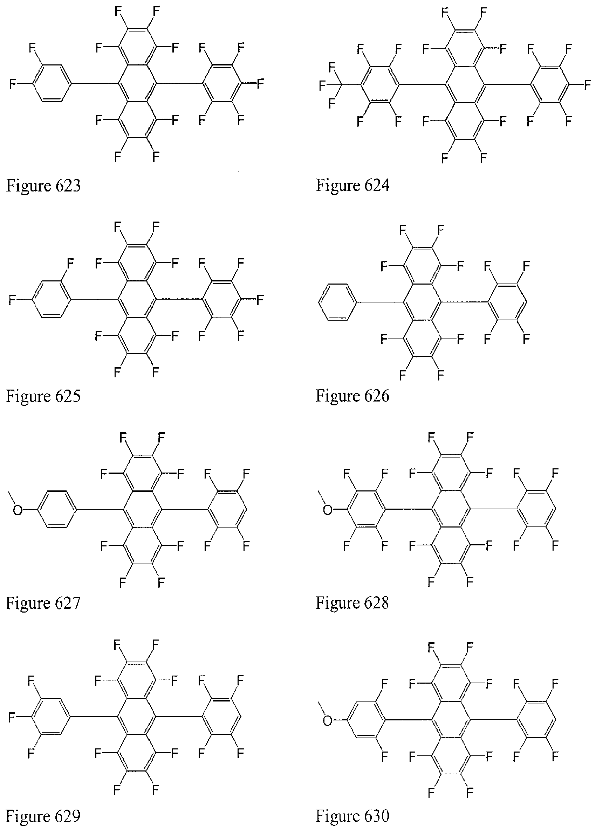

Figure 623 illustrates a 9-(3,4-difluorophenyl)-1,2,3,4,5,6,7,8-octafluoro-10-(2,3,4,5,6-pentafluorophenyl)anthracene;
Figure 624 illustrates a 1,2,3,4,5,6,7,8-octafluoro-9-(2,3,4,5,6-pentafluorophenyl)-10-[2,3,5,6-tetrafluoro-4-(trifluoromethyl)phenyl]anthracene;
Figure 625 illustrates a 9-(2,4-difluorophenyl)-1,2,3,4,5,6,7,8-octafluoro-10-(2,3,4,5,6-pentafluorophenyl)anthracene;
Figure 626 illustrates a 1,2,3,4,5,6,7,8-octafluoro-9-phenyl-10-(2,3,5,6-tetrafluorophenyl)anthracene;
Figure 627 illustrates a 1,2,3,4,5,6,7,8-octafluoro-9-(4-methoxyphenyl)-10-(2,3,5,6-tetrafluorophenyl)anthracene;
Figure 628 illustrates a 1,2,3,4,5,6,7,8-octafluoro-9-(2,3,5,6-tetrafluorophenyl)-10-(2,3,5,6-tetrafluoro-4-methoxyphenyl)anthracene;
Figure 629 illustrates a 1,2,3,4,5,6,7,8-octafluoro-9-(2,3,5,6-tetrafluorophenyl)-10-(3,4,5-trifluorophenyl)anthracene;
Figure 630 illustrates a 9-(2,6-difluoro-4-methoxyphenyl)-1,2,3,4,5,6,7,8-octafluoro-10-(2,3,5,6-tetrafluorophenyl)anthracene;

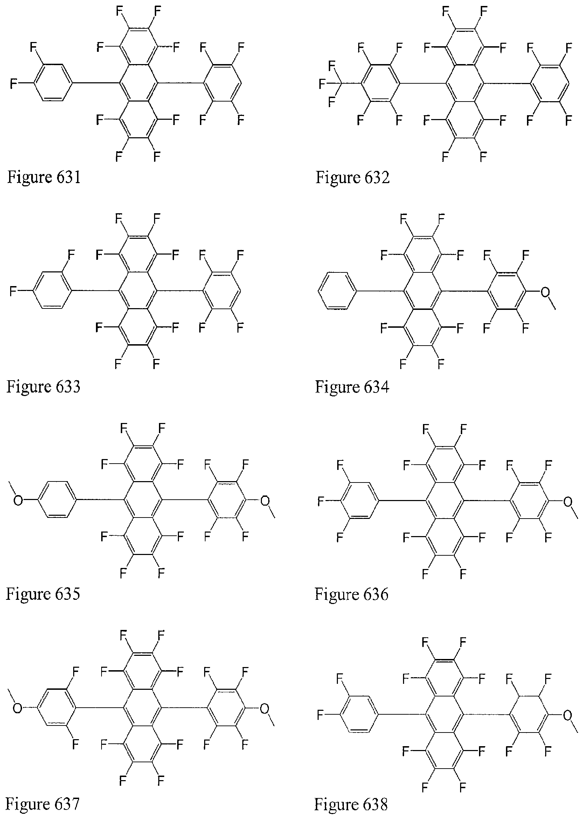

Figure 631 illustrates a 9-(3,4-difluorophenyl)-1,2,3,4,5,6,7,8-octafluoro-10-(2,3,5,6-tetrafluorophenyl)anthracene;
Figure 632 illustrates a 1,2,3,4,5,6,7,8-octafluoro-9-[2,3,5,6-tetrafluoro-4-(trifluoromethyl)phenyl]-10-(2,3,,5,6-tetrafluorophenyl)anthracene;
Figure 633 illustrates a 9-(3,4-difluorophenyl)-1,2,3,4,5,6,7,8-octafluoro-10-(2,3,5,6-tetrafluorophenyl)anthracene;
Figure 634 illustrates a 1,2,3,4,5,6,7,8-octafluoro-9-phenyl-10-(2,3,5,6-tetrafluoro-4-methoxyphenyl)anthracene;
Figure 635 illustrates a 1,2,3,4,5,6,7,8-octafluoro-9-(4-methoxyphenyl)-10-(2,3,5,6-tetrafluoro-4-methoxyphenyl)anthracene;
Figure 636 illustrates a 1,2,3,4,5,6,7,8-octafluoro-9-(2,3,5,6-tetrafluoro-4-methoxyphenyl)-10-(3,4,5-trifluorophenyl)anthracene;
Figure 637 illustrates a 9-(2,6-difluoro-4-methoxyphenyl)-1,2,3,4,5,6,7,8-octafluoro-10-(2,3,5,6-tetrafluoro-4-methoxyphenyl)anthracene;
Figure 638 illustrates a 9-(3,4-difluorophenyl)-1,2,3,4,5,6,7,8-octafluoro-10-(2,3,5,6-tetrafluoro-4-methoxyphenyl)anthracene;

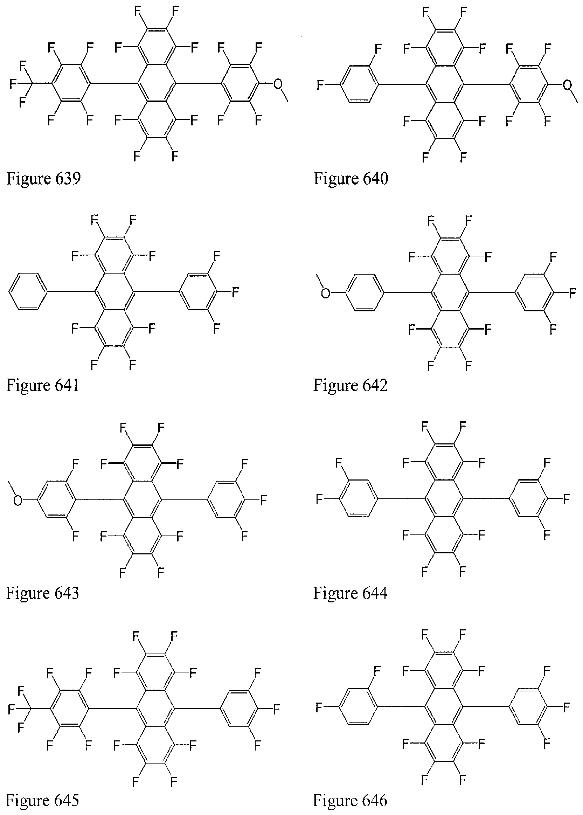

Figure 639 illustrates a 1,2,3,4,5,6,7,8-octafluoro-9-[2,3,5,6-tetrafluoro-4-(trifluoromethyl)phenyl]-10-(2,3,5,6-tetrafluoro-4-methoxyphenyl)anthracene;
Figure 640 illustrates a 9-(2,4-difluorophenyl)-1,2,3,4,5,6,7,8-octafluoro-10-(2,3,5,6-tetrafluoro-4-methoxyphenyl)anthracene;
Figure 641 illustrates a 1,2,3,4,5,6,7,8-octafluoro-9-phenyl-10-(3,4,5-trifluorophenyl)anthracene;
Figure 642 illustrates a 1,2,3,4,5,6,7,8-octafluoro-9-(4-methoxyphenyl)-10-(3,4,5-trifluorophenyl)anthracene;
Figure 643 illustrates a 9-(2,6-difluoro-4-methoxyphenyl)-1,2,3,4,5,6,7,8-octafluoro-10-(3,4,5-trifluorophenyl)anthracene;
Figure 644 illustrates a 9-(3,4-difluorophenyl)-1,2,3,4,5,6,7,8-octafluoro-10-(3,4,5-trifluorophenyl)anthracene;
Figure 645 illustrates a 1,2,3,4,5,6,7,8-octafluoro-9-[2,3,5,6-tetrafluoro-4-(trifluoromethyl)phenyl]-10-(3,4,5-trifluorophenyl)anthracene;
Figure 646 illustrates a 9-(2,4-difluorophenyl)-1,2,3,4,5,6,7,8-octafluoro-10-(3,4,5-trifluorophenyl)anthracene;

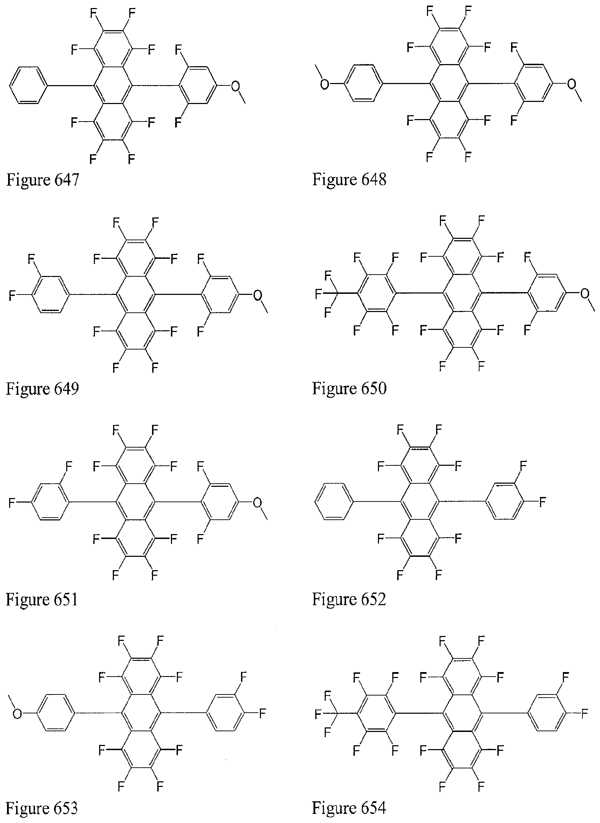

Figure 647 illustrates a 9-(2,6-difluoro-4-methoxyphenyl)-1,2,3,4,5,6,7,8-octafluoro-10-phenylanthracene:
Figure 648 illustrates a 9-(2,6-difluoro-4-methoxyphenyl)-1,2,3,4,5,6,7,8-octafluoro-10-(4-methoxyphenyl)anthracene:
Figure 649 illustrates a 9-(2,6-difluoro-4-methoxyphenyl)-10-(3,4-difluorophenyl)-1,2,3,4,5,6,7,8-octafluoroanthracene;
Figure 650 illustrates a 9-(2,6-difluoro-4-methoxyphenyl)-1,2,3,4,5,6,7,8-octafluoro-10-[2,3,5,6-tetrafluoro-4-(trifluoromethyl)phenyl]anthracene;
Figure 651 illustrates a 9-(2,6-difluoro-4-methoxyphenyl)-1,2,3,4,5,6,7,8-octafluoro-10-(2,4-difluorophenyl)anthracene;
Figure 652 illustrates a 9-(3,4-difluorophenyl)-1,2,3,4,5,6,7,8-octafluoro-10-phenylanthracene;
Figure 653 illustrates a 9-(3,4-difluorophenyl)-1,2,3,4,5,6,7,8-octafluoro-10-(4-methoxyphenyl)anthracene;
Figure 654 illustrates a 9-(3,4-difluorophenyl)-1,2,3,4,5,6,7,8-octafluoro-10-[2,3,5,6-tetrafluoro-4-(trifluoromethyl)phenyl]anthracene;

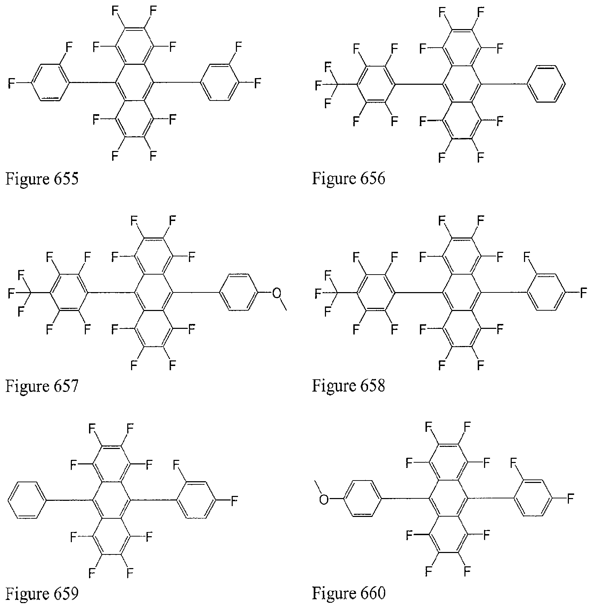

Figure 655 illustrates a 9-(2,4-difluorophenyl)-1,2,3,4,5,6,7,8-octafluoro-10-(3,4-difluorophenyl)anthracene;
Figure 656 illustrates a 1,2,3,4,5,6,7,8-octafluoro-9-phenyl-10-[2,3,5,6-tetrafluoro-4-(trifluoromethyl)phenyl]anthracene;
Figure 657 illustrates a 1,2,3,4,5,6,7,8-octafluoro-9-(4-methoxyphenyl)-10-[2,3,5,6-tetrafluoro-4-(trifluoromethyl)phenyl]anthracene;
Figure 658 illustrates a 9-(2,4-difluorophenyl)-1,2,3,4,5,6,7,8-octafluoro-10-[2,3,5,6-tetrafluoro-4-(trifluoromethyl)phenyl]anthracene;
Figure 659 illustrates a 9-(2,4-difluorophenyl)-1,2,3,4,5,6,7,8-octafluoro-10-phenylanthracene;
Figure 660 illustrates a 9-(2,4-difluorophenyl)-1,2,3,4,5,6,7,8-octafluoro-10-(4-methoxyphenyl)anthracene;
Figure 661 is a graph which illustrates chemiluminescent systems containing several of the instantly disclosed anthracene fluorescers, which are shown to produce light having an emission maximum from about 390 nm to less than 438 nm.

### DETAILED DESCRIPTION OF THE INVENTION

The present invention can be achieved by providing a composition comprising an oxalate solution and an anthracene compound represented by Formula (1): 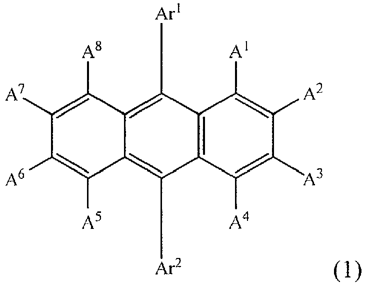 wherein Ar¹ has a structure represented by Formula (2), where An shows the attachment to the anthracene ring as described in Formula (1),
Ar¹ = 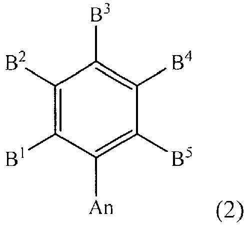 wherein Ar² has a structure represented by Formula (3), where An shows the attachment to the anthracene ring, as described in Formula (1),
Ar² = 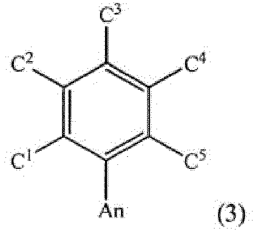 wherein each of A¹, A², A³, A⁴, A⁵, A⁶, A⁷, and A⁸ are selected from the group consisting of hydrogen and fluorine;
wherein at least two of the substituents, B¹, B², B³, B⁴, and B⁵, as shown in Formula 2, are not hydrogen, wherein each of B¹, B², B⁴, and B⁵ are selected from the group consisting of hydrogen and fluorine, and B³ is selected from the group consisting of hydrogen, fluorine, trifluoromethyl, and an alkoxy group with 1-10 carbons; and
wherein at least two of the substituents, C¹, C², C³, C⁴, and C⁵, as shown in Formula 3, are not hydrogen, wherein each of C¹, C², C⁴, and C⁵ are selected from the group consisting of hydrogen and fluorine, and C³ is selected from the group consisting of hydrogen, fluorine, trifluoromethyl, and an alkoxy group with 1-10 carbons,
wherein the composition is for reacting with a hydrogen peroxide solution to provide a chemiluminescent system that produces a blue/violet chemiluminescent light in the region where the emission maximum is from 390 nm to less than 438 nm.

Ar¹ and Ar² can be the same or different from each other.
The new species of anthracenes according to the invention may be prepared by a method, according to one of the Reaction Schemes given below.

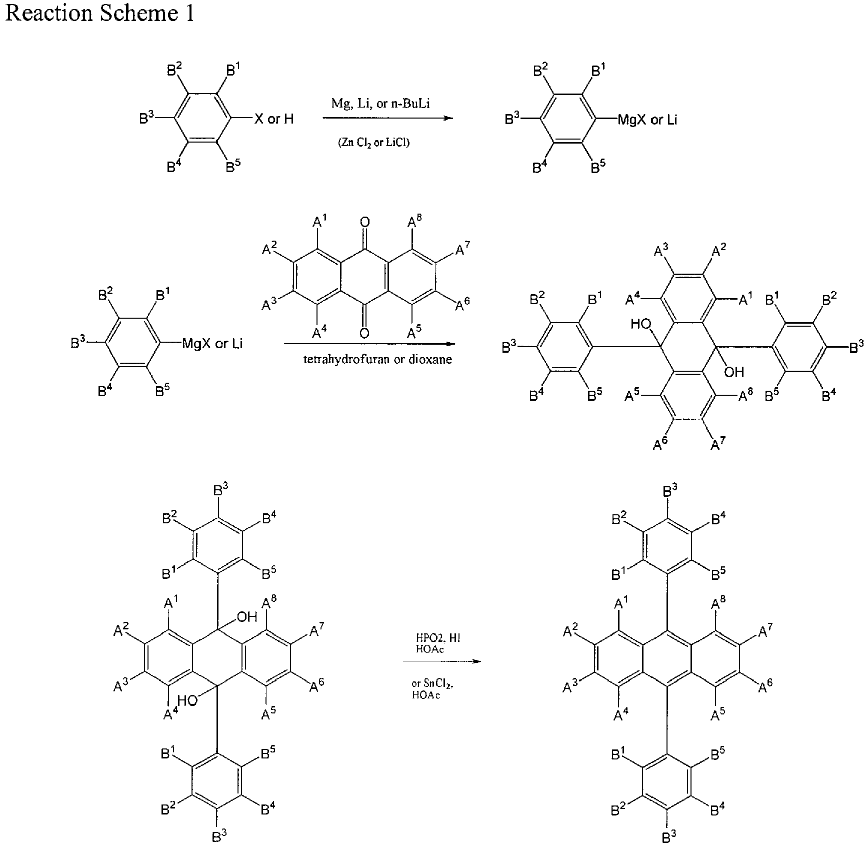

One process disclosed , as shown in Reaction Scheme 1, to obtain derivatives in Formula (1) where Ar¹ and Ar² are the same may be described as a synthetic process comprising steps of:
a) generation of a fluorinated aryl organometallic metallic reagent by the reaction of an fluorinated aryl halide with magnesium or n-butyllithium or by the substitution of an aryl hydrogen using n-butyllithium;
b) reaction of the organometallic reagent [RMgX or RLi] with an anthraquinone derivative to form a diol, wherein R is represented by Formula 2 and X may be a halogen. It is further contemplated that this reaction may be carried out in the presence of lithium chloride or zinc chloride;
c) reduction of said diol to form a DPHA derivative using either a combination of sodium hypophosphite and potassium iodide in acetic acid (U.S. 5,232,635 to Van Moer et al) or a solution of stannous chloride in a solution of stannous chloride in glacial acetic acid (U.S.6,740,263 to Park et al).
wherein, A¹, A², A³, A⁴, A⁵, A⁶, A⁷, and A⁸ represents one selected from the group consisting of hydrogen or fluorine, and B¹, B², B⁴, and B⁵ represents one selected from the group consisting of hydrogen and fluorine, where at least two of these are fluorine, and B³ represents one selected from a group consisting of hydrogen, fluorine, trifluoromethyl, and an alkoxy group with 1-10 carbons.

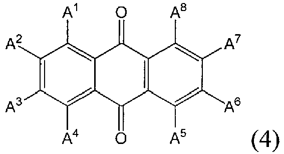

Fluorinated anthraquinones represented by Formula (4) may be purchased from commercial sources or made as described by Tannaci et al. (J. Org. Chem. 2007, 72, 5567-5573), Mallory et al. (J. Am. Chem. Soc. 1990, 112, 2577-2581), and Moer et al. (U.S. Pat. No. 5,232,635).

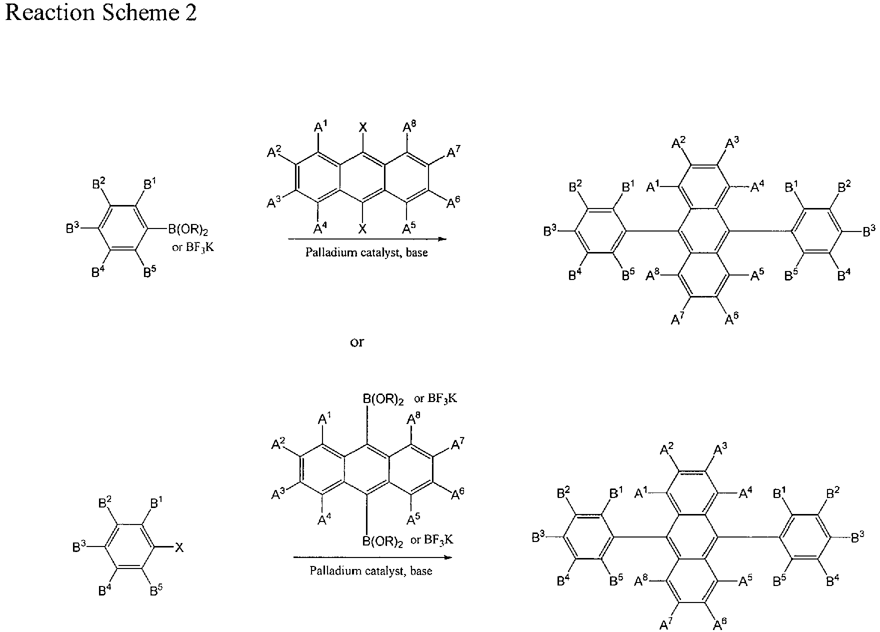

Alternatively, symmetrical derivatives may be made via the Suzuki-Miyaura reaction or variation thereof using either a 9,10-dichloroanthracene, 9,10-dibromoanthracene, or 9,10-diiodoanthracene derivative and an aryl boronic acid, boronic ester, or trifluoroborate. Additionally, a 9,10-anthracenediboronic acid, equivalent ester, or trifluoroborate can be reacted with an aryl halide or triflate. Both pathways are as shown in Reaction Scheme 2 comprising the reaction of:
a) a boronic acid, dialkylboronate ester, or trifluoroborate indicated by B(OR)₂, or BF₃K with the preferred examples being the boronic acid, the dimethyl ester of the boronic acid, or the picolinate ester of boronic acid, an aryl species, where X is either bromine, iodine, or a triflate, with the preferred example being bromine, a palladium catalyst from a large family of commercially available compounds, with the preferred examples being palladium(II) acetate, tetrakis(triphenylphosphine)palladium(0), and dichlorobis(chlorodi-*tert-*butylphosphine)palladium(II), a suitable base, with the preferred examples being potassium phosphate, sodium carbonate, potassium carbonate, and potassium fluoride, a suitable solvent, with the preferred examples being toluene, tetrahydrofuran, isopropanol, and 1,2-dimethoxyethane.

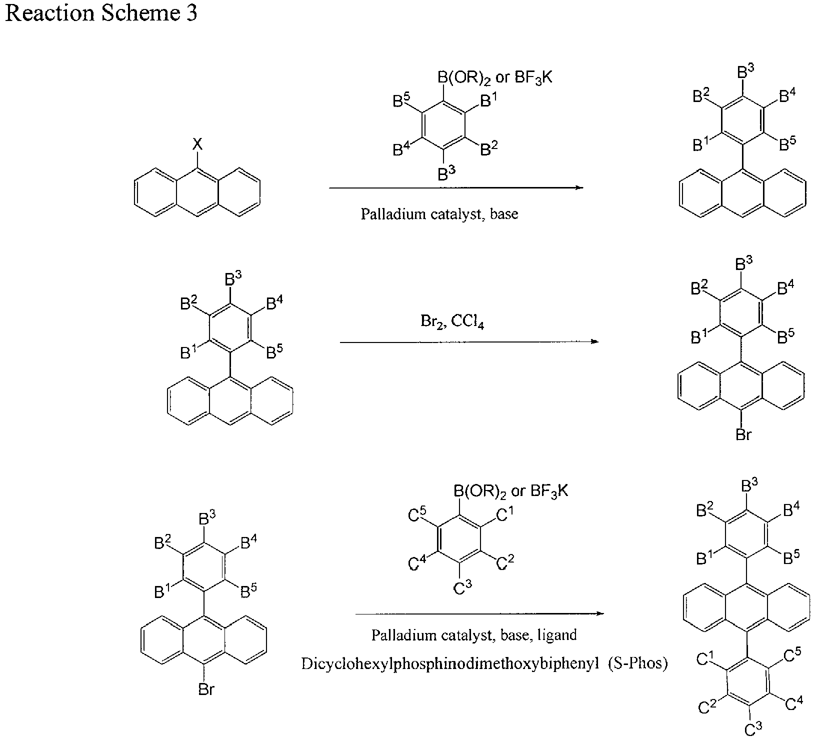

Another process disclosed , as shown is Reaction Scheme 3, to produce asymmetrical anthracene derivatives, where Ar¹ and Ar² are not the same, may be described as a synthetic process comprising steps of:
a) a coupling reaction between: i) a 9-substituted anthracene, where X is bromine, iodine, or triflate; ii) a fluorinated arylboronic acid, arylboronic ester, or aryltrifluoroborate, wherein the other substituents are as defined in Figure 2; iii) a palladium catalyst from a large family of commercially available compounds, with the preferred examples being palladium(II) acetate, tetrakis(triphenylphosphine)palladium(0), and dichlorobis(chlorodi-*tert*-butylphosphine)palladium(II); iv) a suitable base, with the preferred examples being potassium phosphate, sodium carbonate, and potassium fluoride, and a suitable solvent, with the preferred examples being toluene, tetrahydrofuran, isopropanol, and 1,2-dimethoxyethane; v) optionally including a phosphorous ligand;
b) halogenation of the 10 position of the anthracene using, for example, bromine in tetrachloromethane as illustrated; and
c) a second coupling reaction similar to the one described in part a), wherein the other substituents are as defined in Figure 3 with the addition of a phosphorous ligand, with the preferred examples being 2-cyclohexylphosphino-2'6'-dimethoxy-1,1'-biphenyl (S-Phos) or tris(o-tolyl)phosphine.

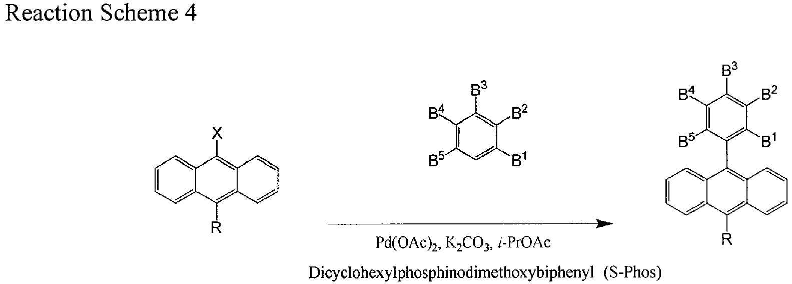

Alternatively, the asymmetric derivatives of the present invention can be made by via a synthetic coupling reaction as shown in Reaction Scheme 4 containing:
a) either a 9-haloanthracene or a 9-halo-10-arylanthracene, where the halogen is bromine or chlorine, with the preferred example being bromine and the aryl group R is described by Figure 3; b) an aryl ring with at least 3 to 4 fluorines, at least one hydrogen, where the remaining substituent can be hydrogen, fluorine, an alkoxy group, or a trifluoromethyl group; c) palladium acetate; d) 2-cyclohexylphosphino-2'6'-dimethoxy-1,1'-biphenyl (S-Phos); e) a suitable acetate solvent, with the preferred example being isopropyl acetate.

The production of a chemiluminescent system exhibiting a blue/violet emission spectrum with a maximum in the range of about 390 nm to less than-438 nm according to the present invention is accomplished by the combination of two solutions, A and B:
wherein solution A comprises:
   an oxalate selected from a family of substituted carbalkoxyphenyloxalates at a concentration of 10 to 30 percent by weight, with the preferred example being bis(2,4,5-trichloro-6-carbopentoxyphenyl)oxalate at a concentration of 14 to 23; percent,
   an anthracene of Formula 1 as described in this invention at a concentration of 0.025 to 0.5 percent by weight, with the preferred amount being 0.10 to 0.25 percent; and
   a suitable solvent from a family of phthalates, benzoates, and citrates, with the preferred solvent being butyl benzoate;
   wherein solution B comprises:
a solution of 70% aqueous hydrogen peroxide at a concentration of 2 to 6.5 percent of the total weight, with the preferred amount being 3 to 4 percent; and
a catalyst from a family of salicylates:
   wherein said catalyst is at a concentration of 0.0015 to 0.05 percent by weight with the preferred amount being 0.005 to 0.010 percent; and
   a solvent from a family of phthalates, benzoates, and citrates, with the preferred example being dimethyl phthalate, dibutyl phthalate, triethyl citrate, benzyl benzoate, butyl benzoate, propylene glycol dibenzoate, ethylhexyl diphenyl phosphate, ethylene glycol dibenzoate, and n-butyl,tri-n-hexyl citrate, or the like phthalates, benzoates and citrates.

Optionally, a solvent miscible in water and most organic solvents at a concentration of 5 to 20 percent by weight, with the preferred example being *tert*-butanol at a concentration of 5 to 15 percent may be included.

Compounds which are generally suitable to catalyze the reaction between the activator and oxalate type esters are basic compounds, such as amines, hydroxides, alkoxides, carboxylic acid salts and phenolic salts. Preferred salts of carboxylic acids and phenols are derived from compounds having a pKa in the range of from about 1 to about 6 as measured in aqueous solution, for example tetrabutylammonium salicylate , sodium salicylate, ethyl-2-acetoxy salicylate, various tetraalkylammonium salicylates, lithium salicylate, lithium 5-t-butyl salicylate sodium-5-bromosalicylate, sodium-5-chlorosalicylate, and sodium-5-fluorosalicylate or the like, with the preferred catalyst being sodium salicylate.

### EXAMPLES

Starting materials and solvents were purchased from Aldrich, Matrix, Synthonix, and Alfa Aesar. All of the desired compounds were analyzed via HPLC, and their spectra (absorption, chemiluminescent emission, and NMR) recorded.

### Example 1

### Procedure for producing 9,10-bis(2,3,4,5,6-pentafluorophenyl)anthracene (Figure 1)

To a suspension of 6.25 g of 9,10-anthraquinone in 60 mL of anhydrous 1,4-dioxane, was added 0.1 mole of pentafluorophenylmagnesium bromide in 30 mL of diethyl ether prepared as described in Organic Synthesis, Coll. Vol. 6, p. 875. The mixture was brought to a reflux and held there overnight. The reaction was quenched in a mixture of 25 mL of saturated aqueous ammonium chloride and 25 mL of water. After bringing the pH of the aqueous portion of the solution to pH 3-4 using dilute aqueous hydrochloric acid, 100 mL of ethyl acetate was added. The layers were separated, and the aqueous layer was extracted with an additional 100 mL of ethyl acetate. The combined organic extracts were dried with magnesium sulfate and concentrated. The residue was recrystallized from toluene to give 8.66 g of off-white crystals.

The reduction of the diol was accomplished by taking 5.44 g of 9,10-bis(pentafluorophenyl)-9,10-dihydro-9,10-anthracenediol in 50 mL of acetic acid and refluxing it with 9 g of potassium iodide and 9 g of sodium hypophosphite for 4 hours. After cooling, white crystals were filtered off. The crude product was recrystallized using a 1:1 mixture of toluene and hexane to yield 2.00 g of 9,10-bis(pentafluorophenyl)anthracene with a melting point of 337-338°C.

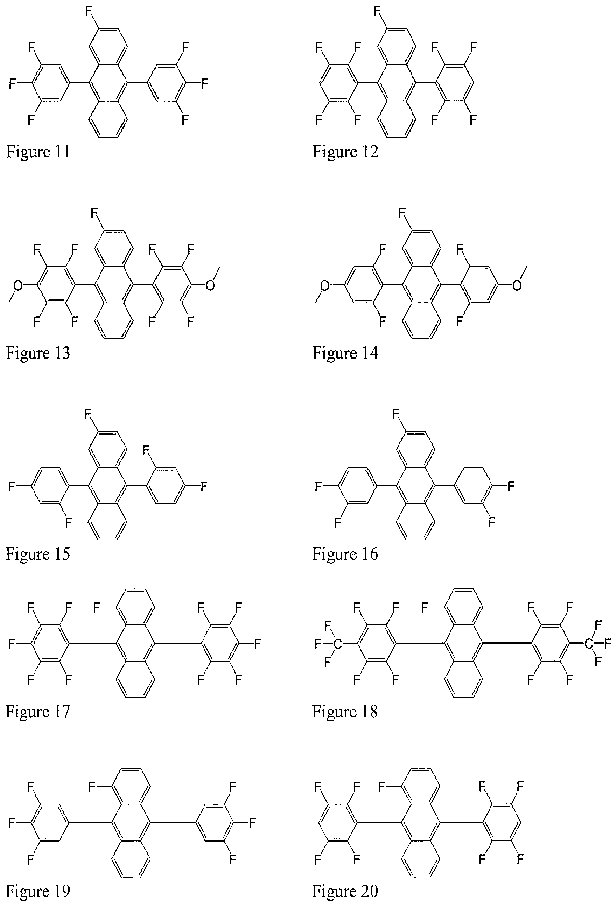

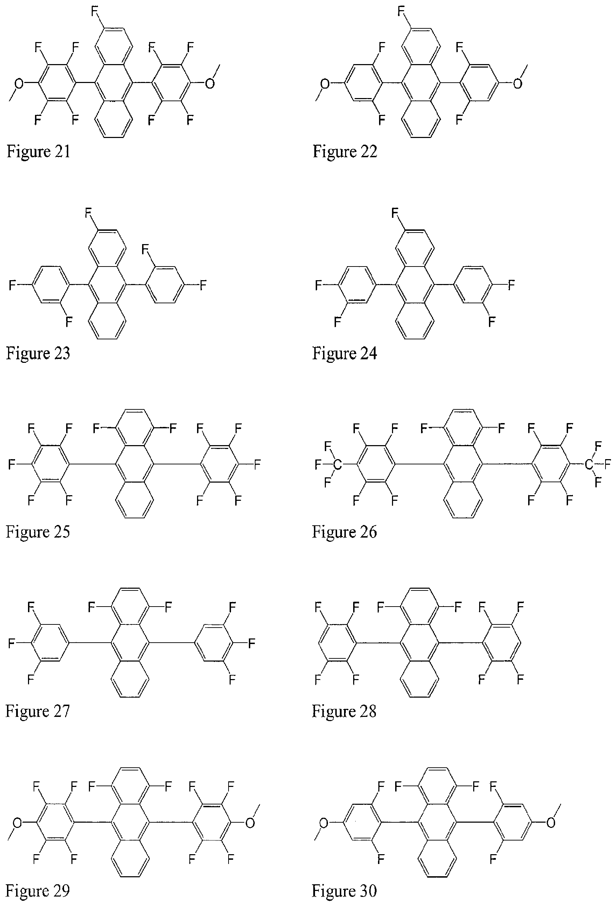

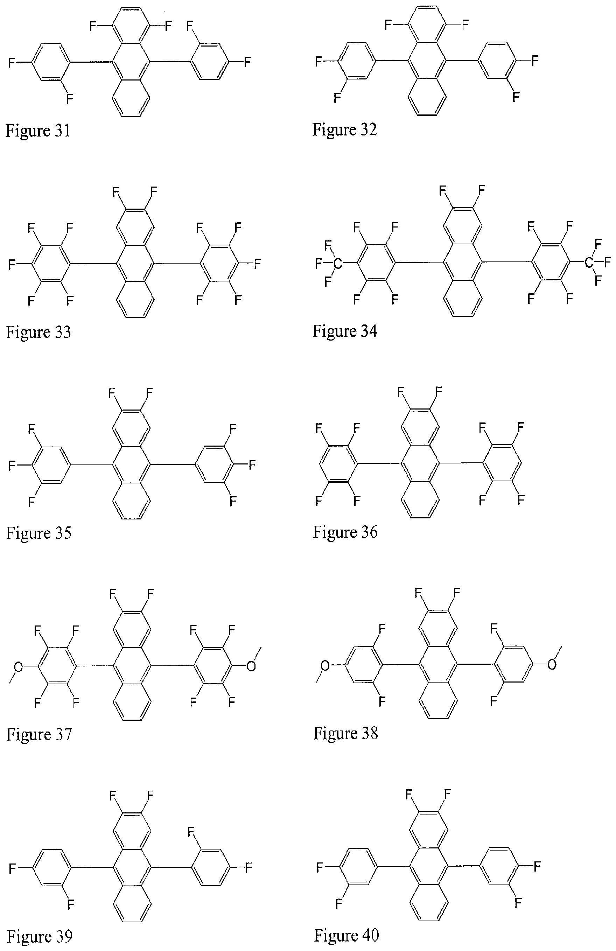

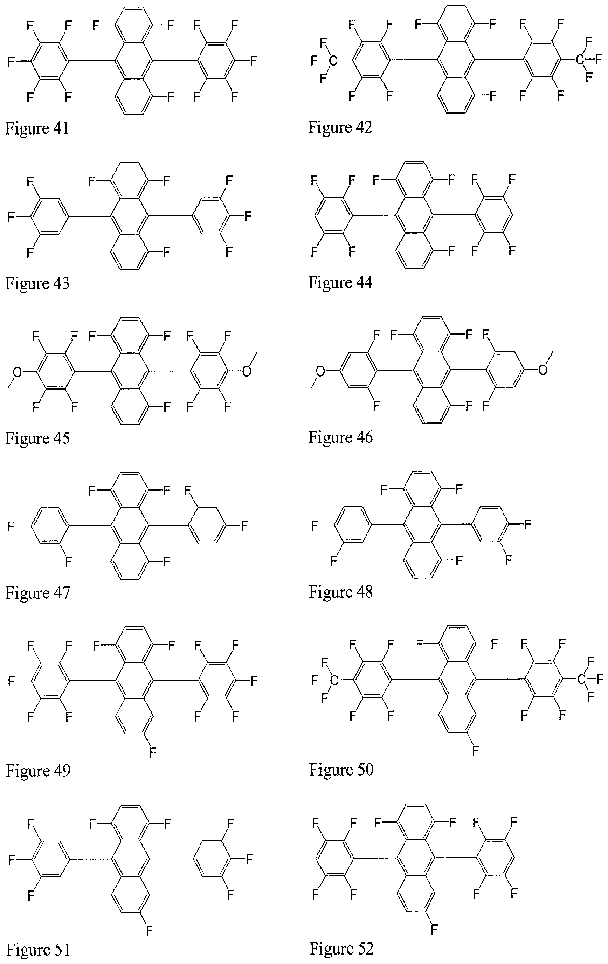

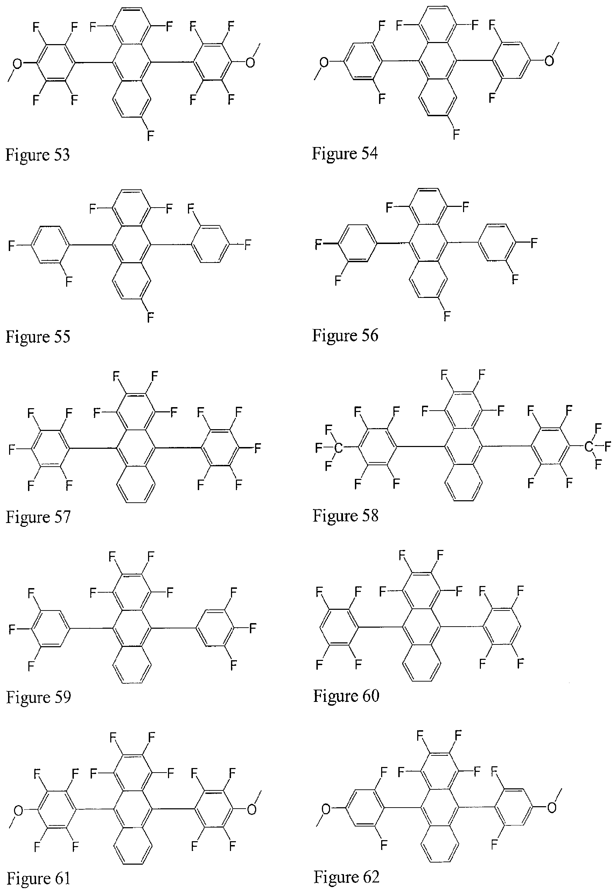

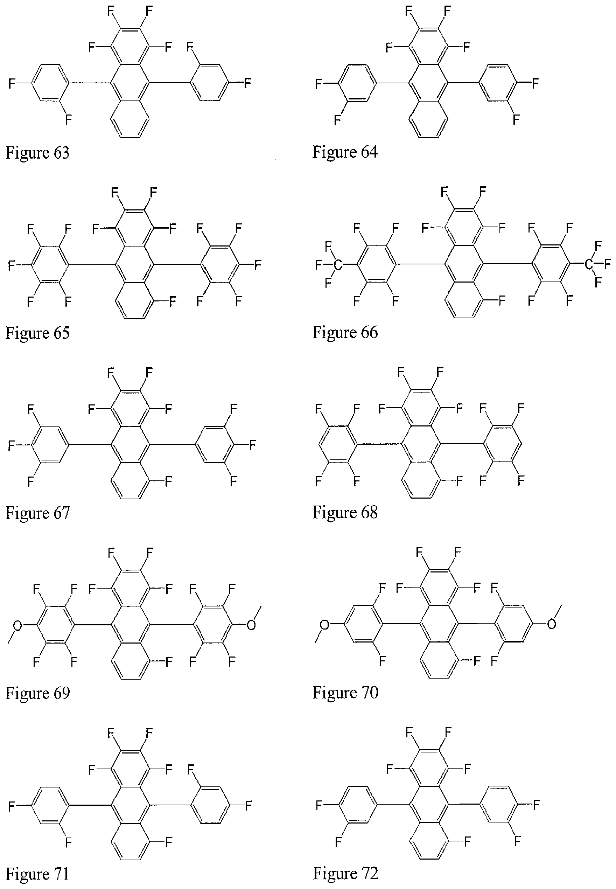

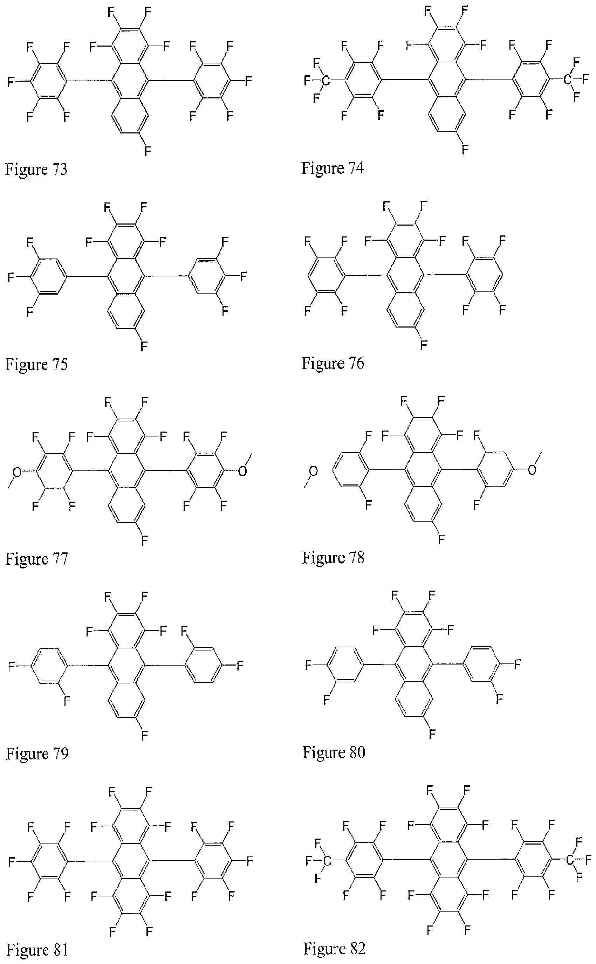

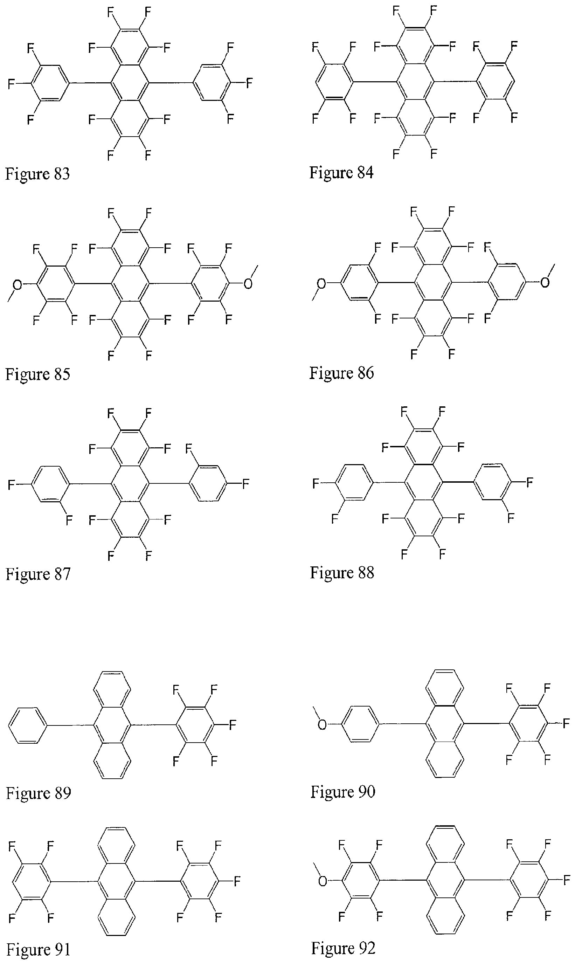

This method was used for compounds depicted in Figures 1-88.

### Example 2

Symmetrically and asymmetrically substituted anthracenes can be made via Reaction Scheme 2 and Reaction Scheme 3 or a variation thereof using the procedures in the following: patents and patent applications, US 2004/0161633 A1, US 6,815,094 B2, US, 2007/0176544 A1; literature references, Org. Lett. 2007, 9, 4893-4896. J. Am. Chem. Soc. 2008, 130, 1012-1016, and J. Org. Chem. 2008, 73, 5589-5591; and references therein.

This method was used for the symmetrically substituted compounds depicted in Figures 3, 6-8, 22-24, and 38-40 and most of the asymmetrically substituted compounds or a portion thereof.

### Example 3

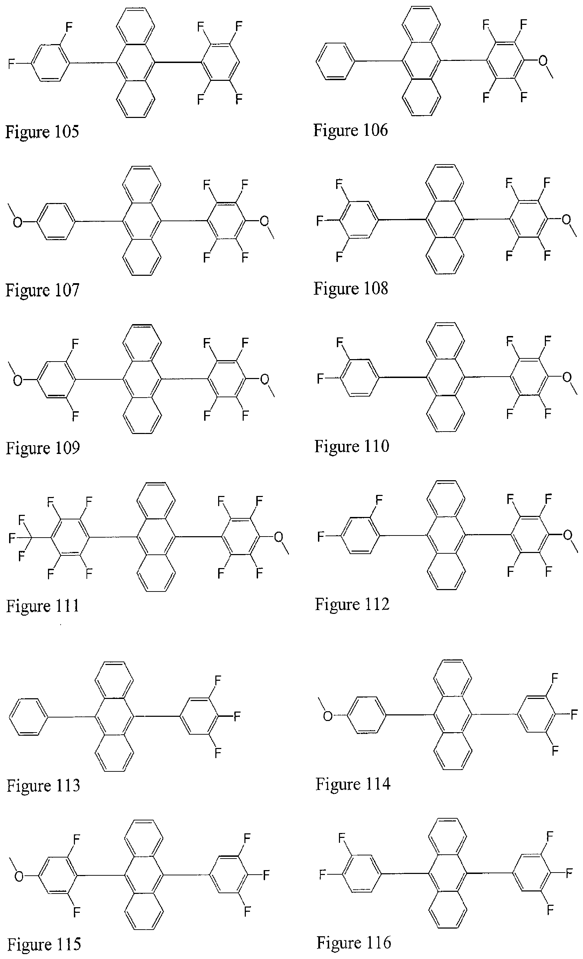

Procedure for producing 9-(4-methoxy-2,3,5,6-tetrafluorophenyl)-10-phenylanthracene (Figure 106)

This compound was made using the method depicted in Reaction Scheme 4. A flask with a magnetic stir bar was charged with 1.00 g of potassium carbonate, 0.15 g of dicyclohexylphosphinodimethoxybiphenyl (S-Phos), and 0.04 g of palladium acetate. The reaction vessel was evacuated and backfilled with argon three times. A solution of 1.29 g of 2,3,5,6-tetrafluoroanisole in 8 mL of isopropyl acetate was added via syringe, and the reaction mixture was stirred for one minute. A solution of 9-bromo-10-phenylanthracene in 4 mL in isopropyl acetate was added. The reaction mixture was heated to 80°C and held at that temperature overnight. The reaction was filtered and the crude material purified via column chromatography on silica gel using 5% diethyl ether in heptane to yield 350 mg of a white solid with a melting point of 153-154°C.

This method was used to attach pentafluorophenyl, 2,3,5,6-tetrafluorophenyl, and 2,3,5,6-tetrafluoro-4-methoxyphenyl groups to a 9-haloanthracene or a 9-halo-10-arylanthracene as described in Org. Lett. 2006, 8, 5097-5100 and references therein.

### Example 4

The light output of the compounds was also tested. The process for producing chemiluminesence is described as follows;
(A) A quantity of butyl benzoate is heated to 70° C and purged with nitrogen gas and brought to room temperature under nitrogen. To a suitable vessel are added 85.81 parts by weight of butyl benzoate and 14.0 parts by weight of bis(2,4,5-trichloro-6-carbopentoxyphenyl)oxalate, with stirring, until the oxalate is dissolved. To the resultant solution is then added 0.19 parts by weight of the blue/violet fluorescer. The solution is allowed to cool and stored under nitrogen. The resultant solution is called Solution A.
(B) To a second vessel are added 86.7 parts by weight of dimethyl phthalate and 10.0 parts by weight of t-butyl alcohol. To this mixture are added 3.3 parts by weight of 70% hydrogen peroxide and 0.0072 parts of sodium salicylate. The resultant solution is called Solution B. Optionally, Solution B can be made by combining 96.7 parts of triethylcitrate, 0.0100 parts of sodium salicylate, and 3.3 parts of 70% hydrogen peroxide.
(C) To 4.7 parts by weight of Solution A is added 5.6 parts by weight of Solution B with thorough mixing. The result is the generation of a blue/violet chemiluminescent light.

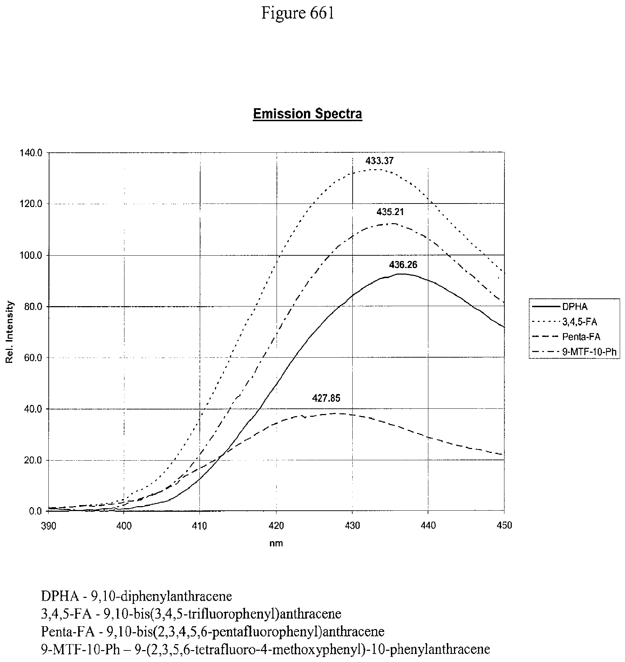

The emission spectra for several of anthracene fluorescers are shown in Figure 661. This figure illustrates the enhanced percentage of light in the region where the emission maximum is from about 390 nm to less than438 nm for compounds described herein.

## Claims

1. A composition comprising an oxalate solution and an anthracene compound represented by Formula (1): 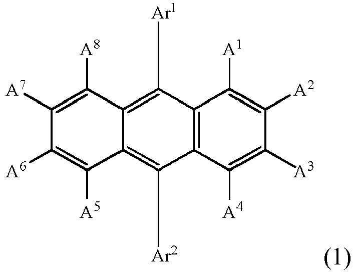 wherein Ar¹ has a structure represented by Formula 2, where An shows the attachment to
the anthracene ring as described in Formula 1, 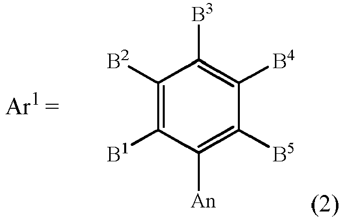 wherein Ar² has a structure represented by Formula 3, where An shows the attachment to the anthracene ring as described in Formula 1, 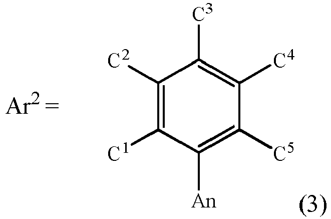 wherein each of A¹, A², A³, A⁴, A⁵, A⁶, A⁷, and A⁸ are selected from the group consisting of hydrogen and fluorine;
wherein at least two of the substituents, B¹, B², B³, B⁴, and B⁵, as shown in Formula 2, are not hydrogen, wherein each of B¹, B², B⁴, and B⁵ are selected from the group consisting of hydrogen and fluorine, and B³ is selected from the group consisting of hydrogen, fluorine, trifluoromethyl, and an alkoxy group with 1-10 carbons; and
wherein at least two of the substituents, C¹, C², C³, C⁴, and C⁵, as shown in Formula 3, are not hydrogen, wherein each of C¹, C², C⁴, and C⁵ are selected from the group consisting of hydrogen and fluorine, and C³ is selected from the group consisting of hydrogen, fluorine, trifluoromethyl, and an alkoxy group with 1-10 carbons,
wherein the composition is for reacting with a hydrogen peroxide solution to provide a chemiluminescent system that produces a blue/violet chemiluminescent light in the region where the emission maximum is from 390 nm to less than 438 nm.

2. The composition of claim 1 wherein
Ar¹ and Ar² are different from each other; and
wherein each of C¹, C², C³, C⁴, and C⁵ are selected from the group consisting of hydrogen, fluorine, trifluoromethyl, and an alkoxy group with 1-10 carbons.

3. The composition of claim 1 or 2, further comprising a solvent.

4. The composition of claim 3, wherein the solvent is selected from the group consisting of dibutyl phthalate, butyl benzoate, propylene glycol dibenzoate, ethylhexyl diphenyl phosphate and mixtures comprising at least one of the foregoing.

5. The composition of claim 1 or 2, wherein the oxalate compound is a bis(phenyl) oxalate ester having the formula: 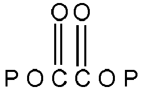
in which the phenyl groups(P) are substituted by at least one carbalkoxy group of the formula 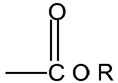
in which R is (a) an alkyl group (1-18 carbons, straight chain, branched chain, cyclic) or (b) a substituted alkyl group, where said substituents are selected from the group consisting of fluoro, chloro, trifluoromethyl, alkoxy, cyano, carbalkoxy, and phenyl; and in which (2) the phenyl groups P, are substituted by at least two additional substituents selected from the group consisting of fluoro, chloro, bromo, cyano, trifluoromethyl, carbalkoxy, nitro, alkoxy, alkoxy methyl, methyl, and higher alkyl, said additional substituents being selected so that the sum of their Hammett sigma constants for phenols lies between 1.0 and 2.

6. The composition of claim 5, wherein the oxalate is bis(2,4,5-trichloro-6-carbopentoxyphenyl)oxalate.

7. A chemiluminescent system comprising the composition of claim 1 or 2, and a hydrogen peroxide solution, wherein the chemiluminescent system is for producing a blue/violet chemiluminescent light in the region where the emission maximum is from 390 nm to less than 438 nm.

8. The chemiluminescent system of claim 7, wherein the chemiluminescent system is for producing a blue/violet chemiluminescent light having an emission maximum in the region from 400nm to about 420 nm.

9. The chemiluminescent system of claim 7, wherein the peroxide-containing component contains a solvent selected from the group consisting of tertiary alcohols, triethyl citrate, dimethyl phthalate, and mixtures comprising at least one of the foregoing.

10. The chemiluminescent system of claim 7, wherein the peroxide-containing component further contains a catalyst.

11. The chemiluminescent system of claim 10, wherein the catalyst is a salicylate.

## Patentansprüche

1. Eine Zusammensetzung, umfassend eine Oxalat-Lösung und eine Anthrazen-Verbindung, dargestellt von Formel (1): 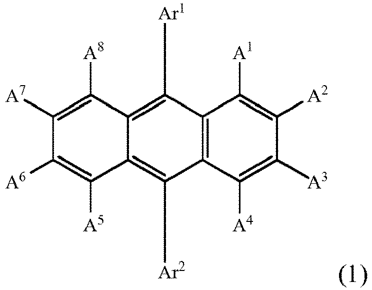 wobei Ar¹ eine von Formel 2 dargestellte Struktur hat, wobei An die Verbindung zum Anthrazen-Ring wie in Formel 1 beschrieben zeigt,
Ar¹ = 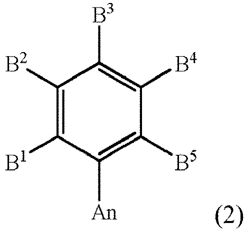 wobei Ar² eine von Formel 3 dargestellte Struktur hat, wobei An die Verbindung zum Anthrazen-Ring wie in Formel 1 beschrieben zeigt,
Ar² = 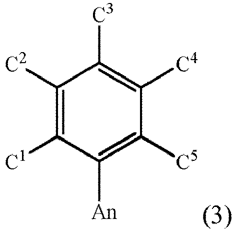 wobei jedes von A¹, A², A³, A⁴, A⁵, A⁶, A⁷ und A⁸ ausgewählt ist aus der Gruppe bestehend aus Wasserstoff und Fluor;
wobei mindestens zwei der Substituenten B¹, B², B³, B⁴ und B⁵, wie in Formel 2 gezeigt, nicht Wasserstoff sind, wobei jedes von B¹, B², B⁴ und B⁵ ausgewählt ist aus der Gruppe bestehend aus Wasserstoff und Fluor und B³ ausgewählt ist aus der Gruppe bestehend aus Wasserstoff, Fluor, Trifluormethyl, und einer Alkoxy-Gruppe mit 1-10 Kohlenstoffen; und
wobei mindestens zwei der Substituenten C¹, C², C³, C⁴, C⁵, wie in Formel 3 gezeigt, nicht Wasserstoff sind, wobei jedes von C¹, C², C⁴ und C⁵ ausgewählt ist aus der Gruppe bestehend aus Wasserstoff und Fluor und C³ ausgewählt ist aus der Gruppe bestehend aus Wasserstoff, Fluor, Trifluormethyl, und einer Alkoxy-Gruppe mit 1-10 Kohlenstoffen,
wobei die Zusammensetzung zur Reaktion mit einer Wasserstoffperoxid-Lösung dient, um ein chemilumineszentes System zu bieten, das blaues/violettes chemilumineszentes Licht produziert, im Bereich, wo das Emissionsmaximum größer/gleich 390 nm und kleiner als 438 nm ist.

2. Die Zusammensetzung von Anspruch 1, wobei
Ar¹ und Ar² voneinander verschieden sind; und
wobei jedes von C¹, C², C³, C⁴ und C⁵ ausgewählt ist aus der Gruppe bestehend aus Wasserstoff, Fluor, Trifluormethyl und einer Alkoxy-Gruppe mit 1-10 Kohlenstoffen.

3. Die Zusammensetzung von Anspruch 1 oder 2, die außerdem ein Lösungsmittel umfasst.

4. Die Zusammensetzung von Anspruch 3, wobei das Lösungsmittel ausgewählt ist aus der Gruppe bestehend aus Dibutylphthalat, Butylbenzoat, Propylenglycoldibenzoat, Ethylhexyldiphenylphosphat und Mischungen, die mindestens eins der Vorhergehenden umfassen.

5. Die Zusammensetzung von Anspruch 1 oder 2, wobei die Oxalat-Verbindung ein Bis(phenyl)oxalatester mit der folgenden Formel ist: 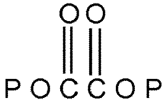 wobei die Phenylgruppen (P) mit mindestens einer Carbonsäureester-Gruppe der Formel 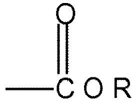 substituiert sind, wobei R (a) eine Alkyl-Gruppe ist (1-18 Kohlenstoffe, unverzweigte Kette, verzweigte Kette, cyclisch) oder (b) eine substituierte Alkyl-Gruppe ist, wobei jene Substituenten ausgewählt sind aus der Gruppe bestehend aus Fluor, Chlor, Trifluormethyl, Alkoxy, Cyano, Carbonsäureester und Phenyl; und wobei (2) die Phenylgruppen P mit mindestens zwei zusätzlichen Substituenten substituiert sind, ausgewählt aus der Gruppe bestehend aus Fluor, Chlor, Brom, Cyano, Trifluormethyl, Carbonsäureester, Nitro, Alkoxy, Alkoxymethyl, Methyl und längerkettigem Alkyl, wobei jene zusätzlichen Substituenten so ausgewählt sind, dass die Summe ihrer Hammett-Sigma-Konstanten für Phenol zwischen 1,0 und 2 liegt.

6. Die Zusammensetzung von Anspruch 5, wobei das Oxalat Bis(2, 4, 5-trichlor-6-carbopentoxyphenyl)oxalat ist.

7. Ein chemilumineszentes System, umfassend die Zusammensetzung von Anspruch 1 oder 2 und eine Wasserstoffperoxid-Lösung, wobei das chemilumineszente System zur Produktion von blau/violettem chemilumineszentem Licht dient, im Bereich, wo das Emissionsmaximum größer/gleich 390 nm und weniger als 438 nm ist.

8. Das chemilumineszente System von Anspruch 7, wobei das chemilumineszente System zur Produktion von blau/violettem chemilumineszentem Licht dient, mit einem Emissionsmaximum im Bereich von 400 nm bis etwa 420 nm.

9. Das chemilumineszente System von Anspruch 7, wobei die Peroxid-enthaltende Komponente ein Lösungsmittel enthält, ausgewählt aus der Gruppe bestehend aus tertiären Alkoholen, Triethylcitrat, Dimethylphthalat und Mischungen, die mindestens eins der Vorhergehenden umfassen.

10. Das chemilumineszente System von Anspruch 7, wobei die Peroxid-enthaltende Komponente außerdem einen Katalysator enthält.

11. Das chemilumineszente System von Anspruch 10, wobei der Katalysator ein Salicylat ist.

## Revendications

1. Composition comprenant une solution d'oxalate et un composé anthracène représenté par la Formule (1) : 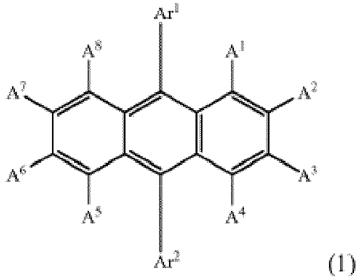 dans laquelle Ar¹ a une structure représentée par la Formule 2, où An représente la fixation au cycle anthracène tel que décrit dans la Formule 1,
Ar¹ = 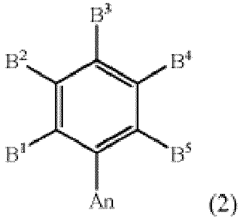 dans laquelle Ar² a une structure représentée par la Formule 3, où An représente la fixation au cycle anthracène tel que décrit dans la Formule 1,
Ar² = 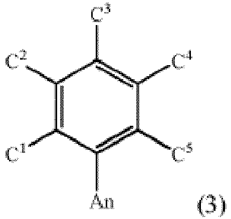 dans laquelle chacun parmi A¹, A², A³, A⁴, A⁵, A⁶, A⁷ et A⁸ sont choisis dans le groupe consistant en hydrogène et fluor ;
dans laquelle au moins deux des substituants, B¹, B², B³, B⁴ et B⁵, tels que représentés dans la Formule 2, ne sont pas hydrogène, chacun parmi B¹, B², B⁴ et B⁵ étant choisi dans le groupe consistant en hydrogène et fluor, et B³ étant choisi dans le groupe consistant en hydrogène, fluor, trifluorométhyle et un groupe alcoxy ayant 1-10 carbones ; et
dans laquelle au moins deux des substituants, C¹, C², C³, C⁴ et C⁵, tels que représentés dans la Formule 3, ne sont pas hydrogène, chacun parmi C¹, C², C⁴ et C⁵ étant choisi dans le groupe consistant en hydrogène et fluor, et C³ étant choisi dans le groupe consistant en hydrogène, fluor, trifluorométhyle et un groupe alcoxy ayant 1-10 carbones,
la composition étant destinée à réagir avec une solution de peroxyde d'hydrogène pour fournir un système chimiluminescent qui produit une lumière chimiluminescente bleue/violette dans la région dans laquelle le maximum d'émission est de 390 nm à moins de 438 nm.

2. Composition selon la revendication 1, dans laquelle
Ar¹ et Ar² sont différents l'un de l'autre ; et
dans laquelle chacun parmi C¹, C², C³, C⁴ et C⁵ est choisi dans le groupe consistant en hydrogène, fluor, trifluorométhyle et un groupe alcoxy ayant 1-10 carbones.

3. Composition selon l'une des revendications 1 ou 2, comprenant en outre un solvant.

4. Composition selon la revendication 3, dans laquelle le solvant est choisi dans le groupe consistant en phtalate de dibutyle, benzoate de butyle, dibenzoate de propylène glycol, éthylhexyl diphényl phosphate et les mélanges comprenant au moins l'un des précédents.

5. Composition selon l'une des revendications 1 ou 2, dans laquelle le composé oxalate est un ester bis(phényl) oxalate ayant la formule : 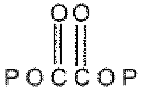 dans laquelle les groupes phényle (P) sont substitués par au moins un groupe carbalcoxy de la formule 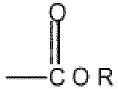 dans laquelle R est (a) un groupe alkyle (1-18 carbones, à chaîne droite, à chaîne ramifiée, cyclique) ou (b) un groupe alkyle substitué, lesdits substituants étant choisis dans le groupe consistant en fluoro, chloro, trifluorométhyle, alcoxy, cyano, carbalcoxy et phényle ; et dans laquelle (2) les groupes phényle P sont substitués par au moins deux substituants supplémentaires choisis dans le groupe consistant en fluoro, chloro, bromo, cyano, trifluorométhyle, carbalcoxy, nitro, alcoxy, alcoxyméthyle, méthyle et alkyle supérieur, lesdits substituants supplémentaires étant choisis de telle sorte que la somme de leurs constantes sigma de Hammett pour les phénols se situe entre 1,0 et 2.

6. Composition selon la revendication 5, dans laquelle l'oxalate est le bis(2,4,5-trichloro-6-carbopentoxyphényl)oxalate.

7. Système chimiluminescent comprenant la composition selon l'une des revendications 1 ou 2, et une solution de peroxyde d'hydrogène, le système chimiluminescent étant destiné à produire une lumière chimiluminescente bleue/violette dans la région dans laquelle le maximum d'émission est de 390 nm à moins de 438 nm.

8. Système chimiluminescent selon la revendication 7, le système chimiluminescent étant destiné à produire une lumière chimiluminescente bleue/violette dans la région dans laquelle le maximum d'émission est de 400 nm à environ 420 nm.

9. Système chimiluminescent selon la revendication 7, dans lequel le composant contenant du peroxyde contient un solvant choisi dans le groupe consistant en alcools tertiaires, citrate de triéthyle, phtalate de diméthyle et les mélanges comprenant au moins l'un des précédents.

10. Système chimiluminescent selon la revendication 7, dans lequel le composant contenant du peroxyde contient en outre un catalyseur.

11. Système chimiluminescent selon la revendication 10, dans lequel le catalyseur est un salicylate.
